(19) Europäisches Patentamt
European Patent Office
Office européen des brevets

(11) EP 1 349 104 A2

(12) EUROPEAN PATENT APPLICATION

(43) Date of publication:
01.10.2003 Bulletin 2003/40

(51) Int Cl.7: G06F 19/00

(21) Application number: 03252027.2

(22) Date of filing: 31.03.2003

(84) Designated Contracting States:
AT BE BG CH CY CZ DE DK EE ES FI FR GB GR HU IE IT LI LU MC NL PT RO SE SI SK TR
Designated Extension States:
AL LT LV MK RO

(30) Priority: 29.03.2002 US 368790 P

(71) Applicant: Ortho-Clinical Diagnostics, Inc.
Rochester, NY 14626-5101 (US)

(72) Inventor: Jatkoe, Tim
La Jolla, CA 92037-4335 (US)

(74) Representative: Mercer, Christopher Paul et al
Carpmaels & Ransford
43, Bloomsbury Square
London WC1A 2RA (GB)

# (54) Method of selecting a portfolio of markers for use in a diagnostic application

(57) Methods of selecting a portfolio of markers for use in a diagnostic applications include defining diagnostic parameters, establishing a relationship among the parameters so that they are optimized, and selecting an optimal group of markers for the diagnostic application. The diagnostic parameters can include a measure of the relative degree of expression of a gene, a measure of the variation in the measurement of the degree of expression of the gene, and the relationship between the diagnostic and discriminating parameters can be a mean variance relationship.

Machines programmed to conduct the method and articles that comprise instructions for their operation are further aspects of the invention.

EP 1 349 104 A2

## Description

## BACKGROUND

This application claims the benefit of U.S Provisional Application No.60/368,790 filed on March 29, 2002.
The invention relates to the selection of portfolios of diagnostic markers.
A few single gene diagnostic markers such as her-2-neu are currently in use. Usually, however, diseases are not easily diagnosed with molecular diagnostics for one particular gene. Multiple markers are often required and the number of such markers that may be included in a assay based on differential gene modulation can be large, even in the hundreds of genes. It is desirable to group markers into portfolios so that the most reliable results are obtained using the smallest number of markers necessary to obtain such a result. This is particularly true in assays that contain multiple steps such as nucleic acid amplification steps.

## SUMMARY OF THE INVENTION

The invention is a method of selecting a portfolio of markers for use in a diagnostic application in which diagnostic parameters are defined, a relationship among the parameters is established so that they are optimized, and the relationship is used to select an optimal group of markers for the diagnostic application.
In another aspect of the invention, the diagnostic parameters include a measure of the relative degree of expression of a gene, a measure of the variation in the measurement of the degree of expression of the gene, and the relationship between the diagnostic and discriminating parameters is a mean variance relationship.
Machines programmed to conduct the inventive method and articles that comprise instructions for their operation are further aspects of the invention.

## DETAILED DESCRIPTION

The methods of this invention can be used in conjunction with any method for determining the gene expression patterns of relevant cells as well as protein based methods of determining gene expression. Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify copy DNA (cDNA) or copy RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637; the disclosures of which are incorporated herein by reference.
Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al., the disclosure of each of which is incorporated herein by reference.
Analysis of the expression levels is conducted by comparing such intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased colon tissue sample vs. normal colon tissue sample). A ratio of these expression intensities indicates the fold-change in gene expression between the test and control samples.
Modulated genes are those that are differentially expressed as up regulated or down regulated in non-normal cells. Up regulation and down regulation are relative terms meaning that a detectable difference (beyond the contribution of noise in the system used to measure it) is found in the amount of expression of the genes relative to some baseline. In this case, the baseline is the measured gene expression of a normal cell. The genes of interest in the non-normal cells are then either up regulated or down regulated relative to the baseline level using the same measurement method.

Preferably, levels of up and down regulation are distinguished based on fold changes of the intensity measurements of hybridized microarray probes. For example, in the case in which a 1.5 fold or more difference is used to make such distinctions, the diseased cell is found to yield at least 1.5 times more, or 1.5 times less intensity than the normal cells.

Other methods of making distinctions are available. For example, statistical tests can be used to find the genes most significantly different between diverse groups of samples. The Student's t-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, there is likelihood to see small p-values just by chance and adjustments for this using a Sidak correction as well as a randomization/permutation experiment can be made.

A p-value less than .05 by the t-test is evidence that the gene is significantly different. More compelling evidence is a p-value less then .05 after the Sidak correct is factored in. For a large number of samples in each group, a p-value less than 0.05 after the randomization/ permutation test is the most compelling evidence of a significant difference.

Genes can be grouped so that information obtained about the set of genes in the group provides a sound basis for making clinically relevant judgments such as a diagnosis, prognosis, or treatment choice. These sets of genes make up the portfolios of the invention. As with most diagnostic markers, it is often desirable to use the fewest number of markers sufficient to make a correct medical judgment. This prevents a delay in treatment pending further analysis as well as inappropriate use of time and resources. Preferred optimal portfolio is one that employs the fewest number of markers for making such judgments while meeting conditions that maximize the probability that such judgments are indeed correct. These conditions will generally include sensitivity and specificity requirements. In the context of microarray based detection methods, the sensitivity of the portfolio can be reflected in the fold differences exhibited by a gene's expression in the diseased or aberrant state relative to the normal state. The detection of the differential expression of a gene is sensitive if it exhibits a large fold change relative to the expression of the gene in another state. Another aspect of sensitivity is the ability to distinguish signal from noise. For example, while the expression of a set of genes may show adequate sensitivity for defining a given disease state, if the signal that is generated by one (e.g., intensity measurements in microarrays) is below a level that easily distinguished from noise in a given setting (e.g., a clinical laboratory) then that gene should be excluded from the optimal portfolio. A procedure for setting conditions such as these that define the optimal portfolio can be incorporated into the inventive methods.

Specificity can be reflected in statistical measurements of the correlation of the signaling of gene expression with the condition of interest. If the differential expression of a set of genes is observed to produce a large fold change but they do so for a number of conditions other than the condition of interest (e.g. multiple disease states) then the gene expression profile for that set of genes is non-specific. Statistical measurements of correlation of data or the degree of consistency of data such as standard deviation, correlation coefficients, and the like can be a used as such measurements. In considering a group of genes for inclusion in a portfolio, a small standard deviation in expression measurements correlates with greater specificity. Genes that display similar expression patterns may be co-regulated by an identical factor that pushes the genes in the same direction. If this factor is sufficient but not necessary for classifying a sample, then these genes will fail to correctly identify a sample if the markers are all related to this single factor. Diversification then results in selecting as few markers as possible, yet covers as many different optimal expression patterns that are contained in the data set

In the method of the invention, a group of genetic markers is selected for use in diagnostic applications. These groups of markers are "portfolios". Diagnostic applications include the detection or identification of a disease state or condition of a subject, determining the likelihood that a subject will contract a given disease or condition, determining the likelihood that a subject with a disease or condition will respond to therapy, determining the prognosis of a subject with a disease or condition (or its likely progression or regression), and determining the effect of a treatment on a subject with a disease or condition. For example, the method can be used to establish portfolios for detecting the presence or likelihood of a subject contracting colon cancer or the likelihood that such a subject will respond favorably to cytotoxic drugs.

The portfolios selected by the method of the invention contain a number and type of markers that assure accurate and precise results and are economized in terms of the number of genes that comprise the portfolio. The method of the invention can be used to establish optimal gene expression portfolios for any disease, condition, or state that is concomitant with the expression of multiple genes. An optimal portfolio in the context of the instant invention refers to a gene expression profile that provides an assessment of the condition of a subject (based upon the condition for which the analysis was undertaken) according to predetermined standards of at least two of the following parameters: accuracy, precision, and number of genes comprising the portfolio.

Most preferably, the markers employed in the portfolio are nucleic acid sequences that express mRNA ("genes"). Expression of the markers may occur ordinarily in a healthy subject and be more highly expressed or less highly expressed when an event that is the object of the diagnostic application occurs. Alternatively, expression may not occur except when the event that is the object of the diagnostic application occurs.

Marker attributes, features, indicia, or measurements that can be compared to make diagnostic judgments are diagnostic parameters used in the method. Indicators of gene expression levels are the most preferred diagnostic parameters. Such indicators include intensity measurements read from microarrays, as described above. Other diagnostic parameters are also possible such as indicators of the relative degree of methylation of the markers.

Distinctions are made among the diagnostic parameters through the use of mathematical/statistical values that are related to each other. The preferred distinctions are mean signal readings indicative of gene expression and measurements of the variance of such readings. The most preferred distinctions are made by use of the mean of signal ratios between different group readings (e.g., microarray intensity measurements) and the standard deviations of the signal ratio measurements. A great number of such mathematical/statistical values can be used in their place such as return at a given percentile.

A relationship among diagnostic parameter distinctions is used to optimize the selection of markers useful for the diagnostic application. Typically, this is done through the use of linear or quadratic programming algorithms. However, heuristic approaches can also be applied or can be used to supplement input data selection or data output. The most preferred relationship is a mean-variance relationship such as that described in *Mean-Variance Analysis in Portfolio Choice and Capital Markets* by Harry M. Markowitz (Frank J. Fabozzi Associates, New Hope, PA: 2000, ISBN: 1-883249-75-9) which is incorporated herein by reference. The relationship is best understood in the context of the selection of stocks for a financial investment portfolio. This is the context for which the relationship was developed and elucidated.

The investor looking to optimize a portfolio of stocks can select from a large number of possible stocks, each having a historical rate of return and a risk factor. The mean variance method uses a critical line algorithm of linear programming or quadratic programming to identify all feasible portfolios that minimize risk (as measured by variance or standard deviation) for a given level of expected return and maximize expected return for a given level of risk. When standard deviation is plotted against expected return an efficient frontier is generated. Selection of stocks along the efficient frontier results in a diversified stock portfolio optimized in terms of return and risk.

When the mean variance relationship is used in the method of the instant invention, diagnostic parameters such as microarray signal intensity and standard deviation replace the return and risk factor values used in the selection of financial portfolios. Most preferably, when the mean variance relationship is applied, a commercial computer software application such as the "Wagner Associates Mean-Variance Optimization Application", referred to as "Wagner Software" throughout this specification. This software uses functions from the "Wagner Associates Mean-Variance Optimization Library" to determine an efficient frontier and optimal portfolios in the Markowitz sense. Since such applications are made for financial applications, it may be necessary to preprocess input data so that it can conform to conventions required by the software. For example, when Wagner Software is employed in conjunction with microarray intensity measurements the following data transformation method is employed.

A relationship between each genes baseline and experimental value must first be established. The preferred process is conducted as follows. A baseline class is selected. Typically, this will comprise genes from a population that does not have the condition of interest. For example, if one were interested in selecting a portfolio of genes that are diagnostic for breast cancer, samples from patients without breast cancer can be used to make the baseline class. Once the baseline class is selected, the arithmetic mean and standard deviation is calculated for the indicator of gene expression of each gene for baseline class samples. This indicator is typically the fluorescent intensity of a microarray reading. The statistical data computed is then used to calculate a baseline value of (X*Standard Deviation + Mean) for each gene. This is the baseline reading for the gene from which all other samples will be compared. X is a stringency variable selected by the person formulating the portfolio. Higher values of X are more stringent than lower. Preferably, X is in the range of .5 to 3 with 2 to 3 being more preferred and 3 being most preferred.

Ratios between each experimental sample (those displaying the condition of interest) versus baseline readings are then calculated. The ratios are then transformed to base 10 logarithmic values for ease of data handling by the software. This enables down regulated genes to display negative values necessary for optimization according to the Markman mean-variance algorithm using the Wagner Software.

The preprocessed data comprising these transformed ratios are used as inputs in place of the asset return values that are normally used in the Wagner Software when it is used for financial analysis purposes.

Once an efficient frontier is formulated, an optimized portfolio is selected for a given input level (return) or variance that corresponds to a point on the frontier. These inputs or variances are the predetermined standards set by the person formulating the portfolio. Stated differently, one seeking the optimum portfolio determines an acceptable input level (indicative of sensitivity) or a given level of variance (indicative of specificity) and selects the genes that lie along the efficient frontier that correspond to that input level or variance. The Wagner Software can select such genes when an input level or variance is selected. It can also assign a weight to each gene in the portfolio as it would for a stock in a stock portfolio.

Determining whether a sample has the condition for which the portfolio is diagnostic can be conducted by comparing the expression of the genes in the portfolio for the patient sample with calculated values of differentially expressed genes used to establish the portfolio. Preferably, a portfolio value is first generated by summing the multiples of the intensity value of each gene in the portfolio by the weight assigned to that gene in the portfolio selection process. A boundary value is then calculated by (Y*standard deviation + mean of the portfolio value for baseline groups) where Y is a stringency value having the same meaning as X described above. A sample having a portfolio value greater than the boundary value of the baseline class is then classified as having the condition. If desired, this process can be conducted iteratively in accordance with well known statistical methods for improving confidence levels.

Optionally one can reiterate this process until best prediction accuracy is obtained.

The process of portfolio selection and characterization of an unknown is summarized as follows:

1. Choose baseline class.
2. Calculate mean, and standard deviation of each gene for baseline class samples.
3. Calculate (X*Standard Deviation + Mean) for each gene. This is the baseline reading from which all other samples will be compared. X is a stringency variable with higher values of X being more stringent than lower.
4. Calculate ratio between each Experimental sample versus baseline reading calculated in step 3.
5. Transform ratios such that ratios less than 1 are negative (eg.using Log base 10). (Down regulated genes now correctly have negative values necessary for MV optimization).
6. These transformed ratios are used as inputs in place of the asset returns that are normally used in the software application.
7. The software will plot the efficient frontier and return an optimized portfolio at any point along the efficient frontier.
8. Choose a desired return or variance on the efficient frontier.
9. Calculate the Portfolio's Value for each sample by summing the multiples of each gene's intensity value by the weight generated by the portfolio selection algorithm.
10. Calculate a boundary value by adding the mean Portfolio Value for Baseline groups to the multiple of Y and the Standard Deviation of the Baseline's Portfolio Values. Values greater than this boundary value shall be classified as the Experimental Class.
11. Optionally one can reiterate this process until best prediction accuracy is obtained.

A second portfolio can optionally be created by reversing the baseline and experimental calculation. This creates a new portfolio of genes which are up-regulated in the original baseline class. This second portfolio's value can be subtracted from the first to create a new classification value based on multiple portfolios.

Another useful method of pre-selecting genes from gene expression data so that it can be used as input for a process for selecting a portfolio is based on a threshold given by

$$1 \leq \left|\frac{(\mu_t - \mu_n)}{(\sigma_t + \sigma_n)}\right|,$$

where $\mu_t$ is the mean of the subset known to possess the disease or condition, $\mu_n$ is the mean of the subset of normal samples, and $\sigma_t + \sigma_n$ represent the combined standard deviations. A signal to noise cutoff can also be used by pre-selecting the data according to a relationship such as

$$0.5 \leq \left|\frac{(\mu_t - MAX_n)}{(\sigma_t + \sigma_n)}\right|.$$

This ensures that genes that are pre-selected based on their differential modulation are differentiated in a clinically significant way. That is, above the noise level of instrumentation appropriate to the task of measuring the diagnostic parameters. For each marker pre-selected according to these criteria, a matrix is established in which columns represents samples, rows represent markers and each element is a normalized intensity measurement for the expression of that marker according to the relationship:

$$\left|\frac{(\mu_t - I)}{\mu_t}\right|$$

where I is the intensity measurement.

Using this process of creating input for financial portfolio software make also allows one to set additional boundary conditions to define the optimal portfolios. For example, portfolio size can be limited to a fixed range or number of markers. This can be done either by making data pre-selection criteria more stringent (e.g,

$$.8 \le \left|\frac{(\mu_t - MAX_n)}{(\sigma_t + \sigma_n)}\right|$$

instead of

$$\left|0.5 \le \frac{(\mu_t - MAX_n)}{(\sigma_t + \sigma_n)}\right|)$$

or by using programming features such as restricting portfolio size. One could, for example, set the boundary condition that the efficient frontier is to be selected from among only the optimal 10 genes. One could also use all of the genes pre-selected for determining the efficient frontier and then limit the number of genes selected (e.g., no more than 10).

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with breast cancer. Output from the method would be an optimized set of genes that could include some genes that are expressed in peripheral blood as well as in diseased breast tissue. If sample used in the testing method are obtained from peripheral blood and certain genes differentially expressed in instances of breast cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply the rule that only a given percentage of the portfolio can be represented by a particular gene or genes. Commercially available software such as the Wagner Software readily accommodates these types of heuristics. This can be useful, for example, when factors other than accuracy and precision (e.g., anticipated licensing fees) have an impact on the desirability of including one or more genes.

Other relationships aside from the mean-variance relationship can be used in the method of the invention provided that they optimize the portfolio according to predetermined attributes such as assay accuracy and precision. Two examples are the Martin simultaneous equation approach (Elton, Edwin J. and Martin J. Gruber (1987), *Modern Portfolio Theory Investment Analysis,* Third Edition, John Wiley, New York, 1987) and Genetic Algorithms (Davis, L., (1989), *Adapting Operator Probabilities in Genetic Algorithms,* in *Proceedings of the Third International Conference on Genetic Algorithms,* Morgan Kaufmann: San Mateo, pp. 61-69). There are also many ways to adapt the mean-variance relationship to handle skewed data such as where a marker detection technology exhibits a known bias. These include, for example, the Semi-Deviation method in which the square root of the average squared (negative) deviation from a reference signal and includes only those signal values that fall below the reference signal.

Articles of this invention include representations of the gene expression profiles that make up the portfolios useful for treating, diagnosing, prognosticating, and otherwise assessing diseases. These representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. When such a microarray contains an optimized portfolio great savings in time, process steps, and resources are attained by minimizing the number of cDNA or oligonucleotides that must be applied to the substrate, reacted with the sample, read by an analyzer, processed for results, and (sometimes) verified.

Other articles according to the invention can be fashioned into reagent kits for conducting hybridization, amplification, and signal generation indicative of the level of expression of the genes in the portfolios established through the method of the invention. Kits made according to the invention include formatted assays for determining the gene expression profiles. These can include all or some of the materials needed to conduct the assays such as reagents and instructions.

## EXAMPLES

### Example 1: Producing an Optimized Portfolio

Gene expression data was recently produced from tissue samples representative of eleven different types of cancers. The data was published in *Cancer Research* 61: 7388-7393, 2001 and http://carrier.gnf.org/welsh/epican/. *See,* Andrew I. Su et al., "Molecular Classification of Human Carcinomas by Use of Gene Expression Signatures."The data included intensity measurements obtained with the use of an "U95"a oligonucleotide microarray (commercially available from Affymetrix, Inc.).

Measurements of the expression of genes from the published data (fluorescent intensity measurements) was used to select optimum gene expression portfolios for a panel of markers to determine whether a circulating cell is indicative of the presence of breast cancer, prostate cancer, ovarian cancer, colorectal cancer, or lung cancer. Such circulating cells would preferably be epithelial cells.

The data in the study was collected from the following samples: 24 adenocarcinomas, 12 infilitrating ductal breast adenocarcenomas, 21 colorectal adenocarcinomas, 23 ovarian adenocarcinomas, 25 lung carcinomas, and data from the following additional samples: 19 prostate adenocarcinomas, 12 breast carinomas, 13 colon carcinomas, 13 ovarian carcinomas, 13 ovarian carcinomas, and 89 lung carcenomas.

Using intensity readings from a collection of normal samples as the baseline class, the arithmetic mean, and standard deviation of each gene were calculated followed by a calculation of the value (X*Standard Deviation + Mean) for each gene. The stringency variable, X, was assigned a value of 3 in this case. Ratios were then calculated between each Experimental sample described in the study versus the baseline value calculations. The ratios were transformed into common logarithms. These values were then used as the input values for the Wagner Software.

This procedure selected an efficient frontier along which a minimum set of markers for each tumor type that have the lowest amount of variation for a selected level of differential (chosen at the best signal to noise ratio point). Optimization by the software resulted in the selection of a portfolio of 24 genes including 2 for prostate cancer, 5 for breast cancer, 6 for colon cancer, 2 for ovarian cancer, and 9 for lung cancer markers (Table 1).

Table 1.

| Cancer Type | Accession | Name | Description | Seq, ID No. |
|---|---|---|---|---|
| PR | NM_001648 | KLK3 | kallikrein 3, (prostate specific antigen) | Seq. ID No. 1 |
| PR | NM_005551 | KLK2 | kallikrein 2, prostatic | Seq. ID No. 2 |
| BR | NM_004064 | CDKN1B | cyclin-dependent kinase inhibitor 1B (p27, Kip1) | Seq. ID No. 34 |
| BR | NM_002411 | MGB1 | mammaglobin 1 | Seq. ID No. 3 |
| BR | NM_005264 | GFRA1 | GDNF family receptor alpha 1 | Seq. ID No. 4 |
| BR | None | C18ORF1 | chromosome 18 open reading frame 1 | Seq. ID No. 98 |
| BR | NM_000095 | COMP | cartilage oligomeric matrix protein | Seq. ID No. 67 |
| CO | NM_001804 | CDX1 | caudal type homeo box transcription factor 1 | Seq. ID No. 8 |
| CO | NM_001046 | SLC12A2 | solute carrier family 12 (sodium/potassium/ chloride transporters), member 2 | Seq. ID No 9 |
| CO | NM_001285 | CLCA1 | chloride channel, calcium activated, family member 1 | Seq. ID No. 11 |
| CO | NM_007052 | NOX1 | NADPH oxidase 1 | Seq. ID No. 13 |
| CO | NM_002457 | MUC2 | mucin 2, intestinal/tracheal | Seq. ID No. 14 |
| CO | NM_004063 | CDH17 | cadherin 17, LI cadherin | Seq. ID No 15 |
| LU_A | NM_021950 | MS4A2 | membrane-spanning 4-domains, subfamily A, member 2 | Seq. ID No. 17 |

Table 1. (continued)

| Cancer Type | Accession | Name | Description | Seq, ID No. |
|---|---|---|---|---|
| LU_A | NM_000964 | ASAHL | N-acylsphingosine amidohydrolase (acid ceramidase)-like | Seq. ID No. 18 |
| LU_A | NM_006495 | EVI2B | ecotropic viral integration site 2B | Seq. ID No. 20 |
| LU_A | NM_006864 | LILRB3 | leukocyte immunoglobulin-like receptor, subfamily B | Seq. ID No. 21 |
| LU_A | X67301 | none | H.sapiens mRNA for IgM heavy chain constant region (Ab63) | Seq. ID No. 22 |
| LU_A | NM_002123 | HLA-DQB1 | major histocompatibility complex, class II, DQ beta 1 | Seq. ID No.23 |
| LU_S | NM_000673 | ADH7 | alcohol dehydrogenase 7 (class IV), mu or sigma polypeptide | Seq. ID No. 24 |
| LU_S | NM_003722 | TP63 | tumor protein 63 kDa with strong homology to p53 | Seq. ID No. 26 |
| LU_S | None | SOX2 | SRY (sex determining region Y)-box 2 | Seq. ID No. 32 |
| OV | NM_000906 | NPR1 | natriuretic peptide receptor A/guanylate cyclase A | Seq. ID No. 28 |
| OV | NM_000378 | WT1 | Wilms tumor 1 | Seq. ID No. 30 |

**Example 2: Heuristic Step**

A heuristic rule was further applied to the portfolio obtained in Example 1. That is, the rule stated that if the gene/marker identified would likely be expressed in peripheral blood or were well-characterized tissue markers (e.g. PSA, mammaglobin, etc.), then such genes/marker would be removed from the portfolio. Application of the rule enabled the establishment of a portfolio of genes/markers that are optimized for use in a screening application in which the patient sample is obtained by assaying components found in the peripheral blood such as epithelial cells. The result of the selected portfolio contains 31 genes as shown in Table 2.

Table 2.

| Cancer Type | Accession | Name | Description | Seq. ID No. |
|---|---|---|---|---|
| PR | Hs.12784 | KIAA0293 | KIAA0293 protein | Seq. ID No. 67 |
| PR | NM_006562 | LBX1 | transcription factor similar to D. melanogaster homeodomain protein lady bird late | Seq. ID No. 33 |
| PR | NM_016026 | LOC51109 | CGI-82 protein | Seq. ID No. 34 |
| PR | HG2261-HT2352 | none | Antigen | Seq. ID No. 99 |
| PR | NM_012449 | STEAP | six transmembrane epithelial antigen of the prostate | Seq. ID No. 35 |
| PR | NM_001634 | AMD1 | S-adenosylmethionine decarboxylase 1 | Seq. ID No. 36 |
| PR | HG2261-HT2351 | none | Antigen \| | Seq. ID No. 100 |
| PR | NM_006457 | LIM | LIM protein (similar to rat protein kinase C-binding enigma) | Seq. ID No. 37 |
| BR | NM_005853 | IRX5 | iroquois homeobox protein 5 | Seq. ID No. 38 |
| BR | NM_005264 | GFRA1 | GDNF family receptor alpha 1 | Seq. ID No. 39 |
| BR | none | C180RF1 | chromosome 18 open reading frame 1 | Seq. ID No. 98 |

Table 2. (continued)

| Cancer Type | Accession | Name | Description | Seq. ID No. |
|---|---|---|---|---|
| BR | NM_000095 | COMP | cartilage oligomeric matrix protein (pseudoachondroplasia, epiphyseal dysplasia 1, multiple) | Seq. ID No. 41 |
| CO | NM_001265 | CDX2 | caudal type homeo box transcription factor 2 | Seq ID No. 43 |
| CO | NM_001046 | SLC12A2 | solute carrier family 12 (sodium/potassium/ chloride transporters), member 2 | Seq. ID No. 44 |
| CO | NM_001285 | CLCA1 | chloride channel, calcium activated, family member 1 | Seq. ID No. 46 |
| CO | NM_004063 | CDH17 | cadherin 17, LI cadherin (liver-intestine) | Seq. ID No.48 |
| OV | NM_000906 | NPR1 | natriuretic peptide receptor A/guanylate cyclase A (atrionatriuretic peptide receptor A) | Seq. ID No. 50 |
| OV | NM_005504 | BCAT1 | branched chain aminotransferase 1, cytosolic | Seq. ID No. 52 |
| OV | NM_002398 | MEIS1 | Meis1 (mouse) homolog | Seq. ID No. 53 |
| OV | none | SPON1 | spondin 1, (f-spondin) extracellular matrix protein | Seq. ID No. 69 |
| OV | NM_001692 | none | M25809:Human endomembrane proton pump subunit mRNA \|GenBank=M25809 | Seq. ID No. 54 |
| OV | NM_002774 | KLK6 | kallikrein 6 (neurosin, zyme) | Seq. ID No. 55 |
| LU_A | NM_000964 | ASAHL | N-acylsphingosine amidohydrolase (acid ceramidase)-like | Seq. ID No. 56 |
| LU_A | NM_002838 | PTPRC | protein tyrosine phosphatase, receptor type, C | Seq. ID No. 58 |
| LU_A | NM_015364 | MD-2 | MD-2 protein | Seq. ID No. 59 |
| LU_A | NM_006875 | PIM2 | pim-2 oncogene | Seq. ID No. 60 |
| LU_S | NM_005554 | KRT6A | keratin 6A | Seq. ID No. 61 |
| LU_S | NM_000673 | ADH7 | alcohol dehydrogenase 7 (class IV), mu or sigma polypeptide | Seq. ID No. 62 |
| LU_S | NM_003722 | TP63 | tumor protein 63 kDa with strong homology to p53 | Seq. ID No. 64 |
| LU_S | none | SOX2 | SRY (sex determining region Y)-box 2 | Seq. ID No. 32 |
| LU_S | NM_005688 | ABCC5 | ATP-binding cassette, sub-family C (CFTR/ MRP), member 5 | Seq. ID No. 66 |

**Example 3: Prognostic Portfolios**

A patient sample set with known clinical outcomes was used to test the portfolio selection method of the invention. The sample set is described in van't Veer, L. J et al. Gene Expression Profiling Predicts Clinical Outcome of Breast Cancer, *Nature,* 415, 530 - 536, (2002), incorporated herein by reference. In that study, breast tissue samples were obtained from 78 patients exhibiting sporadic breast tumors. The patients were all less than 55 years of age and presented with a tumor less than 5cm. All were lymph node negative. Thirty four of the patients presented with distant metastases in less than 5 years while 44 showed no distant metastases in the same period.

The data from the study were then processed according to the method of the invention. Sample number 54 was removed from further analysis due to a high percentage of missing values. The mean and standard deviation of the intensity measurements for each gene were calculated using the non-metastatic samples as the baseline. A discriminating value of X*(Standard Deviation + Mean) was then calculated for each baseline gene ( X was assigned a value of 3). This value was used to ensure the resulting portfolio would be stringent. A ratio of the discriminating value to the baseline value was then calculated for each metastatic sample. This ratio was then converted to a common logarithm. This data was then imported into Wagner Software which produced an efficient frontier from which a portfolio of 16 genes was selected. The baseline and experimental values were then reversed and a second portfolio of 12 markers representing genes up-regulated in the non-metastatic cases was produced. The second portfolio's value is subtracted from the first portfolios value to create a combined portfolio value from all 28 genes. This final portfolio is comprised of genes from Seq. ID No.70 -97. 17 of the genes of this portfolio were also present in the 70 gene portfolio described in the reference. The genes of the portfolio are identified below. (Seq. ID No. 70, Seq. ID No. 72, Seq. ID No. 73-77, Seq. ID No. 79, Seq. ID No. 80, Seq. ID No. 85, Seq. ID No. 87, Seq. ID No. 91-93, Seq. ID No. 95 and Seq. ID. No. 97.)

| 28 Gene list (2 Portfolios)<br>Up in Metastatic Patients (Portfolio 1) | |
|---|---|
| Contig53226_RC | Seq. ID No. 89 |
| NM_012214 | Seq. ID No. 82 |
| NM_020386 | Seq. ID No. 86 |
| NM_004504 | Seq. ID No. 81 |
| AA555029_RC | Seq. ID No. 70 |
| AL080059 | Seq. ID No.74 |
| AF055033 | Seq. ID No. 73 |
| NM_016448 | Seq. ID No. 85 |
| Contig40831_RC | Seq. ID No. 95 |
| Contig63649_RC | Seq. ID No. 91 |
| Contig24252_RC | Seq. ID No. 93 |
| NM_000436 | Seq. ID No. 75 |
| NM_002019 | Seq. ID No. 77 |
| Contig55313_RC | Seq. ID No. 90 |
| Contig25991 | Seq. ID No. 97 |
| NM_000788 | Seq. ID No. 76 |
| *Up in Non-Metastatic Patients (Portfolio 2)* AB033007 | Seq. ID No. 71 |
| Contig42421_RC | Seq. ID No. 96 |
| NM_003748 | Seq. ID No. 78 |
| NM_013262 | Seq. ID No. 83 |
| NM_003862 | Seq. ID No. 79 |
| NM_003882 | Seq. ID No. 80 |
| Contig48328_RC | Seq. ID No. 87 |
| NM_015416 | Seq. ID No. 84 |
| AB037863 | Seq. ID No. 72 |
| Contig27312_RC | Seq. ID No. 88 |
| Contig32125_RC | Seq. ID No. 92 |
| Contig49670_RC | Seq. ID No. 94 |

| 17 Overlap | |
|---|---|
| **Systematic name** | |
| NM_003862 | Seq. ID No. 79 |
| NM_003882 | Seq. ID No. 80 |
| Contig48328_RC | Seq. ID No. 87 |
| AA555029_RC | Seq. ID No. 70 |
| AL080059 | Seq. ID No. 74 |
| AF055033 | Seq. ID No.73 |
| AF055033 | Seq. ID No. 73 |
| NM_016448 | Seq. ID No. 85 |
| AB037863 | Seq. ID No. 72 |
| Contig40831_RC | Seq. ID No. 95 |
| Contig63649_RC | Seq. ID No. 91 |
| Contig24252_RC | Seq. ID No. 93 |
| NM_000436 | Seq. ID No. 75 |
| NM_002019 | Seq. ID No. 77 |
| Contig32125_RC | Seq. ID No. 92 |
| Contig25991 | Seq. ID No. 97 |
| NM_000788 | Seq. ID No. 76 |

The two portfolios were then used to determine the prognosis of the 78 original samples by comparing gene expression signatures from the microarray data according to the method for testing the classification accuracy described in the reference. In the case of the 70 gene portfolio, 81% of the samples were properly characterized according to an optimized threshold biased to include ambiguous signatures as indicative of poor prognosis (85% for an absolute threshold). This portfolio misclassified 3 patients with a poor prognosis as having a good prognosis using the optimized threshold (5 for the absolute threshold). Twelve patients with a good prognosis were misclassified as having a good prognosis when they had a bad prognosis using the optimized threshold (8 for absolute).

In the case of the 28 gene portfolio, 94% of the samples were properly characterized according to an optimized threshold biased to include ambiguous signatures as indicative of poor prognosis (93% for an absolute threshold). This portfolio misclassified 3 patients with a poor prognosis as having a good prognosis using the optimized threshold (5 for the absolute threshold). Three patients with a good prognosis were misclassified as having a good prognosis when they had a bad prognosis using the optimized threshold (2 for absolute).

Comparing the two profiles, it is apparent that the profiles selected according to the method of the invention are much more economical and produce results that are more accurate and reliable than those of the comparative portfolio.

## Claims

**1.** A method of selecting a portfolio of markers for use in a diagnostic application comprising:

a) defining diagnostic parameters,
b) establishing a relationship among the diagnostic parameters that identifies an optimized portfolio of markers, and
c) selecting said portfolio.

**2.** The method of claim 1 wherein the markers comprise genes.

**3.** The method of claim 2 wherein the relationship is based on the expression of the gene.

**4.** The method of claim 2 wherein the relationship is based on the variance of the expression of the gene.

**5.** The method of claim 4 wherein the diagnostic parameters include a measure of the relative degree of expression of a gene and a measure of the variation in the measurement of the degree of expression of the gene; and the relationship is a mean variance relationship.

**6.** The method of claim 1 further comprising the application of a heuristic rule.

**7.** The method of claim 1 wherein the diagnostic parameters represent sensitivity and specificity.

**8.** A machine comprising a general purpose computer programmed to identify a portfolio of markers wherein said machine comprises instructions to employ a relationship among diagnostic parameters so that markers are optimized.

**9.** The machine of claim 8 programmed to operate according to the method of any one of claims 1 to 7

**10.** An article comprising instructions for selecting a portfolio of markers for use in a diagnostic application comprising:

a) instructions for inputting diagnostic parameters into an algorithm,
b) instructions for operating said algorithm wherein said algorithm relates the diagnostic parameters such that an optimized portfolio of markers is identifiable, and
c) instructions for selecting said portfolio.

**11.** The article of claim 10 which are adapted for performance of the method of any one of claims 1 to 7

**12.** The article of claim 10 or claim 11 wherein the instructions are machine readable.

**13.** The article of claim 12 wherein the instructions are computer readable.

**14.** A diagnostic portfolio selected according to the method of any one of claims 1 to 7.

**15.** The portfolio of claim 14 comprising genes.

SEQUENCE LISTING

<110> Jatkoe, Tim

<120> SELECTION OF MARKERS

<130> CDS 265 US NP

<150> 60/368,790
<151> 2002-03-29

<160> 100

<170> PatentIn version 3.1

<210> 1
<211> 1466
<212> DNA
<213> human

<400> 1
agccccaagc ttaccacctg cacccggaga gctgtgtgtc accatgtggg tcccggttgt 60

cttcctcacc ctgtccgtga cgtggattgg tgctgcaccc ctcatcctgt ctcggattgt 120

gggaggctgg gagtgcgaga agcattccca accctggcag gtgcttgtgg cctctcgtgg 180

cagggcagtc tgcggcggtg ttctggtgca cccccagtgg gtcctcacag ctgcccactg 240

catcaggaac aaaagcgtga tcttgctggg tcggcacagc ctgtttcatc ctgaagacac 300

aggccaggta tttcaggtca gccacagctt cccacacccg ctctacgata tgagcctcct 360

gaagaatcga ttcctcaggc caggtgatga ctccagccac gacctcatgc tgctccgcct 420

gtcagagcct gccgagctca cggatgctgt gaaggtcatg gacctgccca cccaggagcc 480

agcactgggg accacctgct acgcctcagg ctggggcagc attgaaccag aggagttctt 540

gaccccaaag aaacttcagt gtgtggacct ccatgttatt tccaatgacg tgtgtgcgca 600

agttcaccct cagaaggtga ccaagttcat gctgtgtgct ggacgctgga cagggggcaa 660

aagcacctgc tcgggtgatt ctgggggccc acttgtctgt aatggtgtgc ttcaaggtat 720

cacgtcatgg ggcagtgaac catgtgccct gcccgaaagg ccttccctgt acaccaaggt 780

ggtgcattac cggaagtgga tcaaggacac catcgtggcc aacccctgag cacccctatc 840

aaccccctat tgtagtaaac ttggaacctt ggaaatgacc aggccaagac tcaagcctcc 900

ccagttctac tgacctttgt ccttaggtgt gaggtccagg gttgctagga aaagaaatca 960

gcagacacag gtgtagacca gagtgtttct taaatggtgt aattttgtcc tctctgtgtc 1020

ctggggaata ctggccatgc ctggagacat atcactcaat ttctctgagg acacagatag 1080

gatggggtgt ctgtgttatt tgtggggtac agagatgaaa gaggggtggg atccacactg 1140

agagagtgga gagtgacatg tgctggacac tgtccatgaa gcactgagca gaagctggag 1200

gcacaacgca ccagacactc acagcaagga tggagctgaa aacataaccc actctgtcct 1260

ggaggcactg ggaagcctag agaaggctgt gagccaagga gggagggtct tcctttggca 1320

tgggatgggg atgaagtaag gagagggact ggaccccctg gaagctgatt cactatgggg 1380

ggaggtgtat tgaagtcctc cagacaaccc tcagatttga tgatttccta gtagaactca 1440

cagaaataaa gagctgttat actgtg 1466

<210> 2
<211> 785
<212> DNA
<213> human

<400> 2
atgtgggacc tggttctctc catcgccttg tctgtggggt gcactggtgc cgtgcccctc 60

atccagtctc ggattgtggg aggctgggag tgtgagaagc attcccaacc ctggcaggtg 120

gctgtgtaca gtcatggatg ggcacactgt gggggtgtcc tggtgcaccc ccagtgggtg 180

ctcacagctg cccattgcct aaagaagaat agccaggtct ggctgggtcg gcacaacctg 240

tttgagcctg aagacacagg ccagagggtc cctgtcagcc acagcttccc acacccgctc 300

tacaatatga gccttctgaa gcatcaaagc cttagaccag atgaagactc cagccatgac 360

ctcatgctgc tccgcctgtc agagcctgcc aagatcacag tgttgtgaag gtcctgggcc 420

tgcccaccca ggagccagca ctggggacca cctgctacgc ctcaggctgg ggcagcatcg 480

aaccagagga gttcttgcgc cccaggagtc ttcagtgtgt gagcctccat ctcctgtcca 540

atgacatgtg tgctagagct tactctgaga aggtgacaga gttcatgttg tgtgctgggc 600

tctggacagg tggtaaagac acttgtgggg gtgattctgg gggtccactt gtctgtaatg 660

gggtgcttca aggtatcaca tcatggggcc ctgagccatg tgccctgcct gaaaagcctg 720

ctgtgtacac caaggtggtg cattaccgga agtggatcaa ggacaccatc gcagccaacc 780

cctga 785

<210> 3
<211> 503
<212> DNA
<213> human

<400> 3
gacagcggct tccttgatcc ttgccacccg cgactgaaca ccgacagcag cagcctcacc 60

atgaagttgc tgatggtcct catgctggcg gccctctccc agcactgcta cgcaggctct 120

ggctgcccct tattggagaa tgtgatttcc aagacaatca atccacaagt gtctaagact 180

gaatacaaag aacttcttca agagttcata gacgacaatg ccactacaaa tgccatagat 240

gaattgaagg aatgttttct taaccaaacg gatgaaactc tgagcaatgt tgaggtgttt 300

atgcaattaa tatatgacag cagtctttgt gatttatttt aactttctgc aagacctttg 360

gctcacagaa ctgcagggta tggtgagaaa ccaactacgg attgctgcaa accacacctt 420

ctctttctta tgtcttttta ctacaaacta caagacaatt gttgaaacct gctatacatg 480

tttattttaa taaattgatg gca 503

<210> 4
<211> 2560
<212> DNA
<213> human

<400> 4

```
gaattccggc cagaagaaat ctggcctcgg aacacgccat tctccgcgcc gcttccaata      60
accactaaca tccctaacga gcatccgagc cgagggctct gctcggaaat cgtcctggcc     120
caactcggcc cttcgagctc tcgaagatta ccgcatctat tttttttttc tttttttttct    180
tttcctagcg cagataaagt gagcccggaa agggaaggag gggcgggga caccattgcc     240
ctgaaagaat aaataagtaa ataaacaaac tggctcctcg ccgcagctgg acgcggtcgg     300
ttgagtccag gttgggtcgg acctgaaccc ctaaaagcgg aaccgcctcc cgccctcgcc     360
atcccggagc tgagtcgccg gcggcggtgg ctgctgccag acccggagtt tcctctttca     420
ctggatggag ctgaactttg ggcggccaga gcagcacagc tgtccgggga tcgctgcacg     480
ctgagctccc tcggcaagac ccagcggcgg ctcgggattt ttttgggggg gcggggacca     540
gccccgcgcc ggcaccatgt tcctggcgac cctgtacttc gcgctgccgc tcttggactt     600
gctcctgtcg gccgaagtga gcggcggaga ccgcctggat tgcgtgaaag ccagtgatca     660
gtgcctgaag gagcagagct gcagcaccaa gtaccgcacg ctaaggcagt gcgtggcggg     720
caaggagacc aacttcagcc tggcatccgg cctggaggcc aaggatgagt gccgcagcgc     780
catggaggcc ctgaagcaga agtcgctcta caactgccgc tgcaagcggg gtatgaagaa     840
ggagaagaac tgcctgcgca tttactggag catgtaccag agcctgcagg gaaatgatct     900
gctggaggat tccccatatg aaccagttaa cagcagattg tcagatatat tccgggtggt     960
cccattcata tcagatgttt ttcagcaagt ggagcacatt cccaaaggga acaactgcct    1020
ggatgcagcg aaggcctgca acctcgacga catttgcaag aagtacaggt cggcgtacat    1080
caccccgtgc accaccagcg tgtccaacga tgtctgcaac cgccgcaagt gccacaaggc    1140
cctccggcag ttctttgaca aggtcccggc caagcacagc tacggaatgc tcttctgctc    1200
ctgccgggac atcgcctgca cagagcggag gcgacagacc atcgtgcctg tgtgctccta    1260
tgaagagagg gagaagccca actgtttgaa tttgcaggac tcctgcaaga cgaattacat    1320
ctgcagatct cgccttgcgg attttttttac caactgccag ccagagtcaa ggtctgtcag    1380
cagctgtcta aaggaaaact acgctgactg cctcctcgcc tactcggggc ttattggcac    1440
agtcatgacc cccaactaca tagactccag tagcctcagt gtggccccat ggtgtgactg    1500
cagcaacagt gggaacgacc tagaagagtg cttgaaattt ttgaatttct tcaaggacaa    1560
tacatgtctt aaaaatgcaa ttcaagcctt tggcaatggc tccgatgtga ccgtgtggca    1620
gccagccttc ccagtacaga ccaccactgc cactaccacc actgccctcc gggttaagaa    1680
caagcccctg ggccagcag ggtctgagaa tgaaattccc actcatgttt tgccaccgtg     1740
tgcaaattta caggcacaga agctgaaatc caatgtgtcg ggcaatacac acctctgtat    1800
ttccaatggt aattatgaaa aagaaggtct cggtgcttcc agccacataa ccacaaaatc    1860
aatggctgct cctccaagct gtggtctgag cccactgctg gtcctggtgg taaccgctct    1920
gtccacccta ttatctttaa cagaaacatc atagctgcat taaaaaaata caatatggac    1980
```

atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160
aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 5
<211> 2560
<212> DNA
<213> human

<400> 5
gaattccggc cagaagaaat ctggcctcgg aacacgccat tctccgcgcc gcttccaata 60
accactaaca tccctaacga gcatccgagc cgagggctct gctcggaaat cgtcctggcc 120
caactcggcc cttcgagctc tcgaagatta ccgcatctat tttttttttc ttttttttct 180
tttcctagcg cagataaagt gagcccggaa agggaaggag ggggcgggga caccattgcc 240
ctgaaagaat aaataagtaa ataaacaaac tggctcctcg ccgcagctgg acgcggtcgg 300
ttgagtccag gttgggtcgg acctgaaccc ctaaaagcgg aaccgcctcc cgccctcgcc 360
atcccggagc tgagtcgccg gcggcggtgg ctgctgccag acccggagtt tcctctttca 420
ctggatggag ctgaactttg ggcggccaga gcagcacagc tgtccgggga tcgctgcacg 480
ctgagctccc tcggcaagac ccagcggcgg ctcgggattt ttttgggggg gcggggacca 540
gccccgcgcc ggcaccatgt tcctggcgac cctgtacttc gcgctgccgc tcttggactt 600
gctcctgtcg gccgaagtga gcggcggaga ccgcctggat tgcgtgaaag ccagtgatca 660
gtgcctgaag gagcagagct gcagcaccaa gtaccgcacg ctaaggcagt gcgtggcggg 720
caaggagacc aacttcagcc tggcatccgg cctggaggcc aaggatgagt gccgcagcgc 780
catggaggcc ctgaagcaga agtcgctcta caactgccgc tgcaagcggg gtatgaagaa 840
ggagaagaac tgcctgcgca tttactggag catgtaccag agcctgcagg gaaatgatct 900
gctggaggat tccccatatg aaccagttaa cagcagattg tcagatatat tccgggtggt 960
cccattcata tcagatgttt ttcagcaagt ggagcacatt cccaaaggga acaactgcct 1020
ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260

tgaagagagg gagaagccca actgtttgaa tttgcaggac tcctgcaaga cgaattacat 1320
ctgcagatct cgccttgcgg attttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg gggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160
aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 6
<211> 2439
<212> DNA
<213> human

<400> 6
cagcacccag ctccccgcca ccgccatggt ccccgacacc gcctgcgttc ttctgctcac 60
cctggctgcc ctcggcgcgt ccggacaggg ccagagcccg ttgggctcag acctgggccc 120
gcagatgctt cgggaactgc aggaaaccaa cgcggcgctg caggacgtgc gggactggct 180
gcggcagcag gtcagggaga tcacgttcct gaaaaacacg gtgatggagt gtgacgcgtg 240
cgggatgcag cagtcagtac gcaccggcct acccagcgtg cggcccctgc tccactgcgc 300
gcccggcttc tgcttccccg gcgtggcctg catccagacg gagagcggcg gccgctgcgg 360
cccctgcccc gcgggcttca cgggcaacgg ctcgcactgc accgacgtca acgagtgcaa 420
cgcccacccc tgcttccccc gagtccgctg tatcaacacc agcccggggt tccgctgcga 480

```
ggcttgcccg ccggggtaca gcggccccac ccaccagggc gtggggctgg ctttcgccaa     540
ggccaacaag caggtttgca cggacatcaa cgagtgtgag accgggcaac ataactgcgt     600
ccccaactcc gtgtgcatca acacccgggg ctccttccag tgcggcccgt gccagcccgg     660
cttcgtgggc gaccaggcgt ccggctgcca gcgcggcgca cagcgcttct gccccgacgg     720
ctcgcccagc gagtgccacg agcatgcaga ctgcgtccta gagcgcgatg gctcgcggtc     780
gtgcgtgtgt cgcgttggct gggccggcaa cgggatcctc tgtggtcgcg acactgacct     840
agacggcttc ccggacgaga agctgcgctg cccggagccg cagtgccgta aggacaactg     900
cgtgactgtg cccaactcag ggcaggagga tgtggaccgc gatggcatcg gagacgcctg     960
cgatccggat gccgacgggg acggggtccc caatgaaaag gacaactgcc cgctggtgcg    1020
gaacccagac cagcgcaaca cggacgagga caagtggggc gatgcgtgcg acaactgccg    1080
gtcccagaag aacgacgacc aaaaggacac agaccaggac ggccggggcg atgcgtgcga    1140
cgacgacatc gacggcgacc ggatccgcaa ccaggccgac aactgcccta gggtacccaa    1200
ctcagaccag aaggacagtg atggcgatgg tataggggat gcctgtgaca actgtcccca    1260
gaagagcaac ccggatcagg cggatgtgga ccacgacttt gtgggagatg cttgtgacag    1320
cgatcaagac caggatggag acggacatca ggactctcgg gacaactgtc ccacggtgcc    1380
taacagtgcc caggaggact cagaccacga tggccagggt gatgcctgcg acgacgacga    1440
cgacaatgac ggagtccctg acagtcggga caactgccgc ctggtgccta accccggcca    1500
ggaggacgcg gacagggacg gcgtgggcga cgtgtgccag gacgactttg atgcagacaa    1560
ggtggtagac aagatcgacg tgtgtccgga gaacgctgaa gtcacgctca ccgacttcag    1620
ggccttccag acagtcgtgc tggacccgga gggtgacgcg cagattgacc ccaactgggt    1680
ggtgctcaac cagggaaggg agatcgtgca gacaatgaac agcgacccag gcctggctgt    1740
gggttacact gccttcaatg gcgtggactt cgagggcacg ttccatgtga acacggtcac    1800
ggatgacgac tatgcgggct tcatctttgg ctaccaggac agctccagct tctacgtggt    1860
catgtggaag cagatggagc aaacgtattg gcaggcgaac cccttccgtg ctgtggccga    1920
gcctggcatc caactcaagg ctgtgaagtc ttccacaggc cccggggaac agctgcggaa    1980
cgctctgtgg catacaggag acacagagtc ccaggtgcgg ctgctgtgga aggacccgcg    2040
aaacgtgggt tggaaggaca agaagtccta tcgttggttc ctgcagcacc ggccccaagt    2100
gggctacatc agggtgcgat tctatgaggg ccctgagctg gtggccgaca gcaacgtggt    2160
cttggacaca accatgcggg gtggccgcct gggggtcttc tgcttctccc aggagaacat    2220
catctgggcc aacctgcgtt accgctgcaa tgacaccatc ccagaggact atgagaccca    2280
tcagctgcgg caagcctagg gaccagggtg aggacccgcc ggatgacagc caccctcacc    2340
gcggctggat gggggctctg cacccagccc aaggggtggc cgtcctgagg gggaagtgag    2400
aagggctcag agaggacaaa ataaagtgtg tgtgcaggg                            2439
```

<210> 7

<211> 2439
<212> DNA
<213> human

<400> 7
cagcacccag ctccccgcca ccgccatggt ccccgacacc gcctgcgttc ttctgctcac 60
cctggctgcc ctcggcgcgt ccggacaggg ccagagcccg ttgggctcag acctgggccc 120
gcagatgctt cgggaactgc aggaaaccaa cgcggcgctg caggacgtgc gggactggct 180
gcggcagcag gtcagggaga tcacgttcct gaaaaacacg gtgatggagt gtgacgcgtg 240
cgggatgcag cagtcagtac gcaccggcct acccagcgtg cggcccctgc tccactgcgc 300
gcccggcttc tgcttccccg gcgtggcctg catccagacg gagagcggcg gccgctgcgg 360
cccctgcccc gcgggcttca cgggcaacgg ctcgcactgc accgacgtca acgagtgcaa 420
cgcccacccc tgcttccccc gagtccgctg tatcaacacc agcccggggt tccgctgcga 480
ggcttgcccg ccggggtaca gcggccccac ccaccagggc gtggggctgg ctttcgccaa 540
ggccaacaag caggtttgca cggacatcaa cgagtgtgag accgggcaac ataactgcgt 600
ccccaactcc gtgtgcatca acacccgggg ctccttccag tgcggcccgt gccagcccgg 660
cttcgtgggc gaccaggcgt ccggctgcca gcgcggcgca cagcgcttct gccccgacgg 720
ctcgcccagc gagtgccacg agcatgcaga ctgcgtccta gagcgcgatg gctcgcggtc 780
gtgcgtgtgt cgcgttggct gggccggcaa cgggatcctc tgtggtcgcg acactgacct 840
agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440
cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920

gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct gggggtcttc tgcttctccc aggagaacat 2220
catctgggcc aacctgcgtt accgctgcaa tgacaccatc ccagaggact atgagaccca 2280
tcagctgcgg caagcctagg gaccagggtg aggacccgcc ggatgacagc caccctcacc 2340
gcggctggat gggggctctg cacccagccc aaggggtggc cgtcctgagg gggaagtgag 2400
aagggctcag agaggacaaa ataaagtgtg tgtgcaggg 2439

<210> 8
<211> 1699
<212> DNA
<213> human

<400> 8
aggtgagcgg ttgctcgtcg tcggggcggc cggcagcggc ggctccaggg cccagcatgc 60
gcgggggacc ccgcggccac catgtatgtg ggctatgtgc tggacaagga ttcgcccgtg 120
taccccggcc cagccaggcc agccagcctc ggcctgggcc cggcaaacta cggccccccg 180
gccccgcccc cggcgccccc gcagtacccc gacttctcca gctactctca cgtggagccg 240
gcccccgcgc ccccgacggc ctggggggcg ccccttccctg cgcccaagga cgactgggcc 300
gccgcctacg gcccgggccc cgcggcccct gccgccagcc cagcttcgct ggcattcggg 360
ccccctccag actttagccc ggtgccggcg ccccctgggc ccggcccggg cctcctggcg 420
cagcccctcg gggggcccggg cacaccgtcc tcgcccggag cgcagaggcc gacgccctac 480
gagtggatgc ggcgcagcgt ggcggccgga ggcggcggtg gcagcggtaa gactcggacc 540
aaggacaagt accgcgtggt ctacaccgac caccaacgcc tggagctgga gaaggagttt 600
cattacagcc gttacatcac aatccggcgg aaatcagagc tggctgccaa tctggggctc 660
actgaacggc aggtgaagat ctggttccaa aaccggcggg caaaggagcg caaagtgaac 720
aagaagaaac agcagcagca acagcccccca cagccgccga tggcccacga catcacggcc 780
accccagccg ggccatccct ggggggcctg tgtcccagca acaccagcct cctggccacc 840
tcctctccaa tgcctgtgaa agaggagttt ctgccatagc cccatgccca gcctgtgcgc 900
cgggggacct ggggactcgg gtgctgggag tgtggctcct gtgggcccag gaggtctggt 960
ccgagtctca gccctgacct tctgggacat ggtggacagt cacctatcca ccctctgcat 1020
ccccttggcc cattgtgtgc agtaagcctg ttggataaag accttccagc tcctgtgttc 1080
tagacctctg gggataaggg gagtccaggg tggatgatct caatctcccg tgggcatctc 1140
aagccccaaa tggttggggg aggggcctag acaaggctcc aggccccacc tcctcctcca 1200
tacgttcaga ggtgcagctg gaggcctgtg tggggaccac actgatcctg gagaaaaggg 1260

atggagctga aaaagatgga atgcttgcag agcatgacct gaggagggag gaacgtggtc 1320
aactcacacc tgcctcttct gcagcctcac ctctacctgc ccccatcata agggcactga 1380
gcccttccca ggctggatac taagcacaaa gcccatagca ctgggctctg atggctgctc 1440
cactgggtta cagaatcaca gccctcatga tcattctcag tgagggctct ggattgagag 1500
ggaggccctg ggaggagaga agggggcaga gtcttcccta ccaggtttct acacccccgc 1560
caggctgccc atcagggccc agggagcccc cagaggactt tattcggacc aagcagagct 1620
cacagctgga caggtgttgt atatagagtg gaatctcttg gatgcagctt caagaataaa 1680
tttttcttct cttttcaaa 1699

<210> 9
<211> 4098
<212> DNA
<213> human

<400> 9
ggtggcctct gtggccgtcc aggctagcgg cggcccgcag gcggcgggga gaaagactct 60
ctcacctggt cttgcggctg tggccaccgc cggccagggg tgtggagggc gtgctgccgg 120
agacgtccgc cgggctctgc agttccgccg ggggtcgggc agctatggag ccgcggccca 180
cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300
ctgcgcccgc gagccgggac ggcggcgggg tccgcgatga gggccccgcg gcggccgggg 360
acgggctggg cagacccttg ggcccaccc cgagccagag ccgtttccag gtggacctgg 420
tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540
gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380

tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atcttttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct tttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420

tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840
tcagtgccta gtgtagtaac ctgaaatctt caatgacaca ttaacatcac aatggcgaat 3900
ggtgactttt ctttcacgat ttcattaatt tgaaagcaca caggaaagct tgctccattg 3960
ataacgtgta tggagacttc ggttttagtc aattccatat ctcaatctta atggtgattc 4020
ttctctgttg aactgaagtt tgtgagagta gttttccttt gctacttgaa tagcaataaa 4080
agcgtgttaa ctttttgg 4098

<210> 10
<211> 4098
<212> DNA
<213> human

<400> 10
ggtggcctct gtggccgtcc aggctagcgg cggcccgcag gcggcgggga gaaagactct 60
ctcacctggt cttgcggctg tggccaccgc cggccagggg tgtggagggc gtgctgccgg 120
agacgtccgc cgggctctgc agttccgccg ggggtcgggc agctatggag ccgcggccca 180
cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300
ctgcgcccgc gagccgggac ggcggcgggg tccgcgatga gggccccgcg gcggccgggg 360
acgggctggg cagacccttg gggcccaccc cgagccagag ccgtttccag gtggacctgg 420
tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540
gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080

ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atcttttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120

ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct ttttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840
tcagtgccta gtgtagtaac ctgaaatctt caatgacaca ttaacatcac aatggcgaat 3900
ggtgactttt ctttcacgat ttcattaatt tgaaagcaca caggaaagct tgctccattg 3960
ataacgtgta tggagacttc ggttttagtc aattccatat ctcaatctta atggtgattc 4020
ttctctgttg aactgaagtt tgtgagagta gttttccttt gctacttgaa tagcaataaa 4080
agcgtgttaa ctttttgg 4098

<210> 11
<211> 3311
<212> DNA
<213> human

<400> 11
tgctaatgct tttggtacaa atggatgtgg aatataattg aatattttct tgtttaaggg 60
gagcatgaag aggtgttgag gttatgtcaa gcatctggca cagctgaagg cagatggaaa 120
tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840

ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520
aagcaagtgt gtttcagcag aacatcctcg ggaggctcat ttgtggcttc tgatgtccca 2580
aatgctccca tacctgatct cttcccacct ggccaaatca ccgacctgaa ggcggaaatt 2640
cacgggggca gtctcattaa tctgacttgg acagctcctg gggatgatta tgaccatgga 2700
acagctcaca agtatatcat tcgaataagt acaagtattc ttgatctcag agacaagttc 2760
aatgaatctc ttcaagtgaa tactactgct ctcatcccaa aggaagccaa ctctgaggaa 2820
gtctttttgt ttaaaccaga aaacattact tttgaaaatg gcacagatct tttcattgct 2880

attcaggctg ttgataaggt cgatctgaaa tcagaaatat ccaacattgc acgagtatct 2940
ttgtttattc ctccacagac tccgccagag acacctagtc ctgatgaaac gtctgctcct 3000
tgtcctaata ttcatatcaa cagcaccatt cctggcattc acattttaaa aattatgtgg 3060
aagtggatag gagaactgca gctgtcaata gcctagggct gaatttttgt cagataaata 3120
aaataaatca ttcatccttt ttttgattat aaaattttct aaaatgtatt ttagacttcc 3180
tgtagggggc gatatactaa atgtatatag tacatttata ctaaatgtat tcctgtaggg 3240
ggcgatatac taaatgtatt ttagacttcc tgtagggggc gataaaataa aatgctaaac 3300
aactgggtaa a 3311

<210> 12
<211> 3311
<212> DNA
<213> human

<400> 12
tgctaatgct tttggtacaa atggatgtgg aatataattg aatattttct tgtttaaggg 60
gagcatgaag aggtgttgag gttatgtcaa gcatctggca cagctgaagg cagatggaaa 120
tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320

```
aatcgactga atcaagcagg ccagcttttc ctgctgcaga cagttgagct ggggtcctgg   1380
gttgggatgg tgacatttga cagtgctgcc catgtacaaa gtgaactcat acagataaac   1440
agtggcagtg acagggacac actcgccaaa agattacctg cagcagcttc aggagggacg   1500
tccatctgca gcgggcttcg atcggcattt actgtgatta ggaagaaata tccaactgat   1560
ggatctgaaa ttgtgctgct gacggatggg gaagacaaca ctataagtgg gtgctttaac   1620
gaggtcaaac aaagtggtgc catcatccac acagtcgctt tggggccctc tgcagctcaa   1680
gaactagagg agctgtccaa aatgacagga ggtttacaga catatgcttc agatcaagtt   1740
cagaacaatg gcctcattga tgcttttggg gccctttcat caggaaatgg agctgtctct   1800
cagcgctcca tccagcttga gagtaaggga ttaaccctcc agaacagcca gtggatgaat   1860
ggcacagtga tcgtggacag caccgtggga aaggacactt tgtttcttat cacctggaca   1920
acgcagcctc cccaaatcct tctctgggat cccagtggac agaagcaagg tggctttgta   1980
gtggacaaaa acaccaaaat ggcctacctc caaatcccag gcattgctaa ggttggcact   2040
tggaaataca gtctgcaagc aagctcacaa accttgaccc tgactgtcac gtcccgtgcg   2100
tccaatgcta ccctgcctcc aattacagtg acttccaaaa cgaacaagga caccagcaaa   2160
ttccccagcc ctctggtagt ttatgcaaat attcgccaag gagcctcccc aattctcagg   2220
gccagtgtca cagccctgat tgaatcagtg aatggaaaaa cagttacctt ggaactactg   2280
gataatggag caggtgctga tgctactaag gatgacggtg tctactcaag gtatttcaca   2340
acttatgaca cgaatggtag atacagtgta aaagtgcggg ctctgggagg agttaacgca   2400
gccagacgga gagtgatacc ccagcagagt ggagcactgt acatacctgg ctggattgag   2460
aatgatgaaa tacaatggaa tccaccaaga cctgaaatta ataaggatga tgttcaacac   2520
aagcaagtgt gtttcagcag aacatcctcg ggaggctcat ttgtggcttc tgatgtccca   2580
aatgctccca tacctgatct cttcccacct ggccaaatca ccgacctgaa ggcggaaatt   2640
cacgggggca gtctcattaa tctgacttgg acagctcctg gggatgatta tgaccatgga   2700
acagctcaca agtatatcat tcgaataagt acaagtattc ttgatctcag agacaagttc   2760
aatgaatctc ttcaagtgaa tactactgct ctcatcccaa aggaagccaa ctctgaggaa   2820
gtctttttgt ttaaaccaga aaacattact tttgaaaatg gcacagatct tttcattgct   2880
attcaggctg ttgataaggt cgatctgaaa tcagaaatat ccaacattgc acgagtatct   2940
ttgtttattc ctccacagac tccgccagag acacctagtc ctgatgaaac gtctgctcct   3000
tgtcctaata ttcatatcaa cagcaccatt cctggcattc acattttaaa aattatgtgg   3060
aagtggatag gagaactgca gctgtcaata gcctagggct gaatttttgt cagataaata   3120
aaataaatca ttcatccttt ttttgattat aaaattttct aaaatgtatt ttagacttcc   3180
tgtagggggc gatatactaa atgtatatag tacatttata ctaaatgtat tcctgtaggg   3240
ggcgatatac taaatgtatt ttagacttcc tgtagggggc gataaaataa aatgctaaac   3300
aactgggtaa a                                                        3311
```

<210> 13
<211> 15720
<212> DNA
<213> human

<400> 13
caacccacac cgcccctgcc agccaccatg gggctgccac tagcccgcct ggcggctgtg 60

tgcctggccc tgtctttggc agggggctcg gagctccaga cagagggcag aacccgatac 120

cacggccgca acgtctgcag cacctggggc aacttccact acaagacctt cgacggggac 180

gtcttccgct tccccggcct ctgcgactac aacttcgcct ccgactgccg aggctcctac 240

aaggaatttg ctgtgcacct gaagcggggt ccgggccagg ctgaggcccc cgccggggtg 300

gagtccatcc tgctgaccat caaggatgac accatctacc tcacccgcca cctggctgtg 360

cttaacgggg ccgtggtcag caccccgcac tacagccccg ggctgctcat tgagaagagc 420

gatgcctaca ccaaagtcta ctcccgcgcc ggcctcaccc tcatgtggaa ccgggaggat 480

gcactcatgc tggagctgga cactaagttc cggaaccaca cctgtggcct ctgcggggac 540

tacaacggcc tgcagagcta ttcagaattc ctctctgacg gcgtgctctt cagtcccctg 600

gagtttggga acatgcagaa gatcaaccag cccgatgtgg tgtgtgagga tcccgaggag 660

gaggtggccc ccgcatcctg ctccgagcac cgcgccgagt gtgagaggct gctgaccgcc 720

gaggccttcg cggactgtca ggacctggtg ccgctggagc cgtatctgcg cgcctgccag 780

caggaccgct gccggtgccc gggcggtgac acctgcgtct gcagcaccgt ggccgagttc 840

tcccgccagt gctcccacgc cggcggccgg cccgggaact ggaggaccgc cacgctctgc 900

cccaagacct gccccgggaa cctggtgtac ctggagagcg gctcgccctg catggacacc 960

tgctcacacc tggaggtgag cagcctgtgc gaggagcacc gcatggacgg ctgtttctgc 1020

ccagaaggca ccgtatatga cgacatcggg gacagtggct gcgttcctgt gagccagtgc 1080

cactgcaggc tgcacggaca cctgtacaca ccgggccagg agatcaccaa tgactgcgag 1140

cagtgtgtct gtaacgctgg ccgctgggtg tgcaaagacc tgccctgccc cggcacctgt 1200

gccctggaag gcggctccca catcaccacc ttcgatggga agacgtacac cttccacggg 1260

gactgctact atgtcctggc caagggtgac cacaacgatt cctacgctct cctgggcgag 1320

ctggccccct gtggctccac agacaagcag acctgcctga agacggtggt gctgctggct 1380

gacaagaaga agaatgcggt ggtcttcaag tccgatggca gtgtactgct caaccagctg 1440

caggtgaacc tgccccacgt gaccgcgagc ttctctgtct tccgcccgtc ttcctaccac 1500

atcatggtga gcatggccat tggcgtccgg ctgcaggtgc agctggcccc agtcatgcaa 1560

ctctttgtga cactggacca ggcctcccag gggcaggtgc agggcctctg cgggaacttc 1620

aacggcctgg aaggtgacga cttcaagacg gccagcgggc tggtggaggc cacgggggcc 1680

ggctttgcca acacctggaa ggcacagtca acctgccatg acaagctgga ctggttggac 1740

gatccctgct ccctgaacat cgagagcgcc aactacgccg agcactggtg ctccctcctg 1800

aagaagacag agacccccctt tggcaggtgc cactcggctg tggaccctgc tgagtattac 1860

aagaggtgca aatatgacac gtgtaactgt cagaacaatg aggactgcct gtgcgccgcc 1920
ctgtcctcct acgcgcgcgc ctgcaccgcc aagggcgtca tgctgtgggg ctggcgggag 1980
catgtctgca acaaggatgt gggctcctgc cccaactcgc aggtcttcct gtacaacctg 2040
accacctgcc agcagacctg ccgctccctc tccgaggccg acagccactg tctcgagggc 2100
tttgcgcctg tggacggctg cggctgccct gaccacacct tcctggacga gaagggccgc 2160
tgcgtacccc tggccaagtg ctcctgttac caccgcggtc tctacctgga ggcgggggat 2220
gtggtcgtca ggcaggaaga acgatgtgtg tgccgggatg ggcggctgca ctgtaggcag 2280
atccggctga tcggccagag ctgcacggcc ccaaagatcc acatggactg cagcaacctg 2340
actgcactgg ccacctcgaa gccccgagcc ctcagctgcc agacgctggc cgccggctat 2400
taccacacag agtgtgtcag tggctgtgtg tgccccgacg ggctgatgga tgacggccgg 2460
ggtggctgcg tggtggagaa ggaatgccct tgcgtccata acaacgacct gtattcttcc 2520
ggcgccaaga tcaaggtgga ctgcaatacc tgcacctgca agagaggacg ctgggtgtgc 2580
acccaggctg tgtgccatgg cacctgctcc atttacggga gtggccacta catcaccttt 2640
gatgggaagt actacgactt tgacggacac tgctcctacg tggctgttca ggactactgc 2700
ggccagaact cctcactggg ctcattcagc atcatcaccg agaacgtccc ctgtggcact 2760
acgggcgtca cctgctccaa ggccatcaag atcttcatgg ggaggacgga gctgaagttg 2820
gaagacaagc accgtgtggt gatccagcgt gatgagggtc accacgtggc ctacaccacg 2880
cgggaggtgg gccagtacct ggtggtggag tccagcacgg gcatcatcgt catctgggac 2940
aagaggacca ccgtgttcat caagctggct ccctcctaca agggcaccgt gtgtggcctg 3000
tgtgggaact ttgaccaccg ctccaacaac gacttcacca cgcgggacca catggtggtg 3060
agcagcgagc tggacttcgg gaacagctgg aaggaggccc ccacctgccc agatgtgagc 3120
accaaccccg agccctgcag cctgaacccg caccgccgct cctgggccga gaagcagtgc 3180
agcatcctca aaagcagcgt gttcagcatc tgccacagca aggtggaccc caagcccttc 3240
tacgaggcct gtgtgcacga ctcgtgctcc tgtgacacgg gtggggactg tgagtgcttc 3300
tgctctgccg tggcctccta cgcccaggag tgtaccaaag agggggcctg cgtgttctgg 3360
aggacgccgg acctgtgccc catattctgc gactactaca accctccgca tgagtgtgag 3420
tggcactatg agccatgtgg gaaccggagc ttcgagacct gcaggaccat caacggcatc 3480
cactccaaca tctccgtgtc ctacctggag ggctgctacc cccggtgccc caaggacagg 3540
cccatctatg aggaggatct gaagaagtgt gtcactgcag acaagtgtgg ctgctatgtc 3600
gaggacaccc actacccacc tggagcatcg gttcccaccg aggagacctg caagtcctgc 3660
gtgtgtacca actcctccca agtcgtctgc aggccggagg aaggaaagat tcttaaccag 3720
acccaggatg gcgccttctg ctactgggag atctgtggcc ccaacgggac ggtggagaag 3780
cacttcaaca tctgttccat tacgacacgc ccgtccaccc tgaccacctt caccaccatc 3840
accctcccca ccacccccac ctccttcacc actaccacca ccaccaccac cccgacctcc 3900

agcacagttt tatcaacaac tccgaagctg tgctgcctct ggtctgactg gatcaatgag 3960
gaccacccca gcagtggcag cgacgacggt gaccgagaac catttgatgg ggtctgcggg 4020
gcccctgagg acatcgagtg caggtcggtc aaggatcccc acctcagctt ggagcagcat 4080
ggccagaagg tgcagtgtga tgtctctgtt gggttcattt gcaagaatga agaccagttt 4140
ggaaatggac catttggact gtgttacgac tacaagatac gtgtcaattg ttgctggccc 4200
atggataagt gtatcaccac tcccagccct ccaactacca ctcccagccc tccaccaacc 4260
acgacgacca cccttccacc aaccaccacc cccagccctc caaccaccac cacaaccacc 4320
cctccaccaa ccaccacccc cagccctcca ataaccacca cgaccacccc tctaccaacc 4380
accactccca gccctccaat aagcaccaca accacccctc caccaaccac cactcccagc 4440
cctccaacca ccactcccag ccctccaacc accactccca gccctccaac aaccaccaca 4500
accacccctc caccaaccac cactcccagc cctccaatga ctacgcccat cactccacca 4560
gccagcacta ccacccttcc accaaccacc actcccagcc ctccaacaac caccacaacc 4620
acccctccac caaccaccac tcccagtcct ccaacgacta cgcccatcac tccaccaacc 4680
agcactacta cccttccacc aaccaccact cccagccctc caccaaccac cacaaccacc 4740
cctccaccaa ccaccactcc cagccctcca acaaccacca ctcccagtcc tccaacaatc 4800
accacaacca cccctccacc aaccaccact cccagccctc caacaacgac cacaaccacc 4860
cctccaccaa ccaccactcc cagccctcca acgactacac ccatcactcc accaaccagc 4920
actaccaccc ttccaccaac caccactccc agccctccac caaccaccac aaccacccct 4980
ccaccaacca ccactcccag ccctccaaca accaccactc ccagccctcc aataaccacc 5040
acaaccaccc ctccaccaac caccactccc agctctccaa taaccaccac tcccagccct 5100
ccaacaacca ccatgaccac cccttcacca accaccaccc ccagctctcc aataaccacc 5160
acaaccaccc cttcctcaac taccactccc agccctccac caaccaccat gaccacccct 5220
tcaccaacca ccactcccag ccctccaaca accaccatga ccacccttcc accaaccacc 5280
acttccagcc ctctaacaac tactcctcta cctccatcaa taactcctcc tacattttca 5340
ccattctcaa cgacaacccc tactacccca tgcgtgcctc tctgcaattg gactggctgg 5400
ctggattctg gaaaacccaa ctttcacaaa ccaggtggag acacagaatt gattggagac 5460
gtctgtggac caggctgggc agctaacatc tcttgcagag ccaccatgta tcctgatgtt 5520
cccattggac agcttggaca aacagtggtg tgtgatgtct ctgtggggct gatatgcaaa 5580
aatgaagacc aaaagccagg tggggtcatc cctatggcct tctgcctcaa ctacgagatc 5640
aacgttcagt gctgtgagtg tgtcacccaa cccaccacca tgacaaccac caccacagag 5700
aacccaactc cgccaaccac gacacccatc accaccacca ctacggtgac ccccaacccca 5760
acacccaccg gcacacagac cccaaccacg acacccatca ccaccaccac tacggtgacc 5820
ccaaccccaa cacccaccgg cacacagacc ccaaccacga cacccatcac caccaccact 5880
acggtgaccc caaccccaac acccaccggc acacagaccc caaccacgac acccatcacc 5940

accaccacta cggtgacccc aaccccaaca cccaccggca cacagacccc aaccacgaca 6000
cccatcacca ccaccactac ggtgacccca accccaacac ccaccggcac acagacccca 6060
accacgacac ccatcaccac caccactacg gtgaccccaa ccccaacacc caccggcaca 6120
cagaccccaa ccacgacacc catcaccacc accactacgg tgaccccaac cccaacaccc 6180
accggcacac agaccccaac cacgacaccc atcaccacca ccactacggt gaccccaacc 6240
ccaacaccca ccggcacaca gaccccaacc acgacaccca tcaccaccac cactacggtg 6300
accccaaccc caacacccac cggcacacag accccaacca cgacacccat caccaccacc 6360
actacggtga ccccaacccc aacacccacc ggcacacaga ccccaaccac gacacccatc 6420
accaccacca ctacggtgac cccaacccca acacccaccg gcacacagac cccaaccacg 6480
acacccatca ccaccaccac tacggtgacc ccaaccccaa cacccaccgg cacacagacc 6540
ccaaccacga cacccatcac caccaccact acggtgaccc caaccccaac acccaccggc 6600
acacagaccc caaccacgac acccatcacc accaccacta cggtgacccc aaccccaaca 6660
cccaccggca cacagacccc aaccacgaca cccatcacca ccaccactac ggtgacccca 6720
accccaacac ccaccggcac acagacccca accacgacac ccatcaccac caccactacg 6780
gtgaccccaa ccccaacacc caccggcaca cagaccccaa ccacgacacc catcaccacc 6840
accactacgg tgaccccaac cccaacaccc accggcacac agaccccaac cacgacaccc 6900
atcaccacca ccactacggt gaccccaacc ccaacaccca ccggcacaca gaccccaacc 6960
acgacaccca tcaccaccac cactacggtg accccaaccc caacacccac cggcacacag 7020
accccaacca cgacacccat caccaccacc actacggtga ccccaacccc aacacccacc 7080
ggcacacaga ccccaaccac gacacccatc accaccacca ctacggtgac cccaacccca 7140
acacccaccg gcacacagac cccaaccacg acacccatca ccaccaccac tacggtgacc 7200
ccaaccccaa cacccaccgg cacacagacc ccaaccacga cacccatcac caccaccact 7260
acggtgaccc caaccccaac acccaccggc acacagaccc caaccacgac acccatcacc 7320
accaccacta cggtgacccc aaccccaaca cccaccggca cacagacccc aaccacgaca 7380
cccatcacca ccaccactac ggtgacccca accccaacac ccaccggcac acagacccca 7440
accacgacac ccatcaccac caccactacg gtgaccccaa ccccaacacc caccggcaca 7500
cagaccccaa ccacgacacc catcaccacc accactacgg tgaccccaac cccaacaccc 7560
accggcacac agaccccaac cacgacaccc atcaccacca ccactacggt gaccccaacc 7620
ccaacaccca ccggcacaca gaccccaacc acgacaccca tcaccaccac cactacggtg 7680
accccaaccc caacacccac cggcacacag accccaacca cgacacccat caccaccacc 7740
actacggtga ccccaacccc aacacccacc ggcacacaga ccccaaccac gacacccatc 7800
accaccacca ctacggtgac cccaacccca acacccaccg gcacacagac cccaaccacg 7860
acacccatca ccaccaccac tacggtgacc ccaaccccaa cacccaccgg cacacagacc 7920
ccaaccacga cacccatcac caccaccact acggtgaccc caaccccaac acccaccggc 7980

acacagaccc caaccacgac acccatcacc accaccacta cggtgacccc aaccccaaca 8040
cccaccggca cacagacccc aaccacgaca cccatcacca ccaccactac ggtgacccca 8100
accccaacac ccaccggcac acagacccca accacgacac ccatcaccac caccactacg 8160
gtgaccccaa ccccaacacc caccggcaca cagaccccaa ccacgacacc catcaccacc 8220
accactacgg tgaccccaac cccaacaccc accggcacac agaccccaac cacgacaccc 8280
atcaccacca ccactacggt gaccccaacc ccaacaccca ccggcacaca gaccccaacc 8340
acgacaccca tcaccaccac cactacggtg accccaaccc caacacccac cggcacacag 8400
accccaacca cgacacccat caccaccacc actacggtga ccccaacccc aacacccacc 8460
ggcacacaga ccccaaccac gacacccatc accaccacca ctacggtgac cccaacccca 8520
acacccaccg gcacacagac cccaaccacg acacccatca ccaccaccac tacggtgacc 8580
ccaaccccaa cacccaccgg cacacagacc ccaaccacga cacccatcac caccaccact 8640
acggtgaccc caaccccaac acccaccggc acacagaccc caaccacgac acccatcacc 8700
accaccacta cggtgacccc aaccccaaca cccaccggca cacagacccc aaccacgaca 8760
cccatcacca ccaccactac ggtgacccca accccaacac ccaccggcac acagacccca 8820
accacgacac ccatcaccac caccactacg gtgaccccaa ccccaacacc caccggcaca 8880
cagaccccaa ccacgacacc catcaccacc accactacgg tgaccccaac cccaacaccc 8940
accggcacac agaccccaac cacgacaccc atcaccacca ccactacggt gaccccaacc 9000
ccaacaccca ccggcacaca gaccccaacc acgacaccca tcaccaccac cactacggtg 9060
accccaaccc caacacccac cggcacacag accccaacca cgacacccat caccaccacc 9120
actacggtga ccccaacccc aacacccacc ggcacacaga ccccaaccac gacacccatc 9180
accaccacca ctacggtgac cccaacccca acacccaccg gcacacagac cccaaccacg 9240
acacccatca ccaccaccac tacggtgacc ccaaccccaa cacccaccgg cacacagacc 9300
ccaaccacga cacccatcac caccaccact acggtgaccc caaccccaac acccaccggc 9360
acacagaccc caaccacgac acccatcacc accaccacta cggtgacccc aaccccaaca 9420
cccaccggca cacagacccc aaccacgaca cccatcacca ccaccactac ggtgacccca 9480
accccaacac ccaccggcac acagacccca accacgacac ccatcaccac caccactacg 9540
gtgaccccaa ccccaacacc caccggcaca cagaccccaa ccacgacacc catcaccacc 9600
accactacgg tgaccccaac cccaacaccc accggcacac agaccccaac cacgacaccc 9660
atcaccacca ccactacggt gaccccaacc ccaacaccca ccggcacaca gaccccaacc 9720
acgacaccca tcaccaccac cactacggtg accccaaccc caacacccac cggcacacag 9780
accccaacca cgacacccat caccaccacc actacggtga ccccaacccc aacacccacc 9840
ggcacacaga ccccaaccac gacacccatc accaccacca ctacggtgac cccaacccca 9900
acacccaccg gcacacagac cccaaccacg acacccatca ccaccaccac tacggtgacc 9960
ccaaccccaa cacccaccgg cacacagacc ccaaccacga cacccatcac caccaccact 10020

acggtgaccc caaccccaac acccaccggc acacagaccc caaccacgac acccatcacc 10080
accaccacta cggtgacccc aaccccaaca cccaccggca cacagacccc aaccacgaca 10140
cccatcacca ccaccactac ggtgacccca accccaacac ccaccggcac acagacccca 10200
accacgacac ccatcaccac caccactacg gtgaccccaa ccccaacacc caccggcaca 10260
cagaccccaa ccacgacacc catcaccacc accactacgg tgaccccaac cccaacaccc 10320
accggcacac agaccccaac cacgacaccc atcaccacca ccactacggt gaccccaacc 10380
ccaacaccca ccggcacaca gaccccaacc acgacaccca tcaccaccac cactacggtg 10440
accccaaccc caacacccac cggcacacag accccaacca cgacacccat caccaccacc 10500
actacggtga ccccaacccc aacacccacc ggcacacaga ccccaaccac gacacccatc 10560
accaccacca ctacggtgac cccaacccca acacccaccg gcacacagac cccaaccacg 10620
acacccatca ccaccaccac tacggtgacc ccaaccccaa cacccaccgg cacacagacc 10680
ccaaccacga cacccatcac caccaccact acggtgaccc caaccccaac acccaccggc 10740
acacagaccc caaccacgac acccatcacc accaccacta cggtgacccc aaccccaaca 10800
cccaccggca cacagacccc aaccacgaca cccatcacca ccaccactac ggtgacccca 10860
accccaacac ccaccggcac acagacccca accacgacac ccatcaccac caccactacg 10920
gtgaccccaa ccccaacacc caccggcaca cagaccccaa ccacgacacc catcaccacc 10980
accactacgg tgaccccaac cccaacaccc accggcacac agaccccaac cacgacaccc 11040
atcaccacca ccactacggt gaccccaacc ccaacaccca ccggcacaca gaccccaacc 11100
acgacaccca tcaccaccac cactacggtg accccaaccc caacacccac cggcacacag 11160
accccaacca cgacacccat caccaccacc actacggtga ccccaacccc aacacccacc 11220
ggcacacaga ccccaaccac gacacccatc accaccacca ctacggtgac cccaacccca 11280
acacccaccg gcacacagac cccaaccacg acacccatca ccaccaccac tacggtgacc 11340
ccaaccccaa cacccaccgg cacacagacc ccaaccacga cacccatcac caccaccact 11400
acggtgaccc caaccccaac acccaccggc acacagaccc caaccacgac acccatcacc 11460
accaccacta cggtgacccc aaccccaaca cccaccggca cacagacccc aaccacgaca 11520
cccatcacca ccaccactac ggtgacccca accccaacac ccaccggcac acagacccca 11580
accacgacac ccatcaccac caccactacg gtgaccccaa ccccaacacc caccggcaca 11640
cagaccccaa ccacgacacc catcaccacc accactacgg tgaccccaac cccaacaccc 11700
accggcacac agaccccaac cacgacaccc atcaccacca ccactacggt gaccccaacc 11760
ccaacaccca ccggcacaca gaccccaacc acgacaccca tcaccaccac cactacggtg 11820
accccaaccc caacacccac cggcacacag accccaacca cgacacccat caccaccacc 11880
actacggtga ccccaacccc aacacccacc ggcacacaga ccccaaccac gacacccatc 11940
accaccacca ctacggtgac cccaacccca acacccaccg gcacacagac cccaaccacg 12000
acacccatca ccaccaccac tacggtgacc ccaaccccaa cacccaccgg cacacagacc 12060

ccaaccacga cacccatcac caccaccact acggtgaccc caaccccaac acccaccggc 12120
acacagaccc caaccacgac acccatcacc accaccacta cggtgacccc aaccccaaca 12180
cccaccggca cacagacccc aaccacgaca cccatcacca ccaccactac ggtgacccca 12240
accccaacac ccaccggcac acagacccca accacgacac ccatcaccac caccactacg 12300
gtgaccccaa ccccaacacc caccggcaca cagaccccaa ccacgacacc catcaccacc 12360
accactacgg tgaccccaac cccaacaccc accggcacac agaccccaac cacgacaccc 12420
atcaccacca ccactacggt gaccccaacc ccaacaccca ccggcacaca gaccccaacc 12480
acgacaccca tcaccaccac cactacggtg accccaaccc caacacccac cggcacacag 12540
accccaacca cgacacccat caccaccacc actacggtga ccccaacccc aacacccacc 12600
ggcacacaga ccgggccccc cacccacaca agcacagcac cgattgctga gttgaccaca 12660
tccaatcctc cgcctgagtc ctcaacccct cagacctctc ggtccacctc ttcccctctc 12720
acggagtcaa ccacccttct gagtacccta ccacctgcca ttgagatgac cagcacggcc 12780
ccaccctcca cacccacggc acccacgacc acgagcggag gccacacact gtctccaccg 12840
cccagcacca ccacgtcccc tccaggcacc cccactcgcg gtaccacgac cgggtcatct 12900
tcagccccca cccccagcac tgtgcagacg accaccacca gtgcctggac cccaacgccg 12960
accccactct ccacacccag catcatcagg accacaggcc tgaggcccta cccttcctct 13020
gtgcttatct gctgtgtcct gaacgacacc tactacgcac caggtgagga ggtgtacaac 13080
ggcacatacg gagacacctg ttatttcgtc aactgctcac tgagctgtac gttggagttc 13140
tataactggt cctgcccatc cacgccctcc ccaacaccca cgccctccaa gtcgacgccc 13200
acgccttcca agccatcgtc cacgccctcc aagccgacgc ccggcaccaa gcccccccgag 13260
tgcccagact ttgatcctcc cagacaggag aacgagactt ggtggctgtg cgactgcttc 13320
atggccacgt gcaagtacaa caacacggtg gagatcgtga aggtggagtg tgagccgccg 13380
cccatgccca cctgctccaa cggcctccaa cccgtgcgcg tcgaggaccc cgacggctgc 13440
tgctggcact gggagtgcga ctgctactgc acgggctggg gcgacccgca ctatgtcacc 13500
ttcgacggac tctactacag ctaccagggc aactgcacct acgtgctggt ggaggagatc 13560
agcccctccg tggacaactt cggagtttac atcgacaact accactgcga tcccaacgac 13620
aaggtgtcct gtccccgcac cctcatcgtg cgccacgaga cccaggaggt gctgatcaag 13680
accgtgcata tgatgcccat gcaggtgcag gtgcaggtga acaggcaggc ggtggcactg 13740
ccctacaaga agtacgggct ggaggtgtac cagtctggca tcaactacgt ggtggacatc 13800
cccgagctgg gtgtcctcgt ctcctacaat ggcctgtcct tctccgtcag gctgccctac 13860
caccggtttg gcaacaacac caagggccag tgtggcacct gcaccaacac cacctccgac 13920
gactgcattc tgcccagcgg ggagatcgtc tccaactgtg aggctgcggc tgaccagtgg 13980
ctggtgaacg acccctccaa gccacactgc cccacagca gctccacgac caagcgcccg 14040
gccgtcactg tgcccggggg cggtaaaacg accccacaca aggactgcac cccatctccc 14100

ctctgccagc tcatcaagga cagcctgttt gcccagtgcc acgcactggt gcccccgcag 14160
cactactacg atgcctgcgt gttcgacagc tgcttcatgc cgggctcgag cctggagtgc 14220
gccagtctgc aggcctacgc agccctctgt gcccagcaga acatctgcct cgactggcgg 14280
aaccacacgc atggggcctg cttggtggag tgcccatctc acagggagta ccaggcctgt 14340
ggccctgcag aagagcccac gtgcaaatcc agctcctccc agcagaacaa cacagtcctg 14400
gtggaaggct gcttctgtcc tgagggcacc atgaactacg ctcctggctt tgatgtctgc 14460
gtgaagacct gcggctgtgt gggacctgac aatgtgccca gagagtttgg ggagcacttc 14520
gagttcgact gcaagaactg tgtctgcctg gagggtggaa gtggcatcat ctgccaaccc 14580
aagaggtgca gccagaagcc cgttacccac tgcgtggaag acggcaccta cctcgccacg 14640
gaggtcaacc ctgccgacac ctgctgcaac attaccgtct gcaagtgcaa caccagcctg 14700
tgcaaagaga agccctccgt gtgcccgctg ggattcgaag tgaagagcaa gatggtgcct 14760
ggaaggtgct gtcccttcta ctggtgtgag tccaaggggg tgtgtgttca cgggaatgct 14820
gagtaccagc ccggttctcc agtttattcc tccaagtgcc aggactgcgt gtgcacggac 14880
aaggtggaca acaacaccct gctcaacgtc atcgcctgca cccacgtgcc ctgcaacacc 14940
tcctgcagcc ctggcttcga actcatggag gcccccgggg agtgctgtaa gaagtgtgaa 15000
cagacgcact gtatcatcaa acggcccgac aaccagcacg tcatcctgaa gcccggggac 15060
ttcaagagcg acccgaagaa caactgcaca ttcttcagct gcgtgaagat ccacaaccag 15120
ctcatctcgt ccgtctccaa catcacctgc cccaactttg atgccagcat ttgcatcccg 15180
ggctccatca cattcatgcc caatggatgc tgcaagacct gcacccctcg caatgagacc 15240
agggtgccct gctccaccgt ccccgtcacc acggaggttt cgtacgccgg ctgcaccaag 15300
accgtcctca tgaatcattg ctccgggtcc tgcgggacat ttgtcatgta ctcggccaag 15360
gcccaggccc tggaccacag ctgctcctgc tgcaaagagg agaaaaccag ccagcgtgag 15420
gtggtcctga gctgccccaa tggcggctcg ctgacacaca cctacaccca catcgagagc 15480
tgccagtgcc aggacaccgt ctgcgggctc cccaccggca cctcccgccg ggcccggcgc 15540
tcccctaggc atctggggag cgggtgagcg gggtgggcac agccccttc actgccctcg 15600
acagctttac ctcccccgga ccctctgagc ctcctaagct cggcttcctc tcttcagata 15660
tttattgtct gagtctttgt tcagtccttg ctttccaata ataaactcag gggacatgc 15720

<210> 14
<211> 15720
<212> DNA
<213> human

<400> 14
caacccacac cgcccctgcc agccaccatg ggctgccac tagcccgcct ggcggctgtg 60
tgcctggccc tgtctttggc agggggctcg gagctccaga cagagggcag aacccgatac 120
cacggccgca acgtctgcag cacctggggc aacttccact acaagacctt cgacggggac 180

gtcttccgct tccccggcct ctgcgactac aacttcgcct ccgactgccg aggctcctac 240
aaggaatttg ctgtgcacct gaagcggggt ccgggccagg ctgaggcccc cgccggggtg 300
gagtccatcc tgctgaccat caaggatgac accatctacc tcacccgcca cctggctgtg 360
cttaacgggg ccgtggtcag caccccgcac tacagccccg ggctgctcat tgagaagagc 420
gatgcctaca ccaaagtcta ctcccgcgcc ggcctcaccc tcatgtggaa ccgggaggat 480
gcactcatgc tggagctgga cactaagttc cggaaccaca cctgtggcct ctgcggggac 540
tacaacggcc tgcagagcta ttcagaattc ctctctgacg gcgtgctctt cagtcccctg 600
gagtttggga acatgcagaa gatcaaccag cccgatgtgg tgtgtgagga tcccgaggag 660
gaggtggccc ccgcatcctg ctccgagcac cgcgccgagt gtgagaggct gctgaccgcc 720
gaggccttcg cggactgtca ggacctggtg ccgctggagc cgtatctgcg cgcctgccag 780
caggaccgct gccggtgccc gggcggtgac acctgcgtct gcagcaccgt ggccgagttc 840
tcccgccagt gctcccacgc cggcggccgg cccgggaact ggaggaccgc cacgctctgc 900
cccaagacct gccccgggaa cctggtgtac ctggagagcg gctcgccctg catggacacc 960
tgctcacacc tggaggtgag cagcctgtgc gaggagcacc gcatggacgg ctgtttctgc 1020
ccagaaggca ccgtatatga cgacatcggg gacagtggct gcgttcctgt gagccagtgc 1080
cactgcaggc tgcacggaca cctgtacaca ccgggccagg agatcaccaa tgactgcgag 1140
cagtgtgtct gtaacgctgg ccgctgggtg tgcaaagacc tgccctgccc cggcacctgt 1200
gccctggaag gcggctccca catcaccacc ttcgatggga agacgtacac cttccacggg 1260
gactgctact atgtcctggc caagggtgac cacaacgatt cctacgctct cctgggcgag 1320
ctggcccccct gtggctccac agacaagcag acctgcctga agacggtggt gctgctggct 1380
gacaagaaga agaatgcggt ggtcttcaag tccgatggca gtgtactgct caaccagctg 1440
caggtgaacc tgccccacgt gaccgcgagc ttctctgtct tccgcccgtc ttcctaccac 1500
atcatggtga gcatggccat tggcgtccgg ctgcaggtgc agctggcccc agtcatgcaa 1560
ctctttgtga cactggacca ggcctcccag gggcaggtgc agggcctctg cgggaacttc 1620
aacggcctgg aaggtgacga cttcaagacg gccagcgggc tggtggaggc cacgggggcc 1680
ggctttgcca acacctggaa ggcacagtca acctgccatg acaagctgga ctggttggac 1740
gatccctgct ccctgaacat cgagagcgcc aactacgccg agcactggtg ctccctcctg 1800
aagaagacag agacccccctt tggcaggtgc cactcggctg tggaccctgc tgagtattac 1860
aagaggtgca aatatgacac gtgtaactgt cagaacaatg aggactgcct gtgcgccgcc 1920
ctgtcctcct acgcgcgcgc ctgcaccgcc aagggcgtca tgctgtgggg ctggcgggag 1980
catgtctgca acaaggatgt gggctcctgc cccaactcgc aggtcttcct gtacaacctg 2040
accacctgcc agcagacctg ccgctccctc tccgaggccg acagccactg tctcgagggc 2100
tttgcgcctg tggacggctg cggctgccct gaccacacct tcctggacga gaagggccgc 2160
tgcgtacccc tggccaagtg ctcctgttac caccgcggtc tctacctgga ggcgggggat 2220

```
gtggtcgtca ggcaggaaga acgatgtgtg tgccgggatg ggcggctgca ctgtaggcag   2280
atccggctga tcggccagag ctgcacggcc ccaaagatcc acatggactg cagcaacctg   2340
actgcactgg ccacctcgaa gccccgagcc ctcagctgcc agacgctggc cgccggctat   2400
taccacacag agtgtgtcag tggctgtgtg tgccccgacg ggctgatgga tgacggccgg   2460
ggtggctgcg tggtggagaa ggaatgccct tgcgtccata acaacgacct gtattcttcc   2520
ggcgccaaga tcaaggtgga ctgcaatacc tgcacctgca agagaggacg ctgggtgtgc   2580
acccaggctg tgtgccatgg cacctgctcc atttacggga gtggccacta catcaccttt   2640
gatgggaagt actacgactt tgacggacac tgctcctacg tggctgttca ggactactgc   2700
ggccagaact cctcactggg ctcattcagc atcatcaccg agaacgtccc ctgtggcact   2760
acgggcgtca cctgctccaa ggccatcaag atcttcatgg ggaggacgga gctgaagttg   2820
gaagacaagc accgtgtggt gatccagcgt gatgagggtc accacgtggc ctacaccacg   2880
cgggaggtgg gccagtacct ggtggtggag tccagcacgg gcatcatcgt catctgggac   2940
aagaggacca ccgtgttcat caagctggct ccctcctaca agggcaccgt gtgtggcctg   3000
tgtgggaact ttgaccaccg ctccaacaac gacttcacca cgcgggacca catggtggtg   3060
agcagcgagc tggacttcgg gaacagctgg aaggaggccc ccacctgccc agatgtgagc   3120
accaaccccg agccctgcag cctgaacccg caccgccgct cctgggccga gaagcagtgc   3180
agcatcctca aaagcagcgt gttcagcatc tgccacagca aggtggaccc caagcccttc   3240
tacgaggcct gtgtgcacga ctcgtgctcc tgtgacacgg gtggggactg tgagtgcttc   3300
tgctctgccg tggcctccta cgcccaggag tgtaccaaag agggggcctg cgtgttctgg   3360
aggacgccgg acctgtgccc catattctgc gactactaca accctccgca tgagtgtgag   3420
tggcactatg agccatgtgg gaaccggagc ttcgagacct gcaggaccat caacggcatc   3480
cactccaaca tctccgtgtc ctacctggag ggctgctacc cccggtgccc caaggacagg   3540
cccatctatg aggaggatct gaagaagtgt gtcactgcag acaagtgtgg ctgctatgtc   3600
gaggacaccc actacccacc tggagcatcg gttcccaccg aggagacctg caagtcctgc   3660
gtgtgtacca actcctccca agtcgtctgc aggccggagg aaggaaagat tcttaaccag   3720
acccaggatg gcgccttctg ctactgggag atctgtggcc ccaacgggac ggtggagaag   3780
cacttcaaca tctgttccat tacgacacgc ccgtccaccc tgaccacctt caccaccatc   3840
accctcccca ccacccccac ctccttcacc actaccacca ccaccaccac cccgacctcc   3900
agcacagttt tatcaacaac tccgaagctg tgctgcctct ggtctgactg gatcaatgag   3960
gaccacccca gcagtggcag cgacgacggt gaccgagaac catttgatgg ggtctgcggg   4020
gcccctgagg acatcgagtg caggtcggtc aaggatcccc acctcagctt ggagcagcat   4080
ggccagaagg tgcagtgtga tgtctctgtt gggttcattt gcaagaatga agaccagttt   4140
ggaaatggac catttggact gtgttacgac tacaagatac gtgtcaattg ttgctggccc   4200
atggataagt gtatcaccac tcccagccct ccaactacca ctcccagccc tccaccaacc   4260
```

acgacgacca cccttccacc aaccaccacc cccagccctc caaccaccac cacaaccacc 4320
cctccaccaa ccaccacccc cagccctcca ataaccacca cgaccacccc tctaccaacc 4380
accactccca gccctccaat aagcaccaca accacccctc caccaaccac cactcccagc 4440
cctccaacca ccactcccag ccctccaacc accactccca gccctccaac aaccaccaca 4500
accacccctc caccaaccac cactcccagc cctccaatga ctacgcccat cactccacca 4560
gccagcacta ccacccttcc accaaccacc actcccagcc ctccaacaac caccacaacc 4620
acccctccac caaccaccac tcccagtcct ccaacgacta cgcccatcac tccaccaacc 4680
agcactacta cccttccacc aaccaccact cccagccctc caccaaccac cacaaccacc 4740
cctccaccaa ccaccactcc cagccctcca acaaccacca ctcccagtcc tccaacaatc 4800
accacaacca cccctccacc aaccaccact cccagccctc caacaacgac cacaaccacc 4860
cctccaccaa ccaccactcc cagccctcca acgactacac ccatcactcc accaaccagc 4920
actaccaccc ttccaccaac caccactccc agccctccac caaccaccac aaccacccct 4980
ccaccaacca ccactcccag ccctccaaca accaccactc ccagccctcc aataaccacc 5040
acaaccaccc ctccaccaac caccactccc agctctccaa taaccaccac tcccagccct 5100
ccaacaacca ccatgaccac cccttcacca accaccaccc ccagctctcc aataaccacc 5160
acaaccaccc cttcctcaac taccactccc agccctccac caaccaccat gaccacccct 5220
tcaccaacca ccactcccag ccctccaaca accaccatga ccacccttcc accaaccacc 5280
acttccagcc ctctaacaac tactcctcta cctccatcaa taactcctcc tacattttca 5340
ccattctcaa cgacaacccc tactacccca tgcgtgcctc tctgcaattg gactggctgg 5400
ctggattctg gaaaacccaa ctttcacaaa ccaggtggag acacagaatt gattggagac 5460
gtctgtggac caggctgggc agctaacatc tcttgcagag ccaccatgta tcctgatgtt 5520
cccattggac agcttggaca aacagtggtg tgtgatgtct ctgtggggct gatatgcaaa 5580
aatgaagacc aaaagccagg tggggtcatc cctatggcct tctgcctcaa ctacgagatc 5640
aacgttcagt gctgtgagtg tgtcacccaa cccaccacca tgacaaccac caccacagag 5700
aacccaactc cgccaaccac gacacccatc accaccacca ctacggtgac ccccaacccca 5760
acacccaccg gcacacagac cccaaccacg acacccatca ccaccaccac tacggtgacc 5820
ccaaccccaa cacccaccgg cacacagacc ccaaccacga cacccatcac caccaccact 5880
acggtgaccc caaccccaac acccaccggc acacagaccc caaccacgac acccatcacc 5940
accaccacta cggtgacccc aaccccaaca cccaccggca cacagacccc aaccacgaca 6000
cccatcacca ccaccactac ggtgacccca accccaacac ccaccggcac acagacccca 6060
accacgacac ccatcaccac caccactacg gtgaccccaa ccccaacacc caccggcaca 6120
cagaccccaa ccacgacacc catcaccacc accactacgg tgaccccaac cccaacaccc 6180
accggcacac agaccccaac cacgacaccc atcaccacca ccactacggt gaccccaacc 6240
ccaacaccca ccggcacaca gaccccaacc acgacaccca tcaccaccac cactacggtg 6300

accccaaccc caacacccac cggcacacag accccaacca cgacacccat caccaccacc 6360
actacggtga ccccaacccc aacacccacc ggcacacaga ccccaaccac gacacccatc 6420
accaccacca ctacggtgac ccccaacccca acacccaccg gcacacagac cccaaccacg 6480
acacccatca ccaccaccac tacggtgacc ccaaccccaa cacccaccgg cacacagacc 6540
ccaaccacga cacccatcac caccaccact acggtgaccc caaccccaac acccaccggc 6600
acacagaccc caaccacgac acccatcacc accaccacta cggtgacccc aaccccaaca 6660
cccaccggca cacagacccc aaccacgaca cccatcacca ccaccactac ggtgacccca 6720
accccaacac ccaccggcac acagacccca accacgacac ccatcaccac caccactacg 6780
gtgaccccaa ccccaacacc caccggcaca cagaccccaa ccacgacacc catcaccacc 6840
accactacgg tgaccccaac cccaacaccc accggcacac agaccccaac cacgacaccc 6900
atcaccacca ccactacggt gaccccaacc ccaacaccca ccggcacaca gaccccaacc 6960
acgacaccca tcaccaccac cactacggtg accccaaccc caacacccac cggcacacag 7020
accccaacca cgacacccat caccaccacc actacggtga ccccaacccc aacacccacc 7080
ggcacacaga ccccaaccac gacacccatc accaccacca ctacggtgac cccaacccca 7140
acacccaccg gcacacagac cccaaccacg acacccatca ccaccaccac tacggtgacc 7200
ccaaccccaa cacccaccgg cacacagacc ccaaccacga cacccatcac caccaccact 7260
acggtgaccc caaccccaac acccaccggc acacagaccc caaccacgac acccatcacc 7320
accaccacta cggtgacccc aaccccaaca cccaccggca cacagacccc aaccacgaca 7380
cccatcacca ccaccactac ggtgacccca accccaacac ccaccggcac acagacccca 7440
accacgacac ccatcaccac caccactacg gtgaccccaa ccccaacacc caccggcaca 7500
cagaccccaa ccacgacacc catcaccacc accactacgg tgaccccaac cccaacaccc 7560
accggcacac agaccccaac cacgacaccc atcaccacca ccactacggt gaccccaacc 7620
ccaacaccca ccggcacaca gaccccaacc acgacaccca tcaccaccac cactacggtg 7680
accccaaccc caacacccac cggcacacag accccaacca cgacacccat caccaccacc 7740
actacggtga ccccaacccc aacacccacc ggcacacaga ccccaaccac gacacccatc 7800
accaccacca ctacggtgac cccaacccca acacccaccg gcacacagac cccaaccacg 7860
acacccatca ccaccaccac tacggtgacc ccaaccccaa cacccaccgg cacacagacc 7920
ccaaccacga cacccatcac caccaccact acggtgaccc caaccccaac acccaccggc 7980
acacagaccc caaccacgac acccatcacc accaccacta cggtgacccc aaccccaaca 8040
cccaccggca cacagacccc aaccacgaca cccatcacca ccaccactac ggtgacccca 8100
accccaacac ccaccggcac acagacccca accacgacac ccatcaccac caccactacg 8160
gtgaccccaa ccccaacacc caccggcaca cagaccccaa ccacgacacc catcaccacc 8220
accactacgg tgaccccaac cccaacaccc accggcacac agaccccaac cacgacaccc 8280
atcaccacca ccactacggt gaccccaacc ccaacaccca ccggcacaca gaccccaacc 8340

acgacaccca tcaccaccac cactacggtg accccaaccc caacacccac cggcacacag 8400
accccaacca cgacacccat caccaccacc actacggtga ccccaacccc aacacccacc 8460
ggcacacaga ccccaaccac gacacccatc accaccacca ctacggtgac cccaacccca 8520
acacccaccg gcacacagac cccaaccacg acacccatca ccaccaccac tacggtgacc 8580
ccaaccccaa cacccaccgg cacacagacc ccaaccacga cacccatcac caccaccact 8640
acggtgaccc caaccccaac acccaccggc acacagaccc caaccacgac acccatcacc 8700
accaccacta cggtgacccc aaccccaaca cccaccggca cacagacccc aaccacgaca 8760
cccatcacca ccaccactac ggtgacccca accccaacac ccaccggcac acagacccca 8820
accacgacac ccatcaccac caccactacg gtgaccccaa ccccaacacc caccggcaca 8880
cagaccccaa ccacgacacc catcaccacc accactacgg tgaccccaac cccaacaccc 8940
accggcacac agaccccaac cacgacaccc atcaccacca ccactacggt gaccccaacc 9000
ccaacaccca ccggcacaca gaccccaacc acgacaccca tcaccaccac cactacggtg 9060
accccaaccc caacacccac cggcacacag accccaacca cgacacccat caccaccacc 9120
actacggtga ccccaacccc aacacccacc ggcacacaga ccccaaccac gacacccatc 9180
accaccacca ctacggtgac cccaacccca acacccaccg gcacacagac cccaaccacg 9240
acacccatca ccaccaccac tacggtgacc ccaaccccaa cacccaccgg cacacagacc 9300
ccaaccacga cacccatcac caccaccact acggtgaccc caaccccaac acccaccggc 9360
acacagaccc caaccacgac acccatcacc accaccacta cggtgacccc aaccccaaca 9420
cccaccggca cacagacccc aaccacgaca cccatcacca ccaccactac ggtgacccca 9480
accccaacac ccaccggcac acagacccca accacgacac ccatcaccac caccactacg 9540
gtgaccccaa ccccaacacc caccggcaca cagaccccaa ccacgacacc catcaccacc 9600
accactacgg tgaccccaac cccaacaccc accggcacac agaccccaac cacgacaccc 9660
atcaccacca ccactacggt gaccccaacc ccaacaccca ccggcacaca gaccccaacc 9720
acgacaccca tcaccaccac cactacggtg accccaaccc caacacccac cggcacacag 9780
accccaacca cgacacccat caccaccacc actacggtga ccccaacccc aacacccacc 9840
ggcacacaga ccccaaccac gacacccatc accaccacca ctacggtgac cccaacccca 9900
acacccaccg gcacacagac cccaaccacg acacccatca ccaccaccac tacggtgacc 9960
ccaaccccaa cacccaccgg cacacagacc ccaaccacga cacccatcac caccaccact 10020
acggtgaccc caaccccaac acccaccggc acacagaccc caaccacgac acccatcacc 10080
accaccacta cggtgacccc aaccccaaca cccaccggca cacagacccc aaccacgaca 10140
cccatcacca ccaccactac ggtgacccca accccaacac ccaccggcac acagacccca 10200
accacgacac ccatcaccac caccactacg gtgaccccaa ccccaacacc caccggcaca 10260
cagaccccaa ccacgacacc catcaccacc accactacgg tgaccccaac cccaacaccc 10320
accggcacac agaccccaac cacgacaccc atcaccacca ccactacggt gaccccaacc 10380

ccaacaccca ccggcacaca gaccccaacc acgacaccca tcaccaccac cactacggtg 10440
accccaaccc caacacccac cggcacacag accccaacca cgacacccat caccaccacc 10500
actacggtga ccccaacccc aacacccacc ggcacacaga ccccaaccac gacacccatc 10560
accaccacca ctacggtgac cccaacccca acacccaccg gcacacagac cccaaccacg 10620
acacccatca ccaccaccac tacggtgacc ccaaccccaa cacccaccgg cacacagacc 10680
ccaaccacga cacccatcac caccaccact acggtgaccc caaccccaac acccaccggc 10740
acacagaccc caaccacgac acccatcacc accaccacta cggtgacccc aaccccaaca 10800
cccaccggca cacagacccc aaccacgaca cccatcacca ccaccactac ggtgacccca 10860
accccaacac ccaccggcac acagacccca accacgacac ccatcaccac caccactacg 10920
gtgaccccaa ccccaacacc caccggcaca cagaccccaa ccacgacacc catcaccacc 10980
accactacgg tgaccccaac cccaacaccc accggcacac agaccccaac cacgacaccc 11040
atcaccacca ccactacggt gaccccaacc ccaacaccca ccggcacaca gaccccaacc 11100
acgacaccca tcaccaccac cactacggtg accccaaccc caacacccac cggcacacag 11160
accccaacca cgacacccat caccaccacc actacggtga ccccaacccc aacacccacc 11220
ggcacacaga ccccaaccac gacacccatc accaccacca ctacggtgac cccaacccca 11280
acacccaccg gcacacagac cccaaccacg acacccatca ccaccaccac tacggtgacc 11340
ccaaccccaa cacccaccgg cacacagacc ccaaccacga cacccatcac caccaccact 11400
acggtgaccc caaccccaac acccaccggc acacagaccc caaccacgac acccatcacc 11460
accaccacta cggtgacccc aaccccaaca cccaccggca cacagacccc aaccacgaca 11520
cccatcacca ccaccactac ggtgacccca accccaacac ccaccggcac acagacccca 11580
accacgacac ccatcaccac caccactacg gtgaccccaa ccccaacacc caccggcaca 11640
cagaccccaa ccacgacacc catcaccacc accactacgg tgaccccaac cccaacaccc 11700
accggcacac agaccccaac cacgacaccc atcaccacca ccactacggt gaccccaacc 11760
ccaacaccca ccggcacaca gaccccaacc acgacaccca tcaccaccac cactacggtg 11820
accccaaccc caacacccac cggcacacag accccaacca cgacacccat caccaccacc 11880
actacggtga ccccaacccc aacacccacc ggcacacaga ccccaaccac gacacccatc 11940
accaccacca ctacggtgac cccaacccca acacccaccg gcacacagac cccaaccacg 12000
acacccatca ccaccaccac tacggtgacc ccaaccccaa cacccaccgg cacacagacc 12060
ccaaccacga cacccatcac caccaccact acggtgaccc caaccccaac acccaccggc 12120
acacagaccc caaccacgac acccatcacc accaccacta cggtgacccc aaccccaaca 12180
cccaccggca cacagacccc aaccacgaca cccatcacca ccaccactac ggtgacccca 12240
accccaacac ccaccggcac acagacccca accacgacac ccatcaccac caccactacg 12300
gtgaccccaa ccccaacacc caccggcaca cagaccccaa ccacgacacc catcaccacc 12360
accactacgg tgaccccaac cccaacaccc accggcacac agaccccaac cacgacaccc 12420

atcaccacca ccactacggt gaccccaacc ccaacaccca ccggcacaca gaccccaacc 12480
acgacaccca tcaccaccac cactacggtg accccaaccc caacacccac cggcacacag 12540
accccaacca cgacacccat caccaccacc actacggtga ccccaacccc aacacccacc 12600
ggcacacaga ccgggccccc cacccacaca agcacagcac cgattgctga gttgaccaca 12660
tccaatcctc cgcctgagtc ctcaacccct cagacctctc ggtccacctc ttcccctctc 12720
acggagtcaa ccacccttct gagtacccta ccacctgcca ttgagatgac cagcacggcc 12780
ccaccctcca cacccacggc acccacgacc acgagcggag gccacacact gtctccaccg 12840
cccagcacca ccacgtcccc tccaggcacc cccactcgcg gtaccacgac cgggtcatct 12900
tcagccccca cccccagcac tgtgcagacg accaccacca gtgcctggac cccaacgccg 12960
accccactct ccacacccag catcatcagg accacaggcc tgaggcccta cccttcctct 13020
gtgcttatct gctgtgtcct gaacgacacc tactacgcac caggtgagga ggtgtacaac 13080
ggcacatacg gagacacctg ttatttcgtc aactgctcac tgagctgtac gttggagttc 13140
tataactggt cctgcccatc cacgccctcc ccaacaccca cgccctccaa gtcgacgccc 13200
acgccttcca agccatcgtc cacgccctcc aagccgacgc ccggcaccaa gcccccccgag 13260
tgcccagact ttgatcctcc cagacaggag aacgagactt ggtggctgtg cgactgcttc 13320
atggccacgt gcaagtacaa caacacggtg gagatcgtga aggtggagtg tgagccgccg 13380
cccatgccca cctgctccaa cggcctccaa cccgtgcgcg tcgaggaccc cgacggctgc 13440
tgctggcact gggagtgcga ctgctactgc acgggctggg gcgacccgca ctatgtcacc 13500
ttcgacggac tctactacag ctaccagggc aactgcacct acgtgctggt ggaggagatc 13560
agcccctccg tggacaactt cggagtttac atcgacaact accactgcga tcccaacgac 13620
aaggtgtcct gtccccgcac cctcatcgtg cgccacgaga cccaggaggt gctgatcaag 13680
accgtgcata tgatgcccat gcaggtgcag gtgcaggtga acaggcaggc ggtggcactg 13740
ccctacaaga agtacgggct ggaggtgtac cagtctggca tcaactacgt ggtggacatc 13800
cccgagctgg gtgtcctcgt ctcctacaat ggcctgtcct tctccgtcag gctgccctac 13860
caccggtttg gcaacaacac caagggccag tgtggcacct gcaccaacac cacctccgac 13920
gactgcattc tgcccagcgg ggagatcgtc tccaactgtg aggctgcggc tgaccagtgg 13980
ctggtgaacg acccctccaa gccacactgc cccccacagca gctccacgac caagcgcccg 14040
gccgtcactg tgcccggggg cggtaaaacg accccacaca aggactgcac cccatctccc 14100
ctctgccagc tcatcaagga cagcctgttt gcccagtgcc acgcactggt gcccccgcag 14160
cactactacg atgcctgcgt gttcgacagc tgcttcatgc cgggctcgag cctggagtgc 14220
gccagtctgc aggcctacgc agccctctgt gcccagcaga acatctgcct cgactggcgg 14280
aaccacacgc atggggcctg cttggtggag tgcccatctc acagggagta ccaggcctgt 14340
ggccctgcag aagagcccac gtgcaaatcc agctcctccc agcagaacaa cacagtcctg 14400
gtggaaggct gcttctgtcc tgagggcacc atgaactacg ctcctggctt tgatgtctgc 14460

gtgaagacct gcggctgtgt gggacctgac aatgtgccca gagagtttgg ggagcacttc 14520
gagttcgact gcaagaactg tgtctgcctg gagggtggaa gtggcatcat ctgccaaccc 14580
aagaggtgca gccagaagcc cgttacccac tgcgtggaag acggcaccta cctcgccacg 14640
gaggtcaacc ctgccgacac ctgctgcaac attaccgtct gcaagtgcaa caccagcctg 14700
tgcaaagaga agccctccgt gtgcccgctg ggattcgaag tgaagagcaa gatggtgcct 14760
ggaaggtgct gtcccttcta ctggtgtgag tccaaggggg tgtgtgttca cgggaatgct 14820
gagtaccagc ccggttctcc agtttattcc tccaagtgcc aggactgcgt gtgcacggac 14880
aaggtggaca acaacaccct gctcaacgtc atcgcctgca cccacgtgcc ctgcaacacc 14940
tcctgcagcc ctggcttcga actcatggag gcccccgggg agtgctgtaa gaagtgtgaa 15000
cagacgcact gtatcatcaa acggcccgac aaccagcacg tcatcctgaa gcccggggac 15060
ttcaagagcg acccgaagaa caactgcaca ttcttcagct gcgtgaagat ccacaaccag 15120
ctcatctcgt ccgtctccaa catcacctgc cccaactttg atgccagcat ttgcatcccg 15180
ggctccatca cattcatgcc caatggatgc tgcaagacct gcacccctcg caatgagacc 15240
agggtgccct gctccaccgt ccccgtcacc acggaggttt cgtacgccgg ctgcaccaag 15300
accgtcctca tgaatcattg ctccgggtcc tgcgggacat ttgtcatgta ctcggccaag 15360
gcccaggccc tggaccacag ctgctcctgc tgcaaagagg agaaaaccag ccagcgtgag 15420
gtggtcctga gctgccccaa tggcggctcg ctgacacaca cctacaccca catcgagagc 15480
tgccagtgcc aggacaccgt ctgcgggctc cccaccggca cctcccgccg ggcccggcgc 15540
tcccctaggc atctggggag cgggtgagcg gggtgggcac agccccccttc actgccctcg 15600
acagctttac ctcccccgga ccctctgagc ctcctaagct cggcttcctc tcttcagata 15660
tttattgtct gagtctttgt tcagtccttg ctttccaata ataaactcag ggggacatgc 15720

<210> 15
<211> 3697
<212> DNA
<213> human

<400> 15
agggagtgtt cccgggggag atactccagt cgtagcaaga gtctcgacca ctgaatggaa 60
gaaaaggact tttaaccacc attttgtgac ttacagaaag gaatttgaat aaagaaaact 120
atgatacttc aggcccatct tcactccctg tgtcttctta tgctttattt ggcaactgga 180
tatggccaag aggggaagtt tagtggaccc ctgaaaccca tgacattttc tatttatgaa 240
ggccaagaac cgagtcaaat tatattccag tttaaggcca atcctcctgc tgtgactttt 300
gaactaactg gggagacaga caacatattt gtgatagaac gggagggact tctgtattac 360
aacagagcct tggacaggga aacaagatct actcacaatc tccaggttgc agccctggac 420
gctaatggaa ttatagtgga gggtccagtc cctatcacca tagaagtgaa ggacatcaac 480
gacaatcgac ccacgtttct ccagtcaaag tacgaaggct cagtaaggca gaactctcgc 540
ccaggaaagc ccttcttgta tgtcaatgcc acagacctgg atgatccggc cactcccaat 600

ggccagcttt attaccagat tgtcatccag cttcccatga tcaacaatgt catgtacttt 660
cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccacccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700
tgcattataa ttttttaaac agatattccc tcttgtcctt taatatttgc taaatatttc 2760
tttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120
cttgtgtttt tccctttgg ggcaagacag actcattaaa tattctgtac atttttcttt 3180
tatcaaggag atatatcagt gttgtctcat agaactgcct ggattccatt tatgtttttt 3240
ctgattccat cctgtgtccc cttcatcctt gactcctttg gtatttcact gaatttcaaa 3300
catttgtcag agaagaaaaa cgtgaggact caggaaaaat aaataaataa aagaacagcc 3360
ttttccctta gtattaacag aaatgtttct gtgtcattaa ccatctttaa tcaatgtgac 3420
atgttgctct ttggctgaaa ttcttcaact tggaaatgac acagacccac agaaggtgtt 3480
caaacacaac ctactctgca aaccttggta aaggaaccag tcagctggcc agatttcctc 3540
actacctgcc atgcatacat gctgcgcatg ttttcttcat tcgtatgtta gtaaagtttt 3600
ggttattata tatttaacat gtggaagaaa acaagacatg aaaagagtgg tgacaaatca 3660
agaataaaca ctggttgtag tcagttttgt ttgttaa 3697

<210> 16
<211> 3697
<212> DNA
<213> human

<400> 16
agggagtgtt cccgggggag atactccagt cgtagcaaga gtctcgacca ctgaatggaa 60
gaaaaggact tttaaccacc attttgtgac ttacagaaag gaatttgaat aaagaaaact 120
atgatacttc aggcccatct tcactccctg tgtcttctta tgctttattt ggcaactgga 180
tatggccaag aggggaagtt tagtggaccc ctgaaaccca tgacattttc tatttatgaa 240
ggccaagaac cgagtcaaat tatattccag tttaaggcca atcctcctgc tgtgactttt 300
gaactaactg gggagacaga caacatattt gtgatagaac gggagggact tctgtattac 360
aacagagcct tggacaggga aacaagatct actcacaatc tccaggttgc agccctggac 420
gctaatggaa ttatagtgga gggtccagtc cctatcacca tagaagtgaa ggacatcaac 480
gacaatcgac ccacgtttct ccagtcaaag tacgaaggct cagtaaggca gaactctcgc 540
ccaggaaagc ccttcttgta tgtcaatgcc acagacctgg atgatccggc cactcccaat 600
ggccagcttt attaccagat tgtcatccag cttcccatga tcaacaatgt catgtacttt 660
cagatcaaca acaaaacggg agccatctct cttacccgag agggatctca ggaattgaat 720

cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccacccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa ttttttaaac agatattccc tcttgtcctt taatatttgc taaatatttc 2760

tttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120
cttgtgtttt tcccttttgg ggcaagacag actcattaaa tattctgtac atttttttctt 3180
tatcaaggag atatatcagt gttgtctcat agaactgcct ggattccatt tatgtttttt 3240
ctgattccat cctgtgtccc cttcatcctt gactcctttg gtatttcact gaatttcaaa 3300
catttgtcag agaagaaaaa cgtgaggact caggaaaaat aaataaataa aagaacagcc 3360
ttttccctta gtattaacag aaatgtttct gtgtcattaa ccatctttaa tcaatgtgac 3420
atgttgctct ttggctgaaa ttcttcaact tggaaatgac acagacccac agaaggtgtt 3480
caaacacaac ctactctgca aaccttggta aaggaaccag tcagctggcc agatttcctc 3540
actacctgcc atgcatacat gctgcgcatg ttttcttcat tcgtatgtta gtaaagtttt 3600
ggttattata tatttaacat gtggaagaaa acaagacatg aaaagagtgg tgacaaatca 3660
agaataaaca ctggttgtag tcagttttgt ttgttaa 3697

<210> 17
<211> 1597
<212> DNA
<213> human

<400> 17
aacaaactgc acccactgaa ctccgcagct agcatccaaa tcagcccttg agatttgagg 60
ccttggagac tcaggagttt tgagagcaaa atgacaacac ccagaaattc agtaaatggg 120
actttcctgg cagagccaat gaaaggccct attgctatgc aatctggtcc aaaaccactc 180
ttcaggagga tgtcttcact ggtgggcccc acgcaaagct tcttcatgag ggaatctaag 240
actttggggg ctgtccagat tatgaatggg ctcttccaca ttgccctggg gggtcttctg 300
atgatcccag cagggatcta tgcacccatc tgtgtgactg tgtggtaccc tctctgggga 360
ggcattatgt atattatttc cggatcactc ctggcagcaa cggagaaaaa ctccaggaag 420
tgtttggtca aaggaaaaat gataatgaat tcattgagcc tctttgctgc catttctgga 480
atgattcttt caatcatgga catacttaat attaaaattt cccatttttt aaaaatggag 540
agtctgaatt ttattagagc tcacacacca tatattaaca tatacaactg tgaaccagct 600
aatccctctg agaaaaactc cccatctacc caatactgtt acagcataca atctctgttc 660
ttgggcattt tgtcagtgat gctgatcttt gccttcttcc aggaacttgt aatagctggc 720
atcgttgaga atgaatggaa aagaacgtgc tccagaccca aatctaacat agttctcctg 780
tcagcagaag aaaaaaaaga acagactatt gaaataaaag aagaagtggt tgggctaact 840
gaaacatctt cccaaccaaa gaatgaagaa gacattgaaa ttattccaat ccaagaagag 900

gaagaagaag aaacagagac gaactttcca gaacctcccc aagatcagga atcctcacca 960
atagaaaatg acagctctcc ttaagtgatt tcttctgttt tctgtttcct tttttaaaca 1020
ttagtgttca tagcttccaa gagacatgct gactttcatt tcttgaggta ctctgcacat 1080
acgcaccaca tctctatctg gcctttgcat ggagtgacca tagctccttc tctcttacat 1140
tgaatgtaga gaatgtagcc attgtagcag cttgtgttgt cacgcttctt cttttgagca 1200
actttcttac actgaagaaa ggcagaatga gtgcttcaga atgtgatttc ctactaacct 1260
gttccttgga taggcttttt agtatagtat tttttttgt cattttctcc atcagcaacc 1320
agggagactg cacctgatgg aaaagatata tgactgcttc atgacattcc taaactatct 1380
ttttttatt ccacatctac gtttttggtg gagtcccttt tgcatcattg ttttaaggat 1440
gataaaaaaa aaataacaac tagggacaat acagaaccca ttccatttat ctttctacag 1500
ggctgacatt gtggcacatt cttagagtta ccacacccca tgagggaagc tctaaatagc 1560
caacacccat ctgtttttg taaaaacagc atagctt 1597

<210> 18
<211> 2907
<212> DNA
<213> human

<400> 18
gccatctggg cccaggcccc atgccccgag gaggggtggt ctgaagccca ccagagcccc 60
ctgccagact gtctgcctcc cttctgactg tggccgcttg gcatggccag caacagcagc 120
tcctgcccga cacctggggg cgggcacctc aatgggtacc cggtgcctcc ctacgccttc 180
ttcttccccc ctatgctggg tggactctcc ccgccaggcg ctctgaccac tctccagcac 240
cagcttccag ttagtggata tagcacacca tccccagcca ccattgagac ccagagcagc 300
agttctgaag agatagtgcc cagccctccc tcgccacccc ctctaccccg catctacaag 360
ccttgctttg tctgtcagga caagtcctca ggctaccact atggggtcag cgcctgtgag 420
ggctgcaagg gcttcttccg ccgcagcatc cagaagaaca tggtgtacac gtgtcaccgg 480
gacaagaact gcatcatcaa caaggtgacc cggaaccgct gccagtactg ccgactgcag 540
aagtgctttg aagtgggcat gtccaaggag tctgtgagaa acgaccgaaa caagaagaag 600
aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg cccccctcacc 1020
gacctggtct ttgccttcgc caaccagctg ctgcccctgg agatggatga tgcggagacg 1080

```
gggctgctca gcgccatctg cctcatctgc ggagaccgcc aggacctgga gcagccggac    1140
cgggtggaca tgctgcagga gccgctgctg gaggcgctaa aggtctacgt gcggaagcgg    1200
aggcccagcc gcccccacat gttccccaag atgctaatga agattactga cctgcgaagc    1260
atcagcgcca agggggctga gcgggtgatc acgctgaaga tggagatccc gggctccatg    1320
ccgcctctca tccaggaaat gttggagaac tcagagggcc tggacactct gagcggacag    1380
ccgggggggtg gggggcggga cgggggtggc ctggcccccc cgccaggcag ctgtagcccc    1440
agcctcagcc ccagctccaa cagaagcagc ccggccaccc actccccgtg accgcccacg    1500
ccacatggac acagccctcg ccctccgccc cggcttttct ctgcctttct accgaccatg    1560
tgaccccgca ccagccctgc ccccacctgc cctcccgggc agtactgggg accttccctg    1620
ggggacgggg agggaggagg cagcgactcc ttggacagag gcctgggccc tcagtggact    1680
gcctgctccc acagcctggg ctgacgtcag aggccgaggc caggaactga gtgaggcccc    1740
tggtcctggg tctcaggatg ggtcctgggg gcctcgtgtt catcaagaca cccctctgcc    1800
cagctcacca catcttcatc accagcaaac gccaggactt ggctccccca tcctcagaac    1860
tcacaagcca ttgctcccca gctggggaac ctcaacctcc cccctgcctc ggttggtgac    1920
agagggggtg ggacaggggc ggggggttcc ccctgtacat accctgccat accaacccca    1980
ggtattaatt ctcgctggtt ttgtttttat tttaattttt ttgttttgat tttttttaata    2040
agaattttca ttttaagcac atttatactg aaggaatttg tgctgtgtat tggggggagc    2100
tggatccaga gctggagggg gtgggtccgg gggagggagt ggctcggaag ggcccccac    2160
tctcctttca tgtccctgtg ccccccagtt ctcctcctca gccttttcct cctcagtttt    2220
ctctttaaaa ctgtgaagta ctaactttcc aaggcctgcc ttcccctccc tcccactgga    2280
gaagccgcca gccccttttct ccctctgcct gaccactggg tgtggacggt gtggggcagc    2340
cctgaaagga caggctcctg gccttggcac ttgcctgcac ccaccatgag gcatggagca    2400
gggcagagca agggccccgg gacagagttt tcccagacct ggctcctcgg cagagctgcc    2460
tcccgtcagg gcccacatca tctaggctcc ccagccccca ctgtgaaggg gctggccagg    2520
ggcccgagct gcccccaccc ccggcctcag ccaccagcac ccccataggg cccccagaca    2580
ccacacacat gcgcgtgcgc acacacacaa acacacacac actggacagt agatgggccg    2640
acacacactt ggcccgagtt cctccatttc cctggcctgc cccccacccc caacctgtcc    2700
caccccccgtg ccccctcctt accccgcagg acgggcctac agggggggtct cccctcaccc    2760
ctgcaccccc agctggggga gctggctctg ccccgacctc cttcaccagg ggttggggcc    2820
ccttcccctg gagcccgtgg gtgcacctgt tactgttggg ctttccactg agatctactg    2880
gataaagaat aaagttctat ttattct                                        2907
```

<210> 19
<211> 2907
<212> DNA
<213> human

<400> 19

```
gccatctggg cccaggcccc atgccccgag gaggggtggt ctgaagccca ccagagcccc     60
ctgccagact gtctgcctcc cttctgactg tggccgcttg gcatggccag caacagcagc    120
tcctgcccga cacctggggg cgggcacctc aatgggtacc cggtgcctcc ctacgccttc    180
ttcttccccc ctatgctggg tggactctcc ccgccaggcg ctctgaccac tctccagcac    240
cagcttccag ttagtggata tagcacacca tccccagcca ccattgagac ccagagcagc    300
agttctgaag agatagtgcc cagccctccc tcgccacccc ctctaccccg catctacaag    360
ccttgctttg tctgtcagga caagtcctca ggctaccact atggggtcag cgcctgtgag    420
ggctgcaagg gcttcttccg ccgcagcatc cagaagaaca tggtgtacac gtgtcaccgg    480
gacaagaact gcatcatcaa caaggtgacc cggaaccgct gccagtactg ccgactgcag    540
aagtgctttg aagtgggcat gtccaaggag tctgtgagaa acgaccgaaa caagaagaag    600
aaggaggtgc ccaagcccga gtgctctgag agctacacgc tgacgccgga ggtgggggag    660
ctcattgaga aggtgcgcaa agcgcaccag gaaaccttcc ctgccctctg ccagctgggc    720
aaatacacta cgaacaacag ctcagaacaa cgtgtctctc tggacattga cctctgggac    780
aagttcagtg aactctccac caagtgcatc attaagactg tggagttcgc caagcagctg    840
cccggcttca ccaccctcac catcgccgac cagatcaccc tcctcaaggc tgcctgcctg    900
gacatcctga tcctgcggat ctgcacgcgg tacacgcccg agcaggacac catgaccttc    960
tcggacgggc tgaccctgaa ccggacccag atgcacaacg ctggcttcgg cccccctcacc  1020
gacctggtct ttgccttcgc caaccagctg ctgcccctgg agatggatga tgcggagacg   1080
gggctgctca gcgccatctg cctcatctgc ggagaccgcc aggacctgga gcagccggac   1140
cgggtggaca tgctgcagga gccgctgctg gaggcgctaa aggtctacgt gcggaagcgg   1200
aggcccagcc gcccccacat gttccccaag atgctaatga agattactga cctgcgaagc   1260
atcagcgcca agggggctga gcgggtgatc acgctgaaga tggagatccc gggctccatg   1320
ccgcctctca tccaggaaat gttggagaac tcagagggcc tggacactct gagcggacag   1380
ccgggggggtg gggggcggga cgggggtggc ctggcccccc cgccaggcag ctgtagcccc   1440
agcctcagcc ccagctccaa cagaagcagc ccggccaccc actccccgtg accgcccacg   1500
ccacatggac acagccctcg ccctccgccc cggcttttct ctgcctttct accgaccatg   1560
tgaccccgca ccagccctgc ccccacctgc cctcccgggc agtactgggg accttccctg   1620
ggggacgggg agggaggagg cagcgactcc ttggacagag gcctgggccc tcagtggact   1680
gcctgctccc acagcctggg ctgacgtcag aggccgaggc caggaactga gtgaggcccc   1740
tggtcctggg tctcaggatg ggtcctgggg gcctcgtgtt catcaagaca cccctctgcc   1800
cagctcacca catcttcatc accagcaaac gccaggactt ggctccccca tcctcagaac   1860
tcacaagcca ttgctcccca gctggggaac ctcaacctcc cccctgcctc ggttggtgac   1920
agagggggtg ggacaggggc ggggggttcc ccctgtacat accctgccat accaacccca   1980
ggtattaatt ctcgctggtt ttgtttttat tttaattttt ttgttttgat ttttttaata   2040
```

agaattttca ttttaagcac atttatactg aaggaatttg tgctgtgtat tggggggagc 2100

tggatccaga gctggagggg gtgggtccgg gggagggagt ggctcggaag gggcccccac 2160

tctcctttca tgtccctgtg ccccccagtt ctcctcctca gccttttcct cctcagtttt 2220

ctctttaaaa ctgtgaagta ctaactttcc aaggcctgcc ttcccctccc tcccactgga 2280

gaagccgcca gcccctttct ccctctgcct gaccactggg tgtggacggt gtggggcagc 2340

cctgaaagga caggctcctg gccttggcac ttgcctgcac ccaccatgag gcatggagca 2400

gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg cccccctcctt accccgcagg acgggcctac aggggggtct cccctcaccc 2760

ctgcaccccc agctgggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 20
<211> 1347
<212> DNA
<213> human

<400> 20
atggatccca aatatttcat cttaattttg ttttgtggac acctgaacaa tacatttttt 60

tcaaagacag agacaattac aacagagaag cagtcacagc ctaccttata cacatcatca 120

atgtcacagg tattggctaa ttctcaaaac acaacaggga atcctttggg tcaaccaaca 180

caattcagcg acactttttc tggacaatca atatcacctg ccaaagtcac tgctggacaa 240

ccaacaccag ctgtctatac ctcttctgaa aaaccagaag cacatacttc tgctggacaa 300

ccacttgcct acaacaccaa acaaccaaca ccaatagcca acacctcctc ccagcaagcc 360

gtgttcacct ctgccagaca actaccatct gcccgtactt ctaccacaca accaccaaag 420

tcatttgtct atacttttac tcaacaatca tcatctgtcc agatcccttc tagaaaacaa 480

ataactgttc ataatccatc cacacaacca acatcaactg tcaaaaattc acctaggagt 540

acaccaggat ttatcttaga tactaccagt aacaaacaaa ccccacaaaa aaacaattat 600

aattcaatag ctgccatact aattggtgta cttctgactt ctatgttggt agctataatc 660

atcattgtac tttggaaatg cttaaggaaa ccagttttaa atgatcaaaa ttgggcaggt 720

agatctccat ttgctgatgg agaaacccct gacatttgta tggataacat cagagaaaat 780

gaaatatcca caaaacgtac atcaatcatt tcacttacac cctggaaacc aagcaaaagc 840

acacttttag cagatgactt agaaattaag ttgtttgaat caagtgaaaa cattgaagac 900

tccaacaacc ccaaaacaga gaaaataaaa gatcaagtaa atggtacatc agaagatagt 960
gctgatggtt caacagttgg aactgctgtt tcttcttcag atgatgcaga tctgcctcca 1020
ccacctcccc ttctggattt ggaaggacag gaaagtaacc aatctgacaa acccacaatg 1080
acaattgtat ctcctcttcc aaatgattct actagtctcc ctccatctct ggactgtctc 1140
aatcaagact gtggagatca taaatctgag ataatacaat catttccacc gcttgactca 1200
cttaacttgc ccctgccacc agtagatttt atgaaaaacc aagaagattc caaccttgag 1260
atccagtgtc aggagttctc tattcctccc aactctgatc aagatcttaa tgaatccctg 1320
ccacctccac ctgcagaact gttataa 1347

<210> 21
<211> 2170
<212> DNA
<213> human

<400> 21
ctgagggctc atccctctgc agagcgcggg gtcaccggga ggagacgcca tgacgcccgc 60
cctcacagcc ctgctctgcc ttgggctgag tctgggcccc aggacccgcg tgcaggcagg 120
gcccttcccc aaacccaccc tctgggctga gccaggctct gtgatcagct gggggagccc 180
cgtgaccatc tggtgtcagg ggagcctgga ggcccaggag taccgactgg ataaagaggg 240
aagcccagag ccccttggaca gaaataaccc actggaaccc aagaacaagg ccagattctc 300
catcccatcc atgacagagc accatgcggg gagataccgc tgccactatt acagctctgc 360
aggctggtca gagcccagcg accccctgga gctggtgatg acaggattct acaacaaacc 420
caccctctca gccctgccca gccctgtggt ggcctcaggg gggaatatga ccctccgatg 480
tggctcacag aagggatatc accattttgt tctgatgaag gaaggagaac accagctccc 540
ccggaccctg gactcacagc agctccacag tggggggttc caggccctgt tccctgtggg 600
ccccgtgaac cccagccaca ggtggaggtt cacatgctat tactattata tgaacacccc 660
ccaggtgtgg tcccacccca gtgacccccct ggagattctg ccctcaggcg tgtctaggaa 720
gccctccctc ctgaccctgc agggccctgt cctggcccct gggcagagcc tgaccctcca 780
gtgtggctct gatgtcggct acgacagatt tgttctgtat aaggaggggg aacgtgactt 840
cctccagcgc cctggccagc agccccaggc tgggctctcc caggccaact tcaccctggg 900
ccctgtgagc cctcccacg ggggccagta caggtgctat ggtgcacaca acctctcctc 960
cgagtggtcg gcccccagcg accccctgaa catcctgatg gcaggacaga tctatgacac 1020
cgtctccctg tcagcacagc cgggccccac agtggcctca ggagagaacg tgaccctgct 1080
gtgtcagtca tggtggcagt ttgacacttt ccttctgacc aaagaagggg cagcccatcc 1140
cccactgcgt ctgagatcaa tgtacggagc tcataagtac caggctgaat tccccatgag 1200
tcctgtgacc tcagcccacg cggggaccta caggtgctac ggctcataca gctccaaccc 1260
ccacctgctg tctttcccca gtgagcccct ggaactcatg gtctcaggac actctggagg 1320
ctccagcctc ccacccacag ggccgccctc cacacctggt ctgggaagat acctggaggt 1380

tttgattggg gtctcggtgg ccttcgtcct gctgctcttc ctcctcctct tcctcctcct 1440
ccgacgtcag cgtcacagca aacacaggac atctgaccag agaaagactg atttccagcg 1500
tcctgcaggg gctgcggaga cagagcccaa ggacaggggc ctgctgagga ggtccagccc 1560
agctgctgac gtccaggaag aaaacctcta tgctgccgtg aaggacacac agtctgagga 1620
cagggtggag ctggacagtc agagcccaca cgatgaagac cccagcgcag tgacgtatgc 1680
cccggtgaaa cactccagtc ctaggagaga aatggcctct cctccctcct cactgtctgg 1740
ggaattcctg gacacaaagg acagacaggt ggaagaggac aggcagatgg acactgaggc 1800
tgctgcatct gaagcctccc aggatgtgac ctacgcccag ctgcacagct tgacccttag 1860
acggaaggca actgagcctc ctccatccca ggaaggggaa cctccagctg agcccagcat 1920
ctacgccact ctggccatcc actagcccgg ggggtacgca gaccccacac tcagcagaag 1980
gagactcagg actgctgaag gcacgggagc tgcccccagt ggacaccagt gaaccccagt 2040
cagcctggac ccctaacaca gaccatgagg agacgctggg aacttgtggg actcacctga 2100
ctcaaagatg actaatatcg tcccattttg gaaataaagc aacagacttc tcaacaatca 2160
atgagttaat 2170

<210> 22
<211> 1453
<212> DNA
<213> human

<400> 22
gggagtgcat ccgccccaac ccttttcccc ctcgtctcct gtgagaattc cccgtcggat 60
acgagcagcg tggccgttgg ctgcctcgca caggacttcc ttcccgactc catcactttc 120
tcctggaaat acaagaacaa ctctgacatc agcagcaccc ggggcttccc atcagtcctg 180
agagggggca agtacgcagc cacctcacag gtgctgctgc cttccaagga cgtcatgcag 240
ggcacagacg aacacgtggt gtgcaaagtc cagcacccca acggcaacaa agaaaagaac 300
gtgcctcttc cagtgattgc cgagctgcct cccaaagtga gcgtcttcgt cccaccccgc 360
gacggcttct tcggcaaccc ccgcaagtcc aagctcatct gccaggccac gggtttcagt 420
ccccggcaga ttcaggtgtc ctggctgcgc gaggggaagc aggtggggtc tggcgtcacc 480
acggaccagg tgcaggctga ggccaaagag tctgggccca cgacctacaa ggtgaccagc 540
acactgacca tcaaagagag cgactggctc agccagagca tgttcacctg ccgcgtggat 600
cacaggggcc tgaccttcca gcagaatgcg tcctccatgt gtgtccccga tcaagacaca 660
gccatccggg tcttcgccat ccccccatcc tttgccagca tcttcctcac caagtccacc 720
aagttgacct gcctggtcac agacctgacc acctatgaca gcgtgaccat ctcctggacc 780
cgccagaatg gcgaagctgt gaaaacccac accaacatct ccgagagcca ccccaatgcc 840
actttcagcg ccgtgggtga ggccagcatc tgcgaggatg actggaattc cggggagagg 900
ttcacgtgca ccgtgaccca cacagacctg ccctcgccac tgaagcagac catctcccgg 960

cccaaggggg tggccctgca caggcccgat gtctacttgc tgccaccagc ccgggagcag 1020
ctgaacctgc gggagtcggc caccatcacg tgcctggtga cgggcttctc tcccgcggac 1080
gtcttcgtgc agtggatgca gagggggcag cccttgtccc cggagaagta tgtgaccagc 1140
gccccaatgc ctgagcccca ggccccaggc cggtacttcg cccacagcat cctgaccgtg 1200
tccgaagagg aatggaacac gggggagacc tacacctgcg tggtggccca tgaggccctg 1260
cccaacaggg tcaccgagag gaccgtggac aagtccaccg gtaaacccac cctgtacaac 1320
gtgtccctgg tcatgtccga cacagctggc acctgctact gaccctgctg gcctgcccac 1380
aggctcgggg cggctggccg ctctgtgtgt gcatgcaaac taacccgtgt caacggggtg 1440
agatgttgca tct 1453

<210> 23
<211> 1192
<212> DNA
<213> human

<400> 23
cagatccatc aggtccaagc tgtgttgact accactgctt ttcccttcgt ctcaattatg 60
tcttggaaga aggctttgcg gatccctgga ggccttcggg tagcaactgt gaccttgatg 120
ctggcgatgc tgagcacccc ggtggctgag ggcagagact ctcccgagga tttcgtgtac 180
cagtttaagg gcatgtgcta cttcaccaac gggacggagc gcgtgcgtct tgtgaccaga 240
tacatctata accgagagga gtacgcacgc ttcgacagcg acgtgggggt gtatcgggcg 300
gtgacgccgc tggggccgcc tgacgccgag tactggaaca gccagaagga agtcctggag 360
aggacccggg cggagttgga cacggtgtgc agacacaact accagttgga gctccgcacg 420
accttgcagc ggcgagtgga gcccacagtg accatctccc catccaggac agaggccctc 480
aaccaccaca acctgctggt ctgctcagtg acagatttct atccagccca gatcaaagtc 540
cggtggtttc ggaatgacca ggaggagaca actggcgttg tgtccacccc ccttattagg 600
aacggtgact ggaccttcca gatcctggtg atgctggaaa tgactcccca gcgtggagac 660
gtctacacct gccacgtgga gcaccccagc ctccagaacc ccatcatcgt ggagtggcgg 720
gctcagtctg aatctgccca gagcaagatg ctgagtggca ttggaggctt cgtgctgggg 780
ctgatcttcc tcgggctggg ccttattatc catcacagga gtcagaaagg gctcctgcac 840
tgactcctga gactatttta actgggattg gttatcactt ttctgtaacg cctgcttgtc 900
cctgcccaga attcccagct gcctgtgtca gcctgtcccc cgagatcaga gtcctaccgt 960
ggctgtcacg cagccaccag gtcatctcct ttcatcccca cctcaaggct gatggctgtg 1020
accctgcttc ctgcactgac ccagagcctc tgcctgtgca cggccagctg cgtctactga 1080
ggccccaagg ggtttctgtt tctattctct cctcagactg ctcaagagaa gcacatgaaa 1140
accattacct gactttagag ctttttaca taattaaaca tgatcctgag tt 1192

<210> 24
<211> 2048

<212> DNA
<213> human

<400> 24
atgtgaaggc acaagctgct gttatataca acagagtgaa ctgagcatca gtcagaaaaa 60
gtctatgttt gcagaaatac agatccaaga caaagacagg atgggcactg ctggaaaagt 120
tattaaatgc aaagcagctg tgctttggga gcagaagcaa cccttctcca ttgaggaaat 180
agaagttgcc ccaccaaaga ctaaagaagt tcgcattaag attttggcca caggaatctg 240
tcgcacagat gaccatgtga taaaaggaac aatggtgtcc aagtttccag tgattgtggg 300
acatgaggca actgggattg tagagagcat tggagaagga gtgactacag tgaaaccagg 360
tgacaaagtc atccctctct ttctgccaca atgtagagaa tgcaatgctt gtcgcaaccc 420
agatggcaac ctttgcatta ggagcgatat tactggtcgt ggagtactgg ctgatggcac 480
caccagattt acatgcaagg gcaaaccagt acaccacttc atgaacacca gtacatttac 540
cgagtacaca gtggtggatg aatcttctgt tgctaagatt gatgatgcag ctcctcctga 600
gaaagtctgt ttaattggct gtgggttttc cactggatat ggcgctgctg ttaaaactgg 660
caaggtcaaa cctggttcca cttgcgtcgt ctttggcctg ggaggagttg gcctgtcagt 720
catcatgggc tgtaagtcag ctggtgcatc taggatcatt gggattgacc tcaacaaaga 780
caaatttgag aaggccatgg ctgtaggtgc cactgagtgt atcagtccca aggactctac 840
caaacccatc agtgaggtgc tgtcagaaat gacaggcaac aacgtgggat acacctttga 900
agttattggg catcttgaaa ccatgattga tgccctggca tcctgccaca tgaactatgg 960
gaccagcgtg gttgtaggag ttcctccatc agccaagatg ctcacctatg acccgatgtt 1020
gctcttcact ggacgcacat ggaagggatg tgtctttgga ggtttgaaaa gcagagatga 1080
tgtcccaaaa ctagtgactg agttcctggc aaagaaattt gacctggacc agttgataac 1140
tcatgtttta ccatttaaaa aaatcagtga aggatttgag ctgctcaatt caggacaaag 1200
cattcgaacg gtcctgacgt tttgagatcc aaagtggcag gaggtctgtg ttgtcatggt 1260
gaactggagt ttctcttgtg agagttccct catctgaaat catgtatctg tctcacaaat 1320
acaagcataa gtagaagatt tgttgaagac atagaaccct tataaagaat tattaacctt 1380
tataaacatt taaagtcttg tgagcacctg ggaattagta taataacaat gttaatattt 1440
ttgatttaca ttttgtaagg ctataattgt atcttttaag aaaacataca cttggatttc 1500
tatgttgaaa tggagatttt taagagtttt aaccagctgc tgcagatata taactcaaaa 1560
cagatatagc gtataaagat atagtaaatg catctcccag agtaatattc acttaacaca 1620
ttgaaactat tattttttag atttgaatat aaatgtattt tttaaacact tgttatgagt 1680
taacttggat tacattttga aatcagttca ttccatgatg catattactg gattagatta 1740
agaaagacag aaaagattaa gggacgggca catttttcaa cgattaagaa tcatcattac 1800
ataacttggt gaaactgaaa aagtatatca tatgggtaca caaggctatt tgccagcata 1860
tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 25
<211> 2048
<212> DNA
<213> human

<400> 25
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tattttttag atttgaatat aaatgtattt tttaaacact tgttatgagt 1680

taacttggat tacattttga aatcagttca ttccatgatg catattactg gattagatta 1740
agaaagacag aaaagattaa gggacgggca catttttcaa cgattaagaa tcatcattac 1800
ataacttggt gaaactgaaa aagtatatca tatgggtaca caaggctatt tgccagcata 1860
tattaatatt ttagaaaata ttccttttgt aatactgaat ataaacatag agctagagtc 1920
atattatcat acttatcata atgttcaatt tgatacagta gaattgcaag tccctaagtc 1980
cctattcact gtgcttagta gtgactccat ttaataaaaa gtgtttttag tttttaacaa 2040
ctaaaccg 2048

<210> 26
<211> 2816
<212> DNA
<213> human

<400> 26
tcgttgatat caaagacagt tgaaggaaat gaattttgaa acttcacggt gtgccaccct 60
acagtactgc cctgaccctt acatccagcg tttcgtagaa acccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380

ctttcagcct gcttcaggaa tgagcttgtg gagccccgga gagaaactcc aaaacaatct 1440
gacgtcttct ttagacattc caagccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt ttttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460
ctgatactgt tcagtgcatt tagccaggag acttacgttt tgagtaagtg agatccaagc 2520
agacgtgtta aaatcagcac tcctggactg gaaattaaag attgaaaggg tagactactt 2580
ttcttttttt tactcaaaag tttagagaat ctctgtttct ttccatttta aaaacatatt 2640
ttaagataat agcataaaga ctttaaaaat gttcctcccc tccatcttcc cacacccagt 2700
caccagcact gtattttctg tcaccaagac aatgatttct tgttattgag gctgttgctt 2760
ttgtggatgt gtgattttaa ttttcaataa acttttgcat cttggtttaa aagaaa 2816

<210> 27
<211> 2816
<212> DNA
<213> human

<400> 27
tcgttgatat caaagacagt tgaaggaaat gaattttgaa acttcacggt gtgccaccct 60
acagtactgc cctgaccctt acatccagcg tttcgtagaa acccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420

agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt ttttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460

ctgatactgt tcagtgcatt tagccaggag acttacgttt tgagtaagtg agatccaagc 2520
agacgtgtta aaatcagcac tcctggactg gaaattaaag attgaaaggg tagactactt 2580
ttcttttttt tactcaaaag tttagagaat ctctgtttct ttccatttta aaaacatatt 2640
ttaagataat agcataaaga ctttaaaaat gttcctcccc tccatcttcc cacacccagt 2700
caccagcact gtattttctg tcaccaagac aatgatttct tgttattgag gctgttgctt 2760
ttgtggatgt gtgattttaa ttttcaataa acttttgcat cttggtttaa aagaaa 2816

<210> 28
<211> 3803
<212> DNA
<213> human

<400> 28
ccatggtagg agcgctcgcc tcgctgcggt gcccgctgag gccatgccgg ggccccggcg 60
ccccgctggc tcccgcctgc gcctgctcct gctcctgctg ctgccgccgc tgctgctgct 120
gctccggggc agccacgcgg gcaacctgac ggtagccgtg gtactgccgc tggccaatac 180
ctcgtacccc tggtcgtggg cgcgcgtggg acccgccgtg gagctggccc tggcccaggt 240
gaaggcgcgc cccgacttgc tgccgggctg gacggtccgc acggtgctgg gcagcagcga 300
aaacgcgctg ggcgtctgct ccgacaccgc agcgcccctg gccgcggtgg acctcaagtg 360
ggagcacaac cccgctgtgt tcctgggccc cggctgcgtg tacgccgccg ccccagtggg 420
gcgcttcacc gcgcactggc gggtcccgct gctgaccgcc ggcgccccgg cgctgggctt 480
cggtgtcaag gacgagtatg cgctgaccac ccgcgcgggg cccagctacg ccaagctggg 540
ggacttcgtg gcggcgctgc accgacggct gggctgggag cgccaagcgc tcatgctcta 600
cgcctaccgg ccgggtgacg aagagcactg cttcttcctc gtggaggggc tgttcatgcg 660
ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380
gtaccctcct cctgacatcc ccaaatgtgg ctttgacaac gaagacccag catgcaacca 1440

agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg gggctatctg ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160
cctgcgaatg gcttcacccc ctgtgcgggg ctcccaggct ggtgacgtat acagctttgg 2220
gatcatcctt caggagattg ccctgaggag tggggtcttc cacgtggaag gtttggacct 2280
gagccccaaa gagatcatcg agcgggtgac tcggggtgag cagcccccct tccggccctc 2340
cctggccctg cagagtcacc tggaggagtt ggggctgctc atgcagcggt gctgggctga 2400
ggacccacag gagaggccac cattccagca gatccgcctg acgttgcgca aatttaacag 2460
ggagaacagc agcaacatcc tggacaacct gctgtcccgc atggagcagt acgcgaacaa 2520
tctggaggaa ctggtggagg agcggaccca ggcatacctg gaggagaagc gcaaggctga 2580
ggccctgctc taccagatcc tgcctcactc agtggctgag cagctgaagc gtggggagac 2640
ggtgcaggcc gaagcctttg acagtgttac catctacttc agtgacattg tgggtttcac 2700
agcgctgtcg gcggagagca cacccatgca ggtggtgacc ctgctcaatg acctgtacac 2760
ttgctttgat gctgtcatag acaactttga tgtgtacaag gtggagacaa ttggcgatgc 2820
ctacatggtg gtgtcagggc tccctgtgcg gaacgggcgg ctacacgcct gcgaggtagc 2880
ccgcatggcc ctggcactgc tggatgctgt gcgctccttc cgaatccgcc accggcccca 2940
ggagcagctg cgcttgcgca ttggcatcca cacaggacct gtgtgtgctg gagtggtggg 3000
actgaagatg ccccgttact gtctctttgg ggatacagtc aacacagcct caagaatgga 3060
gtctaatggg gaagccctga agatccactt gtcttctgag accaaggctg tcctggagga 3120
gtttggtggt ttcgagctgg agcttcgagg ggatgtagaa atgaagggca aaggcaaggt 3180
tcggacctac tggctccttg gggagagggg gagtagcacc cgaggctgac ctgcctcctc 3240
tcctatccct ccacacctcc cctaccctgt gccagaagca acagaggtgc caggcctcag 3300
cctcacccac agcagcccca tcgccaaagg atggaagtaa tttgaatagc tcaggtgtgc 3360
tgaccccagt gaagacacca gataggacct ctgagagggg actggcatgg ggggatctca 3420
gagcttacag gctgagccaa gcccacggcc atgcacaggg acactcacac aggcacacgc 3480

acctgctctc cacctggact caggccgggc tgggctgtgg atccttgatc ccctcccctc 3540
cccatgctct cctccctcag ccttgctacc ctgtgactta ctgggaggag agtcacctga 3600
aggggaacat gaaaagagac taggtgaaga gagggcaggg gagcccacat ctggggctgg 3660
cccacaatac ctgctccccc gaccccctcc acccagcagt agacacagtg cacaggggag 3720
aagaggggtg gcgcagaagg gttgggggcc tgtatgcctt gcttctacca tgagcagaga 3780
caattaaaat ctttattcca gtg 3803

<210> 29
<211> 3803
<212> DNA
<213> human

<400> 29
ccatggtagg agcgctcgcc tcgctgcggt gcccgctgag gccatgccgg ggccccggcg 60
ccccgctggc tcccgcctgc gcctgctcct gctcctgctg ctgccgccgc tgctgctgct 120
gctccggggc agccacgcgg gcaacctgac ggtagccgtg gtactgccgc tggccaatac 180
ctcgtacccc tggtcgtggg cgcgcgtggg acccgccgtg gagctggccc tggcccaggt 240
gaaggcgcgc cccgacttgc tgccgggctg gacggtccgc acggtgctgg gcagcagcga 300
aaacgcgctg ggcgtctgct ccgacaccgc agcgcccctg gccgcggtgg acctcaagtg 360
ggagcacaac cccgctgtgt tcctgggccc cggctgcgtg tacgccgccg ccccagtggg 420
gcgcttcacc gcgcactggc gggtcccgct gctgaccgcc ggcgccccgg cgctgggctt 480
cggtgtcaag gacgagtatg cgctgaccac ccgcgcgggg cccagctacg ccaagctggg 540
ggacttcgtg gcggcgctgc accgacggct gggctgggag cgccaagcgc tcatgctcta 600
cgcctaccgg ccgggtgacg aagagcactg cttcttcctc gtggaggggc tgttcatgcg 660
ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380
gtaccctcct cctgacatcc ccaaatgtgg ctttgacaac gaagacccag catgcaacca 1440
agatcacctt tccaccctgg aggtgctggc tttggtgggc agcctctcct tgctcggcat 1500

```
tctgattgtc tccttcttca tatacaggaa gatgcagctg gagaaggaac tggcctcgga   1560
gctgtggcgg gtgcgctggg aggacgttga gcccagtagc cttgagaggc acctgcggag   1620
tgcaggcagc cggctgaccc tgagcgggag aggctccaat tacggctccc tgctaaccac   1680
agagggccag ttccaagtct ttgccaagac agcatattat aagggcaacc tcgtggctgt   1740
gaaacgtgtg aaccgtaaac gcattgagct gacacgaaaa gtcctgtttg aactgaagca   1800
tatgcgggat gtgcagaatg aacacctgac caggtttgtg ggagcctgca ccgacccccc   1860
caatatctgc atcctcacag agtactgtcc ccgtgggagc ctgcaggaca ttctggagaa   1920
tgagagcatc accctggact ggatgttccg gtactcactc accaatgaca tcgtcaaggg   1980
catgctgttt ctacacaatg gggctatctg ttcccatggg aacctcaagt catccaactg   2040
cgtggtagat gggcgctttg tgctcaagat caccgactat gggctggaga gcttcaggga   2100
cctggaccca gagcaaggac acaccgttta tgccaaaaag ctgtggacgg cccctgagct   2160
cctgcgaatg gcttcacccc ctgtgcgggg ctcccaggct ggtgacgtat acagctttgg   2220
gatcatcctt caggagattg ccctgaggag tggggtcttc cacgtggaag gtttggacct   2280
gagccccaaa gagatcatcg agcgggtgac tcggggtgag cagccccccct tccggccctc   2340
cctggccctg cagagtcacc tggaggagtt ggggctgctc atgcagcggt gctgggctga   2400
ggacccacag gagaggccac cattccagca gatccgcctg acgttgcgca aatttaacag   2460
ggagaacagc agcaacatcc tggacaacct gctgtcccgc atggagcagt acgcgaacaa   2520
tctggaggaa ctggtggagg agcggaccca ggcatacctg gaggagaagc gcaaggctga   2580
ggccctgctc taccagatcc tgcctcactc agtggctgag cagctgaagc gtggggagac   2640
ggtgcaggcc gaagcctttg acagtgttac catctacttc agtgacattg tgggtttcac   2700
agcgctgtcg gcggagagca cacccatgca ggtggtgacc ctgctcaatg acctgtacac   2760
ttgctttgat gctgtcatag acaactttga tgtgtacaag gtggagacaa ttggcgatgc   2820
ctacatggtg gtgtcagggc tccctgtgcg gaacgggcgg ctacacgcct gcgaggtagc   2880
ccgcatggcc ctggcactgc tggatgctgt gcgctccttc cgaatccgcc accggcccca   2940
ggagcagctg cgcttgcgca ttggcatcca cacaggacct gtgtgtgctg gagtggtggg   3000
actgaagatg ccccgttact gtctctttgg ggatacagtc aacacagcct caagaatgga   3060
gtctaatggg gaagccctga agatccactt gtcttctgag accaaggctg tcctggagga   3120
gtttggtggt ttcgagctgg agcttcgagg ggatgtagaa atgaagggca aaggcaaggt   3180
tcggacctac tggctccttg gggagagggg gagtagcacc cgaggctgac ctgcctcctc   3240
tcctatccct ccacacctcc cctaccctgt gccagaagca acagaggtgc caggcctcag   3300
cctcacccac agcagcccca tcgccaaagg atggaagtaa tttgaatagc tcaggtgtgc   3360
tgaccccagt gaagacacca gataggacct ctgagagggg actggcatgg ggggatctca   3420
gagcttacag gctgagccaa gcccacggcc atgcacaggg acactcacac aggcacacgc   3480
acctgctctc cacctggact caggccgggc tgggctgtgg atccttgatc ccctcccctc   3540
```

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gaccccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 30
<211> 2970
<212> DNA
<213> human

<400> 30
ggggtaagga gttcaaggca gcgcccacac ccgggggctc tccgcaaccc gaccgcctgt 60

ccgctccccc acttcccgcc ctccctccca cctactcatt cacccaccca cccacccaga 120

gccgggacgg cagcccaggc gcccgggccc cgccgtctcc tcgccgcgat cctggacttc 180

ctcttgctgc aggacccggc ttccacgtgt gtcccggagc cggcgtctca gcacacgctc 240

cgctccgggc ctgggtgcct acagcagcca gagcagcagg gagtccggga cccgggcggc 300

atctgggcca agttaggcgc cgccgaggcc agcgctgaac gtctccaggg ccggaggagc 360

cgcggggcgt ccgggtctga gcctcagcaa atgggctccg acgtgcggga cctgaacgcg 420

ctgctgcccg ccgtcccctc cctgggtggc ggcggcggct gtgccctgcc tgtgagcggc 480

gcggcgcagt gggcgccggt gctggacttt gcgcccccgg gcgcttcggc ttacgggtcg 540

ttgggcggcc ccgcgccgcc accggctccg ccgccacccc cgccgccgcc gcctcactcc 600

ttcatcaaac aggagccgag ctggggcggc gcggagccgc acgaggagca gtgcctgagc 660

gccttcactg tccacttttc cggccagttc actggcacag ccggagcctg tcgctacggg 720

cccttcggtc ctcctccgcc cagccaggcg tcatccggcc aggccaggat gtttcctaac 780

gcgccctacc tgcccagctg cctcgagagc cagcccgcta ttcgcaatca gggttacagc 840

acggtcacct tcgacgggac gcccagctac ggtcacacgc cctcgcacca tgcggcgcag 900

ttccccaacc actcattcaa gcatgaggat cccatgggcc agcagggctc gctgggtgag 960

cagcagtact cggtgccgcc cccggtctat ggctgccaca cccccaccga cagctgcacc 1020

ggcagccagg ctttgctgct gaggacgccc tacagcagtg acaatttata ccaaatgaca 1080

tcccagcttg aatgcatgac ctggaatcag atgaacttag gagccacctt aaagggccac 1140

agcacagggt acgagagcga taaccacaca acgcccatcc tctgcggagc ccaatacaga 1200

atacacacgc acggtgtctt cagaggcatt caggatgtgc gacgtgtgcc tggagtagcc 1260

ccgactcttg tacggtcggc atctgagacc agtgagaaac gccccttcat gtgtgcttac 1320

ccaggctgca ataagagata ttttaagctg tcccacttac agatgcacag caggaagcac 1380

actggtgaga aaccatacca gtgtgacttc aaggactgtg aacgaaggtt ttctcgttca 1440

gaccagctca aaagacacca aaggagacat acaggtgtga aaccattcca gtgtaaagct 1500

tgtcagcgaa agttctcccg gtccgaccac ctgaagaccc acaccaggac tcatacaggt 1560
gaaaagccct tcagctgtcg gtggccaagt tgtcagaaaa agtttgcccg gtcagatgaa 1620
ttagtccgcc atcacaacat gcatcagaga aacatgacca aactccagct ggcgctttga 1680
ggggtctccc tcggggaccg ttcagtgtcc caggcagcac agtgtgtgaa ctgctttcaa 1740
gtctgactct ccactcctcc tcactaaaaa ggaaacttca gttgatcttc ttcatccaac 1800
ttccaagaca agataccggt gcttctggaa actaccaggt gtgcctggaa gagttggtct 1860
ctgccctgcc tacttttagt tgactcacag gccctggaga agcagctaac aatgtctggt 1920
tagttaaaag cccattgcca tttggtctgg attttctact gtaagaagag ccatagctga 1980
tcatgtcccc ctgacccttc ccttcttttt ttatgctcgt tttcgctggg gatggaatta 2040
ttgtaccatt ttctatcatg gaatatttat aggccagggc atgtgtatgt gtctgctaat 2100
gtaaactttg tcatggtttc catttactaa cagcaacagc aagaaataaa tcagagagca 2160
aggcatcggg ggtgaatctt gtctaacatt cccgaggtca gccaggctgc taacctggaa 2220
agcaggatgt agttctgcca ggcaactttt aaagctcatg catttcaagc agctgaagaa 2280
agaatcagaa ctaaccagta cctctgtata gaaatctaaa agaattttac cattcagtta 2340
attcaatgtg aacactggca cactgctctt aagaaactat gaagatctga gattttttg 2400
tgtatgtttt tgactctttt gagtggtaat catatgtgtc tttatagatg tacatacctc 2460
cttgcacaaa tggaggggaa ttcattttca tcactgggag tgtccttagt gtataaaaac 2520
catgctggta tatggcttca agttgtaaaa atgaaagtga ctttaaaaga aaatagggga 2580
tggtccagga tctccactga taagactgtt tttaagtaac ttaaggacct ttgggtctac 2640
aagtatatgt gaaaaaaatg agacttactg ggtgaggaaa tccattgttt aaagatggtc 2700
gtgtgtgtgt gtgtgtgtgt gtgtgtgttg tgttgtgttt tgttttttaa gggagggaat 2760
ttattattta ccgttgcttg aaattactgt gtaaatatat gtctgataat gatttgctct 2820
ttgacaacta aaattaggac tgtataagta ctagatgcat cactgggtgt tgatcttaca 2880
agatattgat gataacactt aaaattgtaa cctgcatttt tcactttgct ctcaattaaa 2940
gtctattcaa aaggaaaaaa aaaaaaaaaa 2970

<210> 31
<211> 2422
<212> DNA
<213> human

<400> 31
gtcagcctcc cttccaccgc catattgggc cactaaaaaa agggggctcg tcttttcggg 60
gtgtttttct ccccctcccc tgtccccgct tgctcacggc tctgcgactc cgacgccggc 120
aaggtttgga gagcggctgg gttcgcggga cccgcgggct tgcacccgcc cagactcgga 180
cgggctttgc caccctctcc gcttgcctgg tcccctctcc tctccgccct cccgctcgcc 240
agtccatttg atcagcggag actcggcggc cggccgggg cttccccgca gcccctgcgc 300
gctcctagag ctcgggccgt ggctcgtcgg ggtctgtgtc ttttggctcc gagggcagtc 360

```
gctgggcttc cgagaggggt tcgggccgcg taggggcgct ttgttttgtt cggttttgtt    420
tttttgagag tgcgagagag gcggtcgtgc agacccggga gaaagatgtc aaacgtgcga    480
gtgtctaacg ggagccctag cctggagcgg atggacgcca ggcaggcgga gcaccccaag    540
ccctcggcct gcaggaacct cttcggcccg gtggaccacg aagagttaac ccgggacttg    600
gagaagcact gcagagacat ggaagaggcg agccagcgca agtggaattt cgattttcag    660
aatcacaaac ccctagaggg caagtacgag tggcaagagg tggagaaggg cagcttgccc    720
gagttctact acagaccccc gcggcccccc aaaggtgcct gcaaggtgcc ggcgcaggag    780
agccaggatg tcagcgggag ccgcccggcg gcgcctttaa ttggggctcc ggctaactct    840
gaggacacgc atttggtgga cccaaagact gatccgtcgg acagccagac ggggttagcg    900
gagcaatgcg caggaataag gaagcgacct gcaaccgacg attcttctac tcaaaacaaa    960
agagccaaca gaacagaaga aaatgtttca gacggttccc caaatgccgg ttctgtggag   1020
cagacgccca agaagcctgg cctcagaaga cgtcaaacgt aaacagctcg aattaagaat   1080
atgtttcctt gtttatcaga tacatcactg cttgatgaag caaggaagat atacatgaaa   1140
attttaaaaa tacatatcgc tgacttcatg gaatggacat cctgtataag cactgaaaaa   1200
caacaacaca ataacactaa aattttaggc actcttaaat gatctgcctc taaaagcgtt   1260
ggatgtagca ttatgcaatt aggtttttcc ttatttgctt cattgtacta cctgtgtata   1320
tagtttttac cttttatgta gcacataaac tttggggaag ggagggcagg gtggggctga   1380
ggaactgacg tggagcgggg tatgaagagc ttgctttgat ttacagcaag tagataaata   1440
tttgacttgc atgaagagaa gcaattttgg ggaagggttt gaattgtttt ctttaaagat   1500
gtaatgtccc tttcagagac agctgatact tcatttaaaa aaatcacaaa aatttgaaca   1560
ctggctaaag ataattgcta tttattttta caagaagttt attctcattt gggagatctg   1620
gtgatctccc aagctatcta aagtttgtta gatagctgca tgtggctttt ttaaaaaagc   1680
aacagaaacc tatcctcact gccctcccca gtctctctta aagttggaat ttaccagtta   1740
attactcagc agaatggtga tcactccagg tagtttgggg caaaaatccg aggtgcttgg   1800
gagttttgaa tgttaagaat tgaccatctg cttttattaa atttgttgac aaaattttct   1860
cattttcttt tcacttcggg ctgtgtaaac acagtcaaaa taattctaaa tccctcgata   1920
tttttaaaga tctgtaagta acttcacatt aaaaaatgaa atatttttta atttaaagct   1980
tactctgtcc atttatccac aggaaagtgt tatttttaaa ggaaggttca tgtagagaaa   2040
agcacacttg taggataagt gaaatggata ctacatcttt aaacagtatt tcattgcctg   2100
tgtatggaaa aaccatttga agtgtacctg tgtacataac tctgtaaaaa cactgaaaaa   2160
ttatactaac ttatttatgt taaaagattt tttttaatct agacaatata caagccaaag   2220
tggcatgttt tgtgcatttg taaatgctgt gttgggtaga ataggttttc ccctcttttg   2280
ttaaataata tggctatgct taaaaggttg catactgagc caagtataat tttttgtaat   2340
gtgtgaaaaa gatgccaatt attgttacac attaagtaat caataaagaa aacttccata   2400
```

gctaaaaaaa aaaaaaaaaa aa 2422

<210> 32
<211> 1181
<212> DNA
<213> human

<400> 32
ggcacgaggc ccgggccccc caaagtcccg gccgggccga gggtcggcgg ccgccggcgg 60
gccgggcccg cgcacagcgc ccgcatgtac aacatgatgg agacggagct gaagccgccg 120
ggcccgcagc aaacttcggg gggcggcggc ggcaactcca ccgcggcggc ggccggcggc 180
aaccagaaaa acagcccgga ccgcgtcaag cggcccatga atgccttcat ggtgtggtcc 240
cgcgggcagc ggcgcaagat ggcccaggag aaccccaaga tgcacaactc ggagatcagc 300
aagcgcctgg gcgccgagtg gaaacttttg tcggagacgg agaagcggcc gttcatcgac 360
gaggctaagc ggctgcgagc gctgcacatg aaggagcacc cggattataa ataccggccc 420
cggcggaaaa ccaagacgct catgaagaag gataagtaca cgctgcccgg cgggctgctg 480
gcccccggcg gcaatagcat ggcgagcggg gtcggggtgg gcgccggcct gggcgcgggc 540
gtgaaccagc gcatggacag ttacgcgcac atgaacggct ggagcaacgg cagctacagc 600
atgatgcagg accagctggg ctacccgcag cacccgggcc tcaatgcgca cggcgcagcg 660
cagatgcagc ccatgcaccg ctacgacgtg agcgccctgc agtacaactc catgaccagc 720
tcgcagacct acatgaacgg ctcgcccacc tacagcatgt cctactcgca gcagggcacc 780
cctggcatgg ctcttggctc catgggttcg gtggtcaagt ccgaggccag ctccagcccc 840
cctgtggtta cctcttcctc ccactccagg gcgccctgcc aggccgggga cctccgggac 900
atgatcagca tgtatctccc cggcgccgag gtgccggaac ccgccgcccc cagcagactt 960
cacatgtccc agcactacca gagcggcccg gtgcccggca cggccattaa cggcacactg 1020
cccctctcac acatgtgagg gccggacagc gaactggagg ggggagaaat tttcaaagaa 1080
aaacgaggga aatgggaggg gtgcaaaaga ggagagtaag aaacagcatg gagaaaaccc 1140
ggtacgctca aaaaaaaaaa aaaaaaaaaa aaaaaaaaaa a 1181

<210> 33
<211> 1305
<212> DNA
<213> human

<400> 33
ggggccccgc gccggcccgc gccctgccca gtgcggcctc cttccacccg ccgctgcctg 60
gcccgcgccg tccggccgag ctgcccggcg ggctggtccc cgcgcccgag ccgcccggcc 120
gggaccccga acaaggccga gatgacttcc aaggaggacg gcaaggcggc gccgggggag 180
gagcggcgcc gcagcccgct ggaccacctg cctccgcctg ccaactccaa caagccagac 240
gccgttcagc atcgaggaca tcctcaacaa gccgtctgtg cggagaagtt actcgctgcg 300
tggggcggcg cacctgctgg ccgccgcgga caagcacgcg cagggcggct tgccctggcg 360

ggccgcgcgc tgctctcgaa gacctcgccg ctgtgcgcgc tggaggagct cgccagcaag 420
acgtttaagg ggctggaggt cagcgttctg caggcagccg aaggccgcga cggtatgacc 480
atctttgggc agcggcagac ccctaagaag cggcgaaagt cgcgcacggc cttcaccaac 540
caccagatct atgaattgga aaagcgcttt ctataccaga agtacctgtc ccccgccgat 600
cgcgaccaaa tcgcgcagca gctgggcctc accaacgcgc aagtcatcac ctggttccag 660
aatcggcgcg ctaagctcaa gcgggaactg gaggagatga aggccgacgt ggagtccccc 720
aagaaactgg gccccagcgg gcagatggac atcgtggcgc tggccgaact cgagcagaac 780
tcggaggcca cagccggcgg tggcggcggc tgcggcaggg ccaagtcgag gcccggctct 840
ccggtcctcc ccccaggcgc cccgaaggcc cccgggcgct gcgccctgca gctctcgcct 900
gcctctccgc tcacggacca gccggccagc agccaggact gctcggagga cgaggaagac 960
gaagagatcg acgtggacga ttgagcggcg ccccgggtct tccgccgccc tgggctccta 1020
gcgctcgaaa gcccaacgcc tcccggaccg gaccgccgag gggagctggg acctcctctg 1080
ccactcccgc ctcctcccct gtccccggac tcggctcctg gcagccgcct cttccctctc 1140
gaagcaataa acccaggctg gccggccggg ccggccgcca ccagcggcct ccgccgcccc 1200
ggaagccctc gccgagcaat tctgtatggc ttctatataa atatttaaac ctatatagcg 1260
ggttctcccc aaaaaaaaaa aaaaaaaaaa aaaaaaaaaa aaaaa 1305

<210> 34
<211> 1926
<212> DNA
<213> human

<400> 34
gctggagcat cccgctctgg tgccgctgca gccggcagag atggttgagc tcatgttccc 60
gctgttgctc ctccttctgc ccttccttct gtatatggct gcgccccaaa tcaggaaaat 120
gctgtccagt ggggtgtgta catcaactgt tcagcttcct gggaaagtag ttgtggtcac 180
aggagctaat acaggtatcg ggaaggagac agccaaagag ctggctcaga gaggagctcg 240
agtatattta gcttgccggg atgtggaaaa gggggaattg gtggccaaag agatccagac 300
cacgacaggg aaccagcagg tgttggtgcg gaaactggac ctgtctgata ctaagtctat 360
tcgagctttt gctaagggct tcttagctga ggaaaagcac ctccacgttt tgatcaacaa 420
tgcaggagtg atgatgtgtc cgtactcgaa gacagcagat ggctttgaga tgcacatagg 480
agtcaaccac ttgggtcact tcctcctaac ccatctgctg ctagagaaac taaaggaatc 540
agccccatca aggatagtaa atgtgtcttc cctcgcacat cacctgggaa ggatccactt 600
ccataacctg cagggcgaga aattctacaa tgcaggcctg gcctactgtc acagcaagct 660
agccaacatc ctcttcaccc aggaactggc ccggagacta aaaggctctg gcgttacgac 720
gtattctgta caccctggca cagtccaatc tgaactggtt cggcactcat ctttcatgag 780
atggatgtgg tggcttttct cctttttcat caagactcct cagcagggag cccagaccag 840

cctgcactgt gccttaacag aaggtcttga gattctaagt gggaatcatt tcagtgactg 900
tcatgtggca tgggtctctg cccaagctcg taatgagact atagcaaggc ggctgtggga 960
cgtcagttgt gacctgctgg gcctcccaat agactaacag gcagtgcagt tggacccaag 1020
agaagactgc agcagactac acagtacttc ttgtcaaaat gattctcctt caaggttttc 1080
aaaaccttta gcacaaagag agcaaaacct tccagccttg cctgcttggt gtccagttaa 1140
aactcagtgt actgccagat tcgtctaaat gtctgtcatg tccagattta ctttgcttct 1200
gttactgcca gagttactag agatatcata ataggataag aagaccctca tatgacctgc 1260
acagctcatt ttccttctga aagaaactac tacctaggag aatctaagct atagcaggga 1320
tgatttatgc aaatttgaac tagcttcttt gttcacaatt cagttcctcc caaccaacca 1380
gtcttcactt caagagggcc acactgcaac ctcagcttaa catgaataac aaagactggc 1440
tcaggagcag ggcttgccca ggcatggtgg atcaccggag tcagtagttc aagaccagcc 1500
tggccaacat ggtgaaaccc cacctctact aaaaattgtg tatatctttg tgtgtcttcc 1560
tgtttatgtg tgccaaggga gtattttcac aaagttcaaa acagccacaa taatcagaga 1620
tggagcaaac cagtgccatc cagtctttat gcaaatgaaa tgctgcaaag ggaagcagat 1680
tctgtatatg ttggtaacta cccaccaaga gcacatgggt agcagggaag aagtaaaaaa 1740
agagaaggag aatactggaa gataatgcac aaaatgaagg gactagttaa ggattaacta 1800
gccctttaag gattaactag ttaaggatta atagcaaaag acattaaata tgctaacata 1860
gctatggagg aattgagggc aagcacccag gactgatgag gtcttaacaa aaaccagtgt 1920
ggcaaa 1926

<210> 35
<211> 1195
<212> DNA
<213> human

<400> 35
ccgagactca cggtcaagct aaggcgaaga gtgggtggct gaagccatac tattttatag 60
aattaatgga aagcagaaaa gacatcacaa accaagaaga actttggaaa atgaagccta 120
ggagaaattt agaagaagac gattatttgc ataaggacac gggagagacc agcatgctaa 180
aaagacctgt gcttttgcat ttgcaccaaa cagcccatgc tgatgaattt gactgccctt 240
cagaacttca gcacacacag gaactctttc cacagtggca cttgccaatt aaaatagctg 300
ctattatagc atctctgact tttctttaca ctcttctgag ggaagtaatt caccctttag 360
caacttccca tcaacaatat ttttataaaa ttccaatcct ggtcatcaac aaagtcttgc 420
caatggtttc catcactctc ttggcattgg tttacctgcc aggtgtgata gcagcaattg 480
tccaacttca taatggaacc aagtataaga agtttccaca ttggttggat aagtggatgt 540
taacaagaaa gcagtttggg cttctcagtt tcttttttgc tgtactgcat gcaatttata 600
gtctgtctta cccaatgagg cgatcctaca gatacaagtt gctaaactgg gcatatcaac 660
aggtccaaca aaataaagaa gatgcctgga ttgagcatga tgtttggaga atggagattt 720

atgtgtctct gggaattgtg ggattggcaa tactggctct gttggctgtg acatctattc 780
catctgtgag tgactctttg acatggagag aatttcacta tattcagagc aagctaggaa 840
ttgtttccct tctactgggc acaatacacg cattgatttt tgcctggaat aagtggatag 900
atataaaaca atttgtatgg tatacacctc caacttttat gatagctgtt ttccttccaa 960
ttgttgtcct gatatttaaa agcatactat tcctgccatg cttgaggaag aagatactga 1020
agattagaca tggttgggaa gacgtcacca aaattaacaa aactgagata tgttcccagt 1080
tgtagaatta ctgtttacac acatttttgt tcaatattga tatattttat caccaacatt 1140
tcaagtttgt atttgttaat aaaatgatta ttcaaggaaa aaaaaaaaaa aaaaa 1195

<210> 36
<211> 3414
<212> DNA
<213> human

<400> 36
gcttacacag tatggccggc gacattagct agcgctcgct ctactctctc taacgggaaa 60
gcagcggaat acaagagact gaactgtatc tgcctctatt tccaaaagac tcacgttcaa 120
ctttcgctca cacaaagccg ggaaaatttt attagtcctt tttttaaaaa aagttaatat 180
aaaattatag caaaaaaaaa aaggaacctg aactttagta acacagctgg aacaatcgca 240
gcggcggcgg cagcggcggg agaagaggtt taatttagtt gattttctgt ggttgttggt 300
tgttcgctag tctcacggtg atggaagctg cacatttttt cgaagggacc gagaagctgc 360
tggaggtttg gttctcccgg cagcagcccg acgcaaacca aggatctggg gatcttcgca 420
ctatcccaag atctgagtgg gacatacttt tgaaggatgt gcaatgttca atcataagtg 480
tgacaaaaac tgacaagcag gaagcttatg tactcagtga gagtagcatg tttgtctcca 540
agagacgttt cattttgaag acatgtggta ccaccctctt gctgaaagca ctggttcccc 600
tgttgaagct tgctagggat tacagtgggt ttgactcaat tcaaagcttc ttttattctc 660
gtaagaattt catgaagcct tctcaccaag ggtacccaca ccggaatttc caggaagaaa 720
tagagtttct taatgcaatt ttcccaaatg gagcaggata ttgtatggga cgtatgaatt 780
ctgactgttg gtacttatat actctggatt tcccagagag tcgggtaatc agtcagccag 840
atcaaacctt ggaaattctg atgagtgagc ttgacccagc agttatggac cagttctaca 900
tgaaagatgg tgttactgca aaggatgtca ctcgtgagag tggaattcgt gacctgatac 960
caggttctgt cattgatgcc acaatgttca atccttgtgg gtattcgatg aatggaatga 1020
aatcggatgg aacttattgg actattcaca tcactccaga accagaattt tcttatgtta 1080
gctttgaaac aaacttaagt cagacctcct atgatgacct gatcaggaaa gttgtagaag 1140
tcttcaagcc aggaaaattt gtgaccacct tgtttgttaa tcagagttct aaatgtcgca 1200
cagtgcttgc ttcgccccag aagattgaag gttttaagcg tcttgattgc cagagtgcta 1260
tgttcaatga ttacaatttt gtttttacca gttttgctaa gaagcagcaa caacagcaga 1320

gttgattaag aaaaatgaag aaaaaacgca aaaagagaac acatgtagaa ggtggtggat 1380
gctttctaga tgtcgatgct gggggcagtg ctttccataa ccaccactgt gtagttgcag 1440
aaagccctag atgtaatgat agtgtaatca ttttgaattg tatgcattat tatatcaagg 1500
agttagatat cttgcatgaa tgctctcttc tgtgtttagg tattctctgc cactcttgct 1560
gtgaaattga agtggatgta gaaaaaacct tttactatat gaaactttac aacacttgtg 1620
aaagcaactc aatttggttt atgcacagtg taatatttct ccaagtatca tccaaaattc 1680
cccacagaca aggctttcgt cctcattagg tgttggcctc agcctaaccc tctaggactg 1740
ttctattaaa ttgctgccag aattttacat ccagttacct ccactttcta gaacatattc 1800
tttactaatg ttattgaaac caatttctac ttcatactga tgtttttgga aacagcaatt 1860
aaagtttttc ttccatagtt gagtccttag aaaatgattc cagttactca ttttgcatat 1920
tgctatttaa cattattgga ccctgcattt atagtccttt gatttcttcc ctctccctgg 1980
tgtctccccc aagaccccaa ataaagcaat accctgttaa cactgtgggt ttatatacta 2040
attctatacc ccagatgggg aattaggggg gagatggtcc ctgggcttaa tattctttaa 2100
agggcatggg aatttagcct ctcttttatt gtaatgtgct cttttggaaa atagttggtt 2160
agcagggaga ccagagttgt agattgagat tgggtgtact ggctgttctg tggaaaacat 2220
acattctgtg ttcctcgaat aagtgaaatt gagcttctaa tgagatgcac ccctttacta 2280
acttgatgat gatataaaat tcatttttat ttagttaatt accagagaga tttagcataa 2340
ttttgcttct ggattcagta aatcaagtca gcttggatca ttcaccttaa cttttccttt 2400
agcagccctt tccactagtt tcccattaag tagtgttcta taaactttga tccaaagcag 2460
aatcaatgtc ttttccatct cgtgacttaa agttctgtga ctgtgatgca tgtgagtgtt 2520
ccgacttcat ctgttcctct taactacggt gtttccctta ccgatcggca ttcataggat 2580
gaaatgaatg actgtcccag aatgagaatt tgtccagatt attcagataa acatcataaa 2640
gagaataaca ttataaataa gtagaatatg aataaataga ataataaaat tccaaaatac 2700
tcaatgggaa atgactagta atataggctt tcaagagttg gtacctttac gtatatttgc 2760
agattctctg ggattttaag gaactgagaa aacagaaaag ttgactaaat tttatatttc 2820
ttgtcctcta aatattttga taatttctgg attgatgcag tgatgttttt cgttccttgt 2880
atttataaat gaaaaccttt ttttggtgtt tctaaaccta aaatctactt ggtttgaaat 2940
caagtggttg gaacactgtt tgacttttat ttgaagcatg ttgttgattg aaaatttcat 3000
tgaggaagtt ttcaatcagt gtgatcagtt tgattctgta atgagcacag cacctaatat 3060
tttgaggagc tctgttttga ggaccaatgc ttaaggtgga ccttgttgct aaacaatatc 3120
ccaatagatt tgttgacttg aggtctggtt tggttttgtt tttgttttgt tttggttttg 3180
ttttgtttcc caatagaatt aagaattcta atgttgaaaa actgtataaa tttttatggg 3240
acaaagccta gaaaagagaa atgtagtttg aatcataatc taaatcatcg tatgatagga 3300
aggaaaagtt ttggtgccat aatttctcct ttcactggtg ttggacttaa atcagttgaa 3360

atgtatttct gtaccacaat ttacgcttca ataaaagttt aattgtctag tgag 3414

<210> 37
<211> 3287
<212> DNA
<213> human

<400> 37
agactgaggc ggaggcagcc ccgcgccgcg ccggacccga gcatatttca ttttctgtca 60
ttggactttg agccattaga accatgagca actacagtgt gtcactggtt ggcccagctc 120
cttggggttt ccggctgcag ggcggtaagg atttcaacat gcctctgaca atctctagtc 180
taaaagatgg cggcaaggca gcccaggcaa atgtaagaat aggcgatgtg gttctcagca 240
ttgatggaat aaatgcacaa ggaatgactc atcttgaagc ccagaataag attaagggtt 300
gtacaggctc tttgaatatg actctgcaaa gagcatctgc tgcacccaag cctgagccgg 360
ttcctgttca aaagggagaa cctaaagaag tagttaaacc tgtgcccatt acatctcctg 420
ctgtgtccaa agtcacttcc acaaacaaca tggcctacaa taaggcacca cggccttttg 480
gttctgtgtc ttcaccaaaa gtcacatcca tcccatcacc atcgtctgcc ttcaccccag 540
cccatgcgac cacctcatca catgcttccc cttcacccgt ggctgccgtc actcctcccc 600
tgttcgctgc atctggactg catgctaatg ccaatcttag tgctgaccag tctccatctg 660
cactgagcgc tggtaaaact gcagttaatg tcccacggca gcccacagtc accagcgtgt 720
gttccgagac ttctcaggag ctagcagagg gacagagaag aggatcccag ggtgacagta 780
aacagcaaaa tggcccacca agaaaacaca ttgtggagcg ctatacagag ttttatcatg 840
tacccactca cagtgatgcc agcaagaaga gactgattga ggatactgaa gactggcgtc 900
caagaactgg aacaactcag tctcgctctt tccgaatcct tgcccagatc actgggactg 960
aacatttgaa agaatctgaa gccgataata caaagaaggc aaataactct caggagcctt 1020
ctccgcagtt ggcttccttg gtagcttcca cacggagcat gcccgagagc ctggacagcc 1080
caacctctgg cagaccaggg gttaccagcc tcacaactgc agctgccttc aagcctgtag 1140
gatccactgg cgtcatcaag tcaccaagct ggcaacggcc aaaccaagga gtaccttcca 1200
ctggaagaat ctcaaacagc gctacttact caggatcagt ggcaccagcc aactcagctt 1260
tgggacaaac ccagccaagt gaccaggaca ctttagtgca aagagctgag cacattccag 1320
cagggaaacg aactccgatg tgcgcccatt gtaaccaggt catcagagga ccattcttag 1380
tggcactggg gaaatcttgg cacccagaag aattcaactg cgctcactgc aaaaatacaa 1440
tggcctacat tggatttgta gaggagaaag gagccctgta ttgtgagctg tgctatgaga 1500
aattctttgc ccctgaatgt ggtcgatgcc aaaggaagat ccttggagaa gtcatcaatg 1560
cgttgaaaca aacttggcat gtttcctgtt ttgtgtgtgt agcctgtgga aagcccattc 1620
ggaacaatgt ttttcacttg gaggatggtg aaccctactg tgagactgat tattatgccc 1680
tctttggtac tatatgccat ggatgtgaat ttcccataga agctggtgac atgttcctgg 1740
aagctctggg ctacacctgg catgacactt gctttgtatg ctcagtgtgt tgtgaaagtt 1800

tggaaggtca gaccttttc tccaagaagg acaagcccct gtgtaagaaa catgctcatt 1860

ctgtgaattt ttgaaagtca acagttcagg agaagagaag gaatttgaag agaaaaagga 1920

aaattaaaat tactaattaa tttttagatt caatatttat atggagtttt gaaaaataat 1980

agtggccctg aaggaataaa ttccagcttt aaaaaccaag tctgaggaaa tatttggctt 2040

cataaagtaa agagacggtt tggcatttat tattactttt tcctgtattt tatgcccata 2100

aaataagctt tataaaaacc aatttcctga tggactatta aattcatctt agaataaatt 2160

agtgaagaat ttaattttag aataaataat ccaatctgaa ataattatac cttctttcct 2220

tgttaggtag ttatgagtaa atctgcaaaa ggcaatgaaa atgccttaaa ttttatcaat 2280

aacagaatta ttgtatttaa aaaaaaacta atacttatct ttaaaatagt aaataggatt 2340

ttaaacagag aattttatca gtaataggtg tcagttttta aaaaattgct tgtaggctga 2400

gcgcggtggc tcacgcctgt aatcccagca ctttgggagg ccaaggtggg tggaccacat 2460

gaggtcagga gtttgagatc agcctggcca acatggtgaa accccatctc tactaaaaat 2520

acaaaaatta gccggacgca gtggcacgcg cctgtaatcc cagctactca agaggctgag 2580

gcacgagaat cacttgaacc cgggagggag aggttgcagt gagccaagat cgtaccactg 2640

cactccagcc tgggtgacag agtgagactc cgtctccaaa aaaaaacttt gcttgtatat 2700

tatttttgcc ttacagtgga tcattctagt aggaaaggac aataagattt tttatcaaaa 2760

tgtgtcatgc cagtaagaga tgttatattc ttttcttatt tcttccccac ccaaaaataa 2820

gctaccatat agcttataag tctcaaattt ttgcctttta ctaaaatgtg attgtttctg 2880

ttcattgtgt atgcttcatc acctatatta ggcaaattcc atttttttccc ttgcgctaag 2940

gtaaagattt aattaaataa ttttggcctc tcatagtttt ctctctcttt aaagagaata 3000

aatagagggc caggtgtggt ggctcacgcc tgtgatccca gcactttggg aggccaagac 3060

gggcggatca tgaggtcaag agatcaagat catcctggcc aacatggtga aaccctgtct 3120

ctactaaaaa tacaaaaatg agctgggcat ggtggggcgt gcctgtagtc ccatgtactt 3180

gggaggctga ggcaggaaaa ttcttgaacc caggagacgg aagttgcagt gagctgagat 3240

cacaccactg cactccagcc tggtgacaga gcaagactcc ggctctt 3287

<210> 38
<211> 1254
<212> DNA
<213> human

<400> 38
atggcagtgg agaccacggt ccacactcac ctctctgcgt ctccaccgca gggctctccc 60

tacgaccaca cacccggcat ggcgggctcc ttggggtacc atccttacgc ggcgcccctg 120

ggatcgtacc cttacgggga cccagcgtac cggaagaacg ccacaaggga cgccacggct 180

accctcaagg cctggctcaa cgagcaccgc aagaacccct accccaccaa gggcgagaag 240

atcatgctgg ccatcatcac caagatgacc ctcacccagg tgtccacctg gttcgccaac 300

gcgcgccggc gcctcaagaa agagaataaa atgacgtgga cgccgcggaa ccgcagcgag 360
gacgaggaag aggaggagaa cattgacctg gagaagaacg acgaggacga gccccagaag 420
cccgaggaca agggcgaccc cgagggcccc gaagcaggag gagctgagca gaaggcggct 480
tcgggctgcg aacggcttca gggaccaccc acccctgcag gcaaggagac ggagggcagc 540
ctcagcgact cggattttaa ggagccgccc tcggagggcc gcctcgacgc gctgcagggc 600
cccccccgca ccggcgggcc ctccccggct gggccagcgg cggcgcggct ggcggaggac 660
ccggcccctc actaccccgc cggagcgccg gcgcccggcc cgcatccagc cgcgggcgag 720
gtgcctccgg gtcccggcgg gccctcggtt atccattcgc cgcctccgcc gccgcctcct 780
gcggtgctcg ccaagcccaa actgtggtct ttggcagaga tcgccacatt gtcggacaag 840
gtcaaggacg gggcggcgg gaacgagggc tctccatgcc caccgtgtcc cgggcccata 900
gccgggcaag ccctaggagg cagccgggcg tcgccggccc cggcgccgtc acgctcgccc 960
tcggcgcagt gtccttttcc aggcgggacg gtgctgtccc ggcctctcta ctacaccgcg 1020
cccttctatc ccggctacac gaactatggc tccttcggac accttcatgg ccacccgggg 1080
cccgggccag gccccacaac cggtccgggg tctcatttca atggattaaa ccagaccgtg 1140
ttgaaccgag cggacgcttt ggctaaagac ccgaaaatgt tgcggagcca gtctcagcta 1200
gacctgtgca aagactctcc ctatgaattg aagaaaggta tgtccgacat ttaa 1254

<210> 39
<211> 2560
<212> DNA
<213> human

<400> 39
gaattccggc cagaagaaat ctggcctcgg aacacgccat tctccgcgcc gcttccaata 60
accactaaca tccctaacga gcatccgagc cgagggctct gctcggaaat cgtcctggcc 120
caactcggcc cttcgagctc tcgaagatta ccgcatctat ttttttttc ttttttttct 180
tttcctagcg cagataaagt gagcccggaa agggaaggag ggggcgggga caccattgcc 240
ctgaaagaat aaataagtaa ataaacaaac tggctcctcg ccgcagctgg acgcggtcgg 300
ttgagtccag gttgggtcgg acctgaaccc ctaaaagcgg aaccgcctcc cgccctcgcc 360
atcccggagc tgagtcgccg gcggcggtgg ctgctgccag acccggagtt tcctctttca 420
ctggatggag ctgaactttg ggcggccaga gcagcacagc tgtccgggga tcgctgcacg 480
ctgagctccc tcggcaagac ccagcggcgg ctcgggattt ttttgggggg gcggggacca 540
gccccgcgcc ggcaccatgt tcctggcgac cctgtacttc gcgctgccgc tcttggactt 600
gctcctgtcg gccgaagtga gcggcggaga ccgcctggat tgcgtgaaag ccagtgatca 660
gtgcctgaag gagcagagct gcagcaccaa gtaccgcacg ctaaggcagt gcgtggcggg 720
caaggagacc aacttcagcc tggcatccgg cctggaggcc aaggatgagt gccgcagcgc 780
catggaggcc ctgaagcaga agtcgctcta caactgccgc tgcaagcggg gtatgaagaa 840
ggagaagaac tgcctgcgca tttactggag catgtaccag agcctgcagg gaaatgatct 900

gctggaggat tccccatatg aaccagttaa cagcagattg tcagatatat tccgggtggt 960
cccattcata tcagatgttt ttcagcaagt ggagcacatt cccaaaggga acaactgcct 1020
ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260
tgaagagagg gagaagccca actgtttgaa tttgcaggac tcctgcaaga cgaattacat 1320
ctgcagatct cgccttgcgg attttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg gggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160
aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 40
<211> 2560
<212> DNA
<213> human

<400> 40
gaattccggc cagaagaaat ctggcctcgg aacacgccat tctccgcgcc gcttccaata 60
accactaaca tccctaacga gcatccgagc cgagggctct gctcggaaat cgtcctggcc 120

caactcggcc cttcgagctc tcgaagatta ccgcatctat ttttttttc tttttttct 180
tttcctagcg cagataaagt gagcccggaa agggaaggag ggggcgggga caccattgcc 240
ctgaaagaat aaataagtaa ataaacaaac tggctcctcg ccgcagctgg acgcggtcgg 300
ttgagtccag gttgggtcgg acctgaaccc ctaaaagcgg aaccgcctcc cgccctcgcc 360
atcccggagc tgagtcgccg gcggcggtgg ctgctgccag acccggagtt tcctctttca 420
ctggatggag ctgaactttg ggcggccaga gcagcacagc tgtccgggga tcgctgcacg 480
ctgagctccc tcggcaagac ccagcggcgg ctcgggattt ttttgggggg gcggggacca 540
gccccgcgcc ggcaccatgt tcctggcgac cctgtacttc gcgctgccgc tcttggactt 600
gctcctgtcg gccgaagtga gcggcggaga ccgcctggat tgcgtgaaag ccagtgatca 660
gtgcctgaag gagcagagct gcagcaccaa gtaccgcacg ctaaggcagt gcgtggcggg 720
caaggagacc aacttcagcc tggcatccgg cctggaggcc aaggatgagt gccgcagcgc 780
catggaggcc ctgaagcaga agtcgctcta caactgccgc tgcaagcggg gtatgaagaa 840
ggagaagaac tgcctgcgca tttactggag catgtaccag agcctgcagg gaaatgatct 900
gctggaggat tccccatatg aaccagttaa cagcagattg tcagatatat tccgggtggt 960
cccattcata tcagatgttt ttcagcaagt ggagcacatt cccaaaggga acaactgcct 1020
ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260
tgaagagagg gagaagccca actgtttgaa tttgcaggac tcctgcaaga cgaattacat 1320
ctgcagatct cgccttgcgg attttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg gggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160

aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 41
<211> 2439
<212> DNA
<213> human

<400> 41
cagcacccag ctccccgcca ccgccatggt ccccgacacc gcctgcgttc ttctgctcac 60
cctggctgcc ctcggcgcgt ccggacaggg ccagagcccg ttgggctcag acctgggccc 120
gcagatgctt cgggaactgc aggaaaccaa cgcggcgctg caggacgtgc gggactggct 180
gcggcagcag gtcagggaga tcacgttcct gaaaaacacg gtgatggagt gtgacgcgtg 240
cgggatgcag cagtcagtac gcaccggcct acccagcgtg cggcccctgc tccactgcgc 300
gcccggcttc tgcttccccg gcgtggcctg catccagacg gagagcggcg gccgctgcgg 360
cccctgcccc gcgggcttca cgggcaacgg ctcgcactgc accgacgtca acgagtgcaa 420
cgcccacccc tgcttccccc gagtccgctg tatcaacacc agcccggggt tccgctgcga 480
ggcttgcccg ccggggtaca gcggccccac ccaccagggc gtggggctgg ctttcgccaa 540
ggccaacaag caggtttgca cggacatcaa cgagtgtgag accgggcaac ataactgcgt 600
ccccaactcc gtgtgcatca acacccgggg ctccttccag tgcggcccgt gccagcccgg 660
cttcgtgggc gaccaggcgt ccggctgcca gcgcggcgca cagcgcttct gccccgacgg 720
ctcgcccagc gagtgccacg agcatgcaga ctgcgtccta gagcgcgatg gctcgcggtc 780
gtgcgtgtgt cgcgttggct gggccggcaa cgggatcctc tgtggtcgcg acactgacct 840
agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440

cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920
gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct gggggtcttc tgcttctccc aggagaacat 2220
catctgggcc aacctgcgtt accgctgcaa tgacaccatc ccagaggact atgagaccca 2280
tcagctgcgg caagcctagg gaccagggtg aggacccgcc ggatgacagc caccctcacc 2340
gcggctggat gggggctctg cacccagccc aaggggtggc cgtcctgagg gggaagtgag 2400
aagggctcag agaggacaaa ataaagtgtg tgtgcaggg 2439

<210> 42
<211> 2439
<212> DNA
<213> human

<400> 42
cagcacccag ctccccgcca ccgccatggt ccccgacacc gcctgcgttc ttctgctcac 60
cctggctgcc ctcggcgcgt ccggacaggg ccagagcccg ttgggctcag acctgggccc 120
gcagatgctt cgggaactgc aggaaaccaa cgcggcgctg caggacgtgc gggactggct 180
gcggcagcag gtcagggaga tcacgttcct gaaaaacacg gtgatggagt gtgacgcgtg 240
cgggatgcag cagtcagtac gcaccggcct acccagcgtg cggcccctgc tccactgcgc 300
gcccggcttc tgcttccccg gcgtggcctg catccagacg gagagcggcg gccgctgcgg 360
cccctgcccc gcgggcttca cgggcaacgg ctcgcactgc accgacgtca acgagtgcaa 420
cgcccacccc tgcttccccc gagtccgctg tatcaacacc agcccggggt tccgctgcga 480
ggcttgcccg ccggggtaca gcggccccac ccaccagggc gtggggctgg ctttcgccaa 540
ggccaacaag caggtttgca cggacatcaa cgagtgtgag accgggcaac ataactgcgt 600
ccccaactcc gtgtgcatca acacccgggg ctccttccag tgcggcccgt gccagcccgg 660
cttcgtgggc gaccaggcgt ccggctgcca gcgcggcgca cagcgcttct gccccgacgg 720
ctcgcccagc gagtgccacg agcatgcaga ctgcgtccta gagcgcgatg gctcgcggtc 780

gtgcgtgtgt cgcgttggct gggccggcaa cgggatcctc tgtggtcgcg acactgacct 840
agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440
cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920
gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct gggggtcttc tgcttctccc aggagaacat 2220
catctgggcc aacctgcgtt accgctgcaa tgacaccatc ccagaggact atgagaccca 2280
tcagctgcgg caagcctagg gaccagggtg aggacccgcc ggatgacagc caccctcacc 2340
gcggctggat gggggctctg cacccagccc aaggggtggc cgtcctgagg gggaagtgag 2400
aagggctcag agaggacaaa ataaagtgtg tgtgcaggg 2439

<210> 43
<211> 1385
<212> DNA
<213> human

<400> 43
ctccagccat tggtgtctgt gtcattacta atagagtctt gtaaacactc gttaatcacg 60
gaagccgccg gcctggggct ccgcacgcca gcctgtgcgg gtcttccccg cctctgcagc 120
ctagtgggaa ggaggtggga ggaaagaagg aagaaaggga gggagggagg aggcaggcca 180

gagggaggga ccgcctcgga ggcagaagag ccgcgaggag ccagcggagc accgcgggct 240
ggggcgcagc cacccgccgc tcctcgagtc ccctcgcccc tttcccttcg tgccccccgg 300
cagcctccag cgtcggtccc caggcagcat ggtgaggtct gctcccggtc cctcgccacc 360
atgtacgtga gctacctcct ggacaaggac gtgagcatgt accctagctc cgtgcgccac 420
tctggcggcc tcaacctggc gccgcagaac ttcgtcagcc ccccgcagta cccggactac 480
ggcggttacc acgtggcggc cgcagctgca gcggcagcga acttggacag cgcgcagtcc 540
ccggggccat cctggccggc agcgtatggc gccccactcc gggaggactg gaatggctac 600
gcgcccggag gcgccgcggc cgccgccaac gccgtggctc acggcctcaa cggtggctcc 660
ccggccgcag ccatgggcta cagcagcccc gcagactacc atccgcacca ccacccgcat 720
caccacccgc accacccggc cgcggcgcct tcctgcgctt ctgggctgct gcaaacgctc 780
aaccccggcc ctcctgggcc cgccgccacc gctgccgccg agcagctgtc tcccggcggc 840
cagcggcgga acctgtgcga gtggatgcgg aagccggcgc agcagtccct cggcagccaa 900
gtgaaaacca ggacgaaaga caaatatcga gtggtgtaca cggaccacca gcggctggag 960
ctggagaagg agtttcacta cagtcgctac atcaccatcc ggaggaaagc cgagctagcc 1020
gccacgctgg ggctctctga gaggcaggtt aaaatctggt ttcagaaccg cagagcaaag 1080
gagaggaaaa tcaacaagaa gaagttgcag cagcaacagc agcagcagcc accacagccg 1140
cctccgccgc caccacagcc tccccagcct cagccaggtc ctctgagaag tgtcccagag 1200
cccttgagtc cggtgtcttc cctgcaagcc tcagtgtctg gctctgtccc tggggttctg 1260
gggccaactg ggggggtgct aaaccccacc gtcacccagt gacccaccgg ggttctgcag 1320
cggcagagca attccaggct gagccatgag gagcgtggac tctgctagac tcctcaggag 1380
agacc 1385

<210> 44
<211> 4098
<212> DNA
<213> human

<400> 44
ggtggcctct gtggccgtcc aggctagcgg cggcccgcag gcggcgggga gaaagactct 60
ctcacctggt cttgcggctg tggccaccgc cggccagggg tgtggagggc gtgctgccgg 120
agacgtccgc cgggctctgc agttccgccg ggggtcgggc agctatggag ccgcggccca 180
cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300
ctgcgcccgc gagccgggac ggcggcgggg tccgcgatga gggccccgcg gcggccgggg 360
acgggctggg cagacccttg gggcccaccc cgagccagag ccgtttccag gtggacctgg 420
tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540

gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atcttttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580

atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct ttttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840
tcagtgccta gtgtagtaac ctgaaatctt caatgacaca ttaacatcac aatggcgaat 3900
ggtgactttt ctttcacgat ttcattaatt tgaaagcaca caggaaagct tgctccattg 3960
ataacgtgta tggagacttc ggttttagtc aattccatat ctcaatctta atggtgattc 4020
ttctctgttg aactgaagtt tgtgagagta gttttccttt gctacttgaa tagcaataaa 4080
agcgtgttaa ctttttgg 4098

<210> 45
<211> 4098
<212> DNA
<213> human

<400> 45
ggtggcctct gtggccgtcc aggctagcgg cggcccgcag gcggcgggga gaaagactct 60
ctcacctggt cttgcggctg tggccaccgc cggccagggg tgtggagggc gtgctgccgg 120
agacgtccgc cgggctctgc agttccgccg ggggtcgggc agctatggag ccgcggccca 180
cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300

ctgcgcccgc gagccgggac ggcggcgggg tccgcgatga gggccccgcg gcggccgggg 360
acgggctggg cagacccttg gggcccaccc cgagccagag ccgtttccag gtggacctgg 420
tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540
gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atctttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340

taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct ttttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840
tcagtgccta gtgtagtaac ctgaaatctt caatgacaca ttaacatcac aatggcgaat 3900
ggtgactttt ctttcacgat ttcattaatt tgaaagcaca caggaaagct tgctccattg 3960
ataacgtgta tggagacttc ggttttagtc aattccatat ctcaatctta atggtgattc 4020
ttctctgttg aactgaagtt tgtgagagta gttttccttt gctacttgaa tagcaataaa 4080
agcgtgttaa ctttttgg 4098

<210> 46
<211> 3311
<212> DNA
<213> human

<400> 46

tgctaatgct tttggtacaa atggatgtgg aatataattg aatattttct tgtttaaggg 60
gagcatgaag aggtgttgag gttatgtcaa gcatctggca cagctgaagg cagatggaaa 120
tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040

tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520
aagcaagtgt gtttcagcag aacatcctcg ggaggctcat ttgtggcttc tgatgtccca 2580
aatgctccca tacctgatct cttcccacct ggccaaatca ccgacctgaa ggcggaaatt 2640
cacgggggca gtctcattaa tctgacttgg acagctcctg gggatgatta tgaccatgga 2700
acagctcaca agtatatcat tcgaataagt acaagtattc ttgatctcag agacaagttc 2760
aatgaatctc ttcaagtgaa tactactgct ctcatcccaa aggaagccaa ctctgaggaa 2820
gtctttttgt ttaaaccaga aaacattact tttgaaaatg gcacagatct tttcattgct 2880
attcaggctg ttgataaggt cgatctgaaa tcagaaatat ccaacattgc acgagtatct 2940
ttgtttattc ctccacagac tccgccagag acacctagtc ctgatgaaac gtctgctcct 3000
tgtcctaata ttcatatcaa cagcaccatt cctggcattc acattttaaa aattatgtgg 3060
aagtggatag gagaactgca gctgtcaata gcctagggct gaatttttgt cagataaata 3120
aaataaatca ttcatccttt ttttgattat aaaatttttct aaaatgtatt ttagacttcc 3180
tgtagggggc gatatactaa atgtatatag tacatttata ctaaatgtat tcctgtaggg 3240
ggcgatatac taaatgtatt ttagacttcc tgtagggggc gataaaataa aatgctaaac 3300
aactgggtaa a 3311

<210> 47
<211> 3311
<212> DNA
<213> human

<400> 47
tgctaatgct tttggtacaa atggatgtgg aatataattg aatattttct tgtttaaggg 60
gagcatgaag aggtgttgag gttatgtcaa gcatctggca cagctgaagg cagatggaaa 120
tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540

ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520
aagcaagtgt gtttcagcag aacatcctcg ggaggctcat ttgtggcttc tgatgtccca 2580

aatgctccca tacctgatct cttcccacct ggccaaatca ccgacctgaa ggcggaaatt 2640
cacgggggca gtctcattaa tctgacttgg acagctcctg gggatgatta tgaccatgga 2700
acagctcaca agtatatcat tcgaataagt acaagtattc ttgatctcag agacaagttc 2760
aatgaatctc ttcaagtgaa tactactgct ctcatcccaa aggaagccaa ctctgaggaa 2820
gtctttttgt ttaaaccaga aaacattact tttgaaaatg gcacagatct tttcattgct 2880
attcaggctg ttgataaggt cgatctgaaa tcagaaatat ccaacattgc acgagtatct 2940
ttgtttattc ctccacagac tccgccagag acacctagtc ctgatgaaac gtctgctcct 3000
tgtcctaata ttcatatcaa cagcaccatt cctggcattc acattttaaa aattatgtgg 3060
aagtggatag gagaactgca gctgtcaata gcctagggct gaatttttgt cagataaata 3120
aaataaatca ttcatccttt ttttgattat aaaattttct aaaatgtatt ttagacttcc 3180
tgtagggggc gatatactaa atgtatatag tacatttata ctaaatgtat tcctgtaggg 3240
ggcgatatac taaatgtatt ttagacttcc tgtagggggc gataaaataa aatgctaaac 3300
aactgggtaa a 3311

<210> 48
<211> 3697
<212> DNA
<213> human

<400> 48
agggagtgtt cccgggggag atactccagt cgtagcaaga gtctcgacca ctgaatggaa 60
gaaaaggact tttaaccacc attttgtgac ttacagaaag gaatttgaat aaagaaaact 120
atgatacttc aggcccatct tcactccctg tgtcttctta tgctttattt ggcaactgga 180
tatggccaag aggggaagtt tagtggaccc ctgaaaccca tgacattttc tatttatgaa 240
ggccaagaac cgagtcaaat tatattccag tttaaggcca atcctcctgc tgtgactttt 300
gaactaactg gggagacaga caacatattt gtgatagaac gggagggact tctgtattac 360
aacagagcct tggacaggga aacaagatct actcacaatc tccaggttgc agccctggac 420
gctaatggaa ttatagtgga gggtccagtc cctatcacca tagaagtgaa ggacatcaac 480
gacaatcgac ccacgtttct ccagtcaaag tacgaaggct cagtaaggca gaactctcgc 540
ccaggaaagc ccttcttgta tgtcaatgcc acagacctgg atgatccggc cactcccaat 600
ggccagcttt attaccagat tgtcatccag cttcccatga tcaacaatgt catgtacttt 660
cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020

gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccaccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa ttttttaaac agatattccc tcttgtcctt taatatttgc taaatatttc 2760
ttttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tcccttttgg ggcaagacag actcattaaa tattctgtac attttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 49
<211> 3697
<212> DNA
<213> human

<400> 49
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420

gctaatggaa ttatagtgga gggtccagtc cctatcacca tagaagtgaa ggacatcaac 480

gacaatcgac ccacgtttct ccagtcaaag tacgaaggct cagtaaggca gaactctcgc 540

ccaggaaagc ccttcttgta tgtcaatgcc acagacctgg atgatccggc cactcccaat 600

ggccagcttt attaccagat tgtcatccag cttcccatga tcaacaatgt catgtacttt 660

cagatcaaca acaaaacggg agccatctct cttacccgag agggatctca ggaattgaat 720

cctgctaaga atccttccta taatctggtg atctcagtga aggacatggg aggccagagt 780

gagaattcct tcagtgatac cacatctgtg gatatcatag tgacagagaa tatttggaaa 840

gcaccaaaac ctgtggagat ggtggaaaac tcaactgatc ctcaccccat caaaatcact 900

caggtgcggt ggaatgatcc cggtgcacaa tattccttag ttgacaaaga gaagctgcca 960

agattcccat tttcaattga ccaggaagga gatatttacg tgactcagcc cttggaccga 1020

gaagaaaagg atgcatatgt tttttatgca gttgcaaagg atgagtacgg aaaaccactt 1080

tcatatccgc tggaaattca tgtaaaagtt aaagatatta atgataatcc acctacatgt 1140

ccgtcaccag taaccgtatt tgaggtccag gagaatgaac gactgggtaa cagtatcggg 1200

acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag accctttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccaccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa ttttttaaac agatattccc tcttgtcctt taatatttgc taaatatttc 2760
ttttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120
cttgtgtttt tcccttttgg ggcaagacag actcattaaa tattctgtac atttttttctt 3180
tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 50
<211> 3803
<212> DNA
<213> human

<400> 50
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300

aaacgcgctg ggcgtctgct ccgacaccgc agcgcccctg gccgcggtgg acctcaagtg 360

ggagcacaac cccgctgtgt tcctgggccc cggctgcgtg tacgccgccg ccccagtggg 420

gcgcttcacc gcgcactggc gggtcccgct gctgaccgcc ggcgccccgg cgctgggctt 480

cggtgtcaag gacgagtatg cgctgaccac ccgcgcgggg cccagctacg ccaagctggg 540

ggacttcgtg gcggcgctgc accgacggct gggctgggag cgccaagcgc tcatgctcta 600

cgcctaccgg ccgggtgacg aagagcactg cttcttcctc gtggaggggc tgttcatgcg 660

ggtccgcgac cgcctcaata ttacggtgga ccacctggag ttcgccgagg acgacctcag 720

ccactacacc aggctgctgc ggaccatgcc gcgcaaaggc cgagttatct acatctgcag 780

ctcccctgat gccttcagaa ccctcatgct cctggccctg gaagctggct tgtgtgggga 840

ggactacgtt ttcttccacc tggatatctt tgggcaaagc ctgcaaggtg gacagggccc 900

tgctccccgc aggccctggg agagagggga tgggcaggat gtcagtgccc gccaggcctt 960

tcaggctgcc aaaatcatta catataaaga cccagataat cccgagtact tggaattcct 1020

gaagcagtta aaacacctgg cctatgagca gttcaacttc accatggagg atggcctggt 1080

gaacaccatc ccagcatcct tccacgacgg gctcctgctc tatatccagg cagtgacgga 1140

gactctggca catgggggaa ctgttactga tggggagaac atcactcagc ggatgtggaa 1200

ccgaagcttt caaggtgtga caggatacct gaaaattgat agcagtggcg atcgggaaac 1260

agacttctcc ctctgggata tggatcccga gaatggtgcc ttcagggttg tactgaacta 1320

caatgggact tcccaagagc tggtggctgt gtcggggcgc aaactgaact ggcccctggg 1380
gtaccctcct cctgacatcc ccaaatgtgg ctttgacaac gaagacccag catgcaacca 1440
agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg ggctatctgt ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160
cctgcgaatg gcttcacccc ctgtgcgggg ctcccaggct ggtgacgtat acagctttgg 2220
gatcatcctt caggagattg ccctgaggag tggggtcttc cacgtggaag gtttggacct 2280
gagccccaaa gagatcatcg agcgggtgac tcggggtgag cagcccccct tccggccctc 2340
cctggccctg cagagtcacc tggaggagtt ggggctgctc atgcagcggt gctgggctga 2400
ggacccacag gagaggccac cattccagca gatccgcctg acgttgcgca aatttaacag 2460
ggagaacagc agcaacatcc tggacaacct gctgtcccgc atggagcagt acgcgaacaa 2520
tctggaggaa ctggtggagg agcggaccca ggcatacctg gaggagaagc gcaaggctga 2580
ggccctgctc taccagatcc tgcctcactc agtggctgag cagctgaagc gtggggagac 2640
ggtgcaggcc gaagcctttg acagtgttac catctacttc agtgacattg tgggtttcac 2700
agcgctgtcg gcggagagca cacccatgca ggtggtgacc ctgctcaatg acctgtacac 2760
ttgctttgat gctgtcatag acaactttga tgtgtacaag gtggagacaa ttggcgatgc 2820
ctacatggtg gtgtcagggc tccctgtgcg gaacgggcgg ctacacgcct gcgaggtagc 2880
ccgcatggcc ctggcactgc tggatgctgt gcgctccttc cgaatccgcc accggcccca 2940
ggagcagctg cgcttgcgca ttggcatcca cacaggacct gtgtgtgctg gagtggtggg 3000
actgaagatg ccccgttact gtctctttgg ggatacagtc aacacagcct caagaatgga 3060
gtctaatggg gaagccctga agatccactt gtcttctgag accaaggctg tcctggagga 3120
gtttggtggt ttcgagctgg agcttcgagg ggatgtagaa atgaagggca aaggcaaggt 3180
tcggacctac tggctccttg ggagagggg gagtagcacc cgaggctgac ctgcctcctc 3240
tcctatccct ccacacctcc cctaccctgt gccagaagca acagaggtgc caggcctcag 3300
cctcacccac agcagcccca tcgccaaagg atggaagtaa tttgaatagc tcaggtgtgc 3360

tgaccccagt gaagacacca gataggacct ctgagagggg actggcatgg ggggatctca 3420
gagcttacag gctgagccaa gcccacggcc atgcacaggg acactcacac aggcacacgc 3480
acctgctctc cacctggact caggccgggc tgggctgtgg atccttgatc ccctcccctc 3540
cccatgctct cctccctcag ccttgctacc ctgtgactta ctgggaggag agtcacctga 3600
aggggaacat gaaaagagac taggtgaaga gagggcaggg gagcccacat ctggggctgg 3660
cccacaatac ctgctccccc gaccccctcc acccagcagt agacacagtg cacaggggag 3720
aagaggggtg gcgcagaagg gttgggggcc tgtatgcctt gcttctacca tgagcagaga 3780
caattaaaat ctttattcca gtg 3803

<210> 51
<211> 3803
<212> DNA
<213> human

<400> 51
ccatggtagg agcgctcgcc tcgctgcggt gcccgctgag gccatgccgg ggccccggcg 60
ccccgctggc tcccgcctgc gcctgctcct gctcctgctg ctgccgccgc tgctgctgct 120
gctccggggc agccacgcgg gcaacctgac ggtagccgtg gtactgccgc tggccaatac 180
ctcgtacccc tggtcgtggg cgcgcgtggg acccgccgtg gagctggccc tggcccaggt 240
gaaggcgcgc cccgacttgc tgccgggctg gacggtccgc acggtgctgg gcagcagcga 300
aaacgcgctg ggcgtctgct ccgacaccgc agcgcccctg gccgcggtgg acctcaagtg 360
ggagcacaac cccgctgtgt tcctgggccc cggctgcgtg tacgccgccg ccccagtggg 420
gcgcttcacc gcgcactggc gggtcccgct gctgaccgcc ggcgccccgg cgctgggctt 480
cggtgtcaag gacgagtatg cgctgaccac ccgcgcgggg cccagctacg ccaagctggg 540
ggacttcgtg gcggcgctgc accgacggct gggctgggag cgccaagcgc tcatgctcta 600
cgcctaccgg ccgggtgacg aagagcactg cttcttcctc gtggaggggc tgttcatgcg 660
ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380

gtaccctcct cctgacatcc ccaaatgtgg ctttgacaac gaagacccag catgcaacca 1440
agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg ggctatctg ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160
cctgcgaatg gcttcacccc ctgtgcgggg ctcccaggct ggtgacgtat acagctttgg 2220
gatcatcctt caggagattg ccctgaggag tggggtcttc cacgtggaag gtttggacct 2280
gagccccaaa gagatcatcg agcgggtgac tcggggtgag cagcccccct tccggccctc 2340
cctggccctg cagagtcacc tggaggagtt ggggctgctc atgcagcggt gctgggctga 2400
ggacccacag gagaggccac cattccagca gatccgcctg acgttgcgca aatttaacag 2460
ggagaacagc agcaacatcc tggacaacct gctgtcccgc atggagcagt acgcgaacaa 2520
tctggaggaa ctggtggagg agcggaccca ggcatacctg gaggagaagc gcaaggctga 2580
ggccctgctc taccagatcc tgcctcactc agtggctgag cagctgaagc gtggggagac 2640
ggtgcaggcc gaagcctttg acagtgttac catctacttc agtgacattg tgggtttcac 2700
agcgctgtcg gcggagagca cacccatgca ggtggtgacc ctgctcaatg acctgtacac 2760
ttgctttgat gctgtcatag acaactttga tgtgtacaag gtggagacaa ttggcgatgc 2820
ctacatggtg gtgtcagggc tccctgtgcg gaacgggcgg ctacacgcct gcgaggtagc 2880
ccgcatggcc ctggcactgc tggatgctgt gcgctccttc cgaatccgcc accggcccca 2940
ggagcagctg cgcttgcgca ttggcatcca cacaggacct gtgtgtgctg gagtggtggg 3000
actgaagatg ccccgttact gtctctttgg ggatacagtc aacacagcct caagaatgga 3060
gtctaatggg gaagccctga agatccactt gtcttctgag accaaggctg tcctggagga 3120
gtttggtggt ttcgagctgg agcttcgagg ggatgtagaa atgaagggca aaggcaaggt 3180
tcggacctac tggctccttg gggagagggg gagtagcacc cgaggctgac ctgcctcctc 3240
tcctatccct ccacacctcc cctaccctgt gccagaagca acagaggtgc caggcctcag 3300
cctcacccac agcagcccca tcgccaaagg atggaagtaa tttgaatagc tcaggtgtgc 3360
tgaccccagt gaagacacca gataggacct ctgagagggg actggcatgg ggggatctca 3420

gagcttacag gctgagccaa gcccacggcc atgcacaggg acactcacac aggcacacgc 3480
acctgctctc cacctggact caggccgggc tgggctgtgg atccttgatc ccctcccctc 3540
cccatgctct cctccctcag ccttgctacc ctgtgactta ctgggaggag agtcacctga 3600
aggggaacat gaaaagagac taggtgaaga gagggcaggg gagcccacat ctggggctgg 3660
cccacaatac ctgctccccc gaccccctcc acccagcagt agacacagtg cacaggggag 3720
aagaggggtg gcgcagaagg gttgggggcc tgtatgcctt gcttctacca tgagcagaga 3780
caattaaaat ctttattcca gtg 3803

<210> 52
<211> 1155
<212> DNA
<213> human

<400> 52
atggattgca gtaacggatc ggcagagtgt accggagaag gaggatcaaa agaggtggtg 60
gggactttta aggctaaaga cctaatagtc acaccagcta ccattttaaa ggaaaaacca 120
gaccccaata atctggtttt tggaactgtg ttcacggatc atatgctgac ggtggagtgg 180
tcctcagagt ttggatggga gaaacctcat atcaagcctc ttcagaacct gtcattgcac 240
cctggctcat cagctttgca ctatgcagtg gaattatttg aaggattgaa ggcatttcga 300
ggagtagata ataaaattcg actgtttcag ccaaacctca acatggatag aatgtatcgc 360
tctgctgtga gggcaactct gccggtattt gacaaagaag agctcttaga gtgtattcaa 420
cagcttgtga aattggatca agaatgggtc ccatattcaa catctgctag tctgtatatt 480
cgtcctgcat tcattggaac tgagccttct cttggagtca agaagcctac caaagccctg 540
ctctttgtac tcttgagccc agtgggacct tatttttcaa gtggaacctt taatccagtg 600
tccctgtggg ccaatcccaa gtatgtaaga gcctggaaag gtggaactgg ggactgcaag 660
atgggaggga attacggctc atctcttttt gcccaatgtg aagacgtaga taatgggtgt 720
cagcaggtcc tgtggctcta tggcagagac catcagatca ctgaagtggg aactatgaat 780
ctttttcttt actggataaa tgaagatgga gaagaagaac tggcaactcc tccactagat 840
ggcatcattc ttccaggagt gacaaggcgg tgcattctgg acctggcaca tcagtggggt 900
gaatttaagg tgtcagagag atacctcacc atggatgact tgacaacagc cctggagggg 960
aacagagtga gagagatgtt tagctctggt acagcctgtg ttgtttgccc agtttctgat 1020
atactgtaca aaggcgagac aatacacatt ccaactatgg agaatggtcc taagctggca 1080
agccgcatct tgagcaaatt aactgatatc cagtatggaa gagaagagag cgactggaca 1140
attgtgctat cctga 1155

<210> 53
<211> 2511
<212> DNA
<213> human

<400> 53

```
cttttcacac tggccttaaa gaggatatat tagaagttga agtaggaagg gagccagaga      60
ggccgatggc gcaaaggtac gacgatctac cccattacgg gggcatggat ggagtaggca     120
tcccctccac gatgtatggg gacccgcatg cagccaggtc catgcagccg gtccaccacc     180
tgaaccacgg gcctcctctg cactcgcatc agtacccgca cacagctcat accaacgcca     240
tggcccccag catgggctcc tctgtcaatg acgctttaaa gagagataaa gatgccattt     300
atggacaccc cctcttccct ctcttagcac tgatttttga gaaatgtgaa ttagctactt     360
gtacccccg cgagccgggg gtggcgggcg gggacgtctg ctcgtcagag tcattcaatg      420
aagatatagc cgtgttcgcc aaacagattc gcgcagaaaa acctctattt tcttctaatc     480
cagaactgga taacttgatg attcaagcca tacaagtatt aaggtttcat ctattggaat     540
tagagaaggt acacgaatta tgtgacaatt tctgccaccg gtatattagc tgtttgaaag     600
ggaaaatgcc tatcgatttg gtgatagacg atagagaagg aggatcaaaa tcagacagtg     660
aagatataac aagatcagca aatctaactg accagccctc ttggaacaga gatcatgatg     720
acacggcatc tactcgttca ggaggaaccc caggcccttc cagcggtggc cacacgtcac     780
acagtgggga caacagcagt gagcaaggtg atggcttgga caacagtgta gcttccccca     840
gcacaggtga cgatgatgac cctgataagg acaaaaagcg tcacaaaaag cgtggcatct     900
ttcccaaagt agccacaaat atcatgaggg cgtggctgtt ccagcatcta acacaccctt     960
acccttctga agaacagaaa aagcagttgg cacaagacac gggactcacc atccttcaag    1020
tgaacaattg gtttattaat gcccggagaa gaatagtgca gcccatgata gaccagtcca    1080
accgagcagt aagtcaagga acaccttata atcctgatgg acagcccatg ggaggtttcg    1140
taatggacgg tcagcaacat atgggaatta gagcaccagg acctatgagt ggaatgggca    1200
tgaatatggg catggagggg cagtggcact acatgtaacc ttcatctagt taaccaatcg    1260
caaagcaagg gggaaggctg caaagtatgc caggggagta tgtagcccgg ggtggtccaa    1320
tgggtgtgag tatgggacag ccaagttata cccaacccca gatgcccccc catcctgctc    1380
agctgcgtca tgggccccc atgcatacgt acattcctgg acaccctcac cacccaacag     1440
tgatgatgca tggaggaccg ccccaccctg gaatgccaat gtcagcatca agccccacag    1500
ttcttaatac aggagaccca acaatgagtg gacaagtcat ggacattcat gctcagtagc    1560
ttaagggaat atgcattgtc tgcaatggtg actgatttca aatcatgttt tttctgcaat    1620
gactgtggag ttccattctt ggcatctact ctggaccaag gagcatccct aattcttcat    1680
agggaccttt aaaaagcagg aaataccaac tgaagtcaat ttgggggaca tgctaaataa    1740
ctatataaga cattaagaga acaaagagtg aaatattgta aatgctatta tactgttatc    1800
catattacgt tgtttcttat agattttta aaaaaaatgt gaaattttc cacactatgt      1860
gtgttgtttc catagctctt cacttcctcc agaagcctcc ttacattaaa aagccttaca    1920
gttatcctgc aagggacagg aaggtctgat ttgcaggatt tttagagcat taaaataact    1980
atcaggcaga agaatctttc ttctcgccta ggatttcagc catgcgcgcg ctctctctct    2040
```

ttctctctct tttcctctct ctccctcttt ctagcctggg gcttgaattt gcatgtctaa 2100

ttcatttact caccatattt gaattggcct gaacagatgt aaatcgggaa ggatgggaaa 2160

aactgcagtc atcaacaatg attaatcagc tgttgcaggc agtgtcttaa ggagactggt 2220

aggaggaggc atggaaacca aaaggccgtg tgtttagaag cctaattgtc acatcaagca 2280

tcattgtccc catgcaacaa ccaccacctt atacatcact tcctgtttta agcagctcta 2340

aaacatagac tgaagattta tttttaatat gttgacttta tttctgagca aagcatcggt 2400

catgtgtgta ttttttcata gtcccacctt ggagcattta tgtagacatt gtaaataaat 2460

tttgtgcaaa aaggactgga aaaatgaact gtattattgc aatttttttt t 2511

<210> 54
<211> 1887
<212> DNA
<213> human

<400> 54
tcgcccaatt ccgggctcag acactgggct cccagctggg gactgctcca tggccatgga 60

gatagacagc aggcctgggg ggctccccgg cagtagctgc aacctaggtg cagcccgaga 120

acacatgcag gcggtcaccc gaaactacat cacccacccc cgtgtcacct acaggactgt 180

gtgcagcgtg aacgggcccc tggtggtgct ggaccgggtc aagtttgccc agtatgcgga 240

gatcgtccac ttcaccctcc cagatgggac tcagaggagc gggcaggtgc ttgaggtggc 300

tggcaccaag gcgattgttc aggtgtttga agggacatca gggatcgatg ccaggaagac 360

cacttgcgaa tttacagggg acatcctacg aactccggtg tcagaggaca tgctgggtcg 420

ggttttcaat ggctccggca agcccattga caaggggcca gtggtcatgg cggaggactt 480

tctggatatc aatggccagc ccatcaaccc gcactcccgc atctaccccg aggagatgat 540

tcagacgggc atttctccta ttgacgtcat gaacagcatt gcccgcggcc agaagatccc 600

catcttctca gcagccgggc tcccccacaa tgagattgcc gctcagatct gccgccaggc 660

ggggctggtg aagaagtcca aggctgtgct ggattaccat gacgacaact tcgccatcgt 720

ctttgcagcc atgggggtga acatggagac agccagattc ttcaagtctg actttgagca 780

gaatggaacc atggggaacg tctgcctctt cctgaacttg gccaatgacc ccacgatcga 840

gcggatcatc accccgcgcc tggcgctgac cactgctgaa ttccttgcct accagtgtga 900

gaagcatgtg ctggtcatac tgacggacat gagttcctat gcagaggcct tgcgggaggt 960

ctctgctgct agagaggagg tgcctgggcg ccgagggttt cctggatata tgtacacaga 1020

cctggccacc atctacgagc gggcgggccg tgtggagggt cggggaggat ccatcacaca 1080

gatccccatc ctcaccatgc ccaacgacga tatcacccac cctatcccag acttgacggg 1140

cttcatcaca gagggacaga tctacgtgga cagacagctt cacaacagac agatctaccc 1200

ccccatcaac gtgctccctt ccctgtcgcg gctgatgaag tcagccattg gggaaggcat 1260

gacaagaaag gaccatggag atgtctccaa ccagctgtac gcctgctatg ccatcgggaa 1320

ggacgtgcag gccatgaagg cagtagttgg ggaggaggcg ctcacctctg aggacctgct 1380
ctacctggaa ttcctgcaga agtttgagaa gaacttcatc aatcagggcc cctacgagaa 1440
ccgctcgatg ttcgagtcgc tggaccttag ctggaagctg ctgcgcatct tccccaagga 1500
gatgctgaag cgcattccgc aggccgtgat cgacgagttc tattcccgcg aggggcggct 1560
gcaggacctc gcgcctgaca ctgcgctcta gccccgcgcg ccgtggcacc ccaacaccgg 1620
caggaaccta ccctcggctc ccgggtctcc ccgtccctcg ccacccctaa ccagcggctt 1680
tcgcgccgcc ctccgccctc cgtggctccg aggtggtggg gggcgccgca gtcatccctt 1740
tcctcgctcg attccttttc ccgcgctcca tgcctccccc tcagctcccg gtgctgcgga 1800
agaactgaag gttcatgcct actctgacgg gagcatctgt attttttatg ttaaaagccc 1860
acaaaataaa aataaaaatg aactgag 1887

<210> 55
<211> 1506
<212> DNA
<213> human

<400> 55
aggcggacaa agcccgattg ttcctgggcc ctttccccat cgcgcctggg cctgctcccc 60
agcccggggc aggggcgggg gccagtgtgg tgacacacgc tgtagctgtc tccccggctg 120
gctggctcgc tctctcctgg ggacacagag gtcggcaggc agcacacaga gggacctacg 180
ggcagctgtt ccttcccccg actcaagaat ccccggaggc ccggaggcct gcagcaggag 240
cggccatgaa gaagctgatg gtggtgctga gtctgattgc tgcagcctgg gcagaggagc 300
agaataagtt ggtgcatggc ggaccctgcg acaagacatc tcacccctac caagctgccc 360
tctacacctc gggccacttg ctctgtggtg gggtccttat ccatccactg tgggtcctca 420
cagctgccca ctgcaaaaaa ccgaatcttc aggtcttcct ggggaagcat aaccttcggc 480
aaagggagag ttcccaggag cagagttctg ttgtccgggc tgtgatccac cctgactatg 540
atgccgccag ccatgaccag gacatcatgc tgttgcgcct ggcacgccca gccaaactct 600
ctgaactcat ccagcccctt cccctggaga gggactgctc agccaacacc accagctgcc 660
acatcctggg ctggggcaag acagcagatg gtgatttccc tgacaccatc cagtgtgcat 720
acatccacct ggtgtcccgt gaggagtgtg agcatgccta ccctggccag atcacccaga 780
acatgttgtg tgctggggat gagaagtacg ggaaggattc ctgccagggt gattctgggg 840
gtccgctggt atgtggagac cacctccgag gccttgtgtc atggggtaac atcccctgtg 900
gatcaaagga gaagccagga gtctacacca acgtctgcag atacacgaac tggatccaaa 960
aaaccattca ggccaagtga ccctgacatg tgacatctac ctcccgacct accaccccac 1020
tggctggttc cagaacgtct ctcacctaga ccttgcctcc cctcctctcc tgcccagctc 1080
tgaccctgat gcttaataaa cgcagcgacg tgagggtcct gattctccct ggttttaccc 1140
cagctccatc cttgcatcac tggggaggac gtgatgagtg aggacttggg tcctcggtct 1200
tacccccacc actaagagaa tacaggaaaa tcccttctag gcatctcctc tccccaaccc 1260

ttccacacgt ttgatttctt cctgcagagg cccagccacg tgtctggaat cccagctccg 1320
ctgcttactg tcggtgtccc cttgggatgt acctttcttc actgcagatt tctcacctgt 1380
aagatgaaga taaggatgat acagtctcca tcaggcagtg gctgttggaa agatttaaga 1440
tttcacacct atgacataca tgggatagca cctgggccgc catgcactca ataaagaatg 1500
tatttt 1506

<210> 56
<211> 2907
<212> DNA
<213> human

<400> 56
gccatctggg cccaggcccc atgccccgag gaggggtggt ctgaagccca ccagagcccc 60
ctgccagact gtctgcctcc cttctgactg tggccgcttg gcatggccag caacagcagc 120
tcctgcccga cacctggggg cgggcacctc aatgggtacc cggtgcctcc ctacgccttc 180
ttcttccccc ctatgctggg tggactctcc ccgccaggcg ctctgaccac tctccagcac 240
cagcttccag ttagtggata tagcacacca tccccagcca ccattgagac ccagagcagc 300
agttctgaag agatagtgcc cagccctccc tcgccacccc ctctaccccg catctacaag 360
ccttgctttg tctgtcagga caagtcctca ggctaccact atggggtcag cgcctgtgag 420
ggctgcaagg gcttcttccg ccgcagcatc cagaagaaca tggtgtacac gtgtcaccgg 480
gacaagaact gcatcatcaa caaggtgacc cggaaccgct gccagtactg ccgactgcag 540
aagtgctttg aagtgggcat gtccaaggag tctgtgagaa acgaccgaaa caagaagaag 600
aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg ccccctcacc 1020
gacctggtct ttgccttcgc caaccagctg ctgcccctgg agatggatga tgcggagacg 1080
gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca agggggctga gcgggtgatc acgctgaaga tggagatccc gggctccatg 1320
ccgcctctca tccaggaaat gttggagaac tcagagggcc tggacactct gagcggacag 1380
ccgggggggtg gggggcggga cgggggtggc ctggcccccc cgccaggcag ctgtagcccc 1440
agcctcagcc ccagctccaa cagaagcagc ccggccaccc actccccgtg accgcccacg 1500

ccacatggac acagccctcg ccctccgccc cggcttttct ctgcctttct accgaccatg 1560

tgaccccgca ccagccctgc ccccacctgc cctcccgggc agtactgggg accttccctg 1620

ggggacgggg agggaggagg cagcgactcc ttggacagag gcctgggccc tcagtggact 1680

gcctgctccc acagcctggg ctgacgtcag aggccgaggc caggaactga gtgaggcccc 1740

tggtcctggg tctcaggatg ggtcctgggg gcctcgtgtt catcaagaca cccctctgcc 1800

cagctcacca catcttcatc accagcaaac gccaggactt ggctccccca tcctcagaac 1860

tcacaagcca ttgctcccca gctggggaac ctcaacctcc cccctgcctc ggttggtgac 1920

agagggggtg ggacaggggc ggggggttcc ccctgtacat accctgccat accaacccca 1980

ggtattaatt ctcgctggtt ttgtttttat tttaattttt ttgttttgat ttttttaata 2040

agaattttca ttttaagcac atttatactg aaggaatttg tgctgtgtat tggggggagc 2100

tggatccaga gctggagggg gtgggtccgg gggagggagt ggctcggaag gggcccccac 2160

tctcctttca tgtccctgtg ccccccagtt ctcctcctca gccttttcct cctcagtttt 2220

ctctttaaaa ctgtgaagta ctaactttcc aaggcctgcc ttcccctccc tcccactgga 2280

gaagccgcca gccccttttct ccctctgcct gaccactggg tgtggacggt gtggggcagc 2340

cctgaaagga caggctcctg gccttggcac ttgcctgcac ccaccatgag gcatggagca 2400

gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg ccccctcctt accccgcagg acgggcctac aggggggtct cccctcaccc 2760

ctgcaccccc agctggggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 57
<211> 2907
<212> DNA
<213> human

<400> 57
gccatctggg cccaggcccc atgccccgag gaggggtggt ctgaagccca ccagagcccc 60

ctgccagact gtctgcctcc cttctgactg tggccgcttg gcatggccag caacagcagc 120

tcctgcccga cacctggggg cgggcacctc aatgggtacc cggtgcctcc ctacgccttc 180

ttcttccccc ctatgctggg tggactctcc ccgccaggcg ctctgaccac tctccagcac 240

cagcttccag ttagtggata tagcacacca tccccagcca ccattgagac ccagagcagc 300

agttctgaag agatagtgcc cagccctccc tcgccacccc ctctaccccg catctacaag 360

ccttgctttg tctgtcagga caagtcctca ggctaccact atggggtcag cgcctgtgag 420

ggctgcaagg gcttcttccg ccgcagcatc cagaagaaca tggtgtacac gtgtcaccgg 480
gacaagaact gcatcatcaa caaggtgacc cggaaccgct gccagtactg ccgactgcag 540
aagtgctttg aagtgggcat gtccaaggag tctgtgagaa acgaccgaaa caagaagaag 600
aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg cccccctcacc 1020
gacctggtct ttgccttcgc caaccagctg ctgcccctgg agatggatga tgcggagacg 1080
gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca agggggctga gcgggtgatc acgctgaaga tggagatccc gggctccatg 1320
ccgcctctca tccaggaaat gttggagaac tcagagggcc tggacactct gagcggacag 1380
ccggggggtg gggggcggga cgggggtggc ctggcccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680
gcctgctccc acagcctggg ctgacgtcag aggccgaggc caggaactga gtgaggcccc 1740
tggtcctggg tctcaggatg ggtcctgggg gcctcgtgtt catcaagaca cccctctgcc 1800
cagctcacca catcttcatc accagcaaac gccaggactt ggctccccca tcctcagaac 1860
tcacaagcca ttgctcccca gctggggaac ctcaacctcc cccctgcctc ggttggtgac 1920
agagggggtg ggacaggggc gggggggttcc ccctgtacat accctgccat accaacccca 1980
ggtattaatt ctcgctggtt ttgtttttat tttaattttt ttgttttgat ttttttaata 2040
agaatttca ttttaagcac atttatactg aaggaatttg tgctgtgtat tggggggagc 2100
tggatccaga gctggagggg gtgggtccgg gggagggagt ggctcggaag ggcccccac 2160
tctcctttca tgtccctgtg ccccccagtt ctcctcctca gccttttcct cctcagtttt 2220
ctctttaaaa ctgtgaagta ctaactttcc aaggcctgcc ttcccctccc tcccactgga 2280
gaagccgcca gcccctttct ccctctgcct gaccactggg tgtggacggt gtggggcagc 2340
cctgaaagga caggctcctg gccttggcac ttgcctgcac ccaccatgag gcatggagca 2400
gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg ccccctcctt accccgcagg acgggcctac agggggtct cccctcaccc 2760

ctgcaccccc agctggggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 58
<211> 5026
<212> DNA
<213> human

<400> 58
agaggaggaa attgttcctc gtctgataag acaacagtgg agaaaggacg catgctgttt 60

cttagggaca cggctgactt ccagatatga ccatgtattt gtggcttaaa ctcttggcat 120

ttggctttgc ctttctggac acagaagtat ttgtgacagg gcaaagccca acaccttccc 180

ccactggatt gactacagca aagatgccca gtgttccact ttcaagtgac cccttaccta 240

ctcacaccac tgcattctca cccgcaagca cctttgaaag agaaaatgac ttctcagaga 300

ccacaacttc tcttagtcca gacaatactt ccacccaagt atccccggac tctttggata 360

atgctagtgc ttttaatacc acaggtgttt catcagtaca gacgcctcac cttcccacgc 420

acgcagactc gcagacgccc tctgctggaa ctgacacgca gacattcagc ggctccgccg 480

ccaatgcaaa actcaaccct accccaggca gcaatgctat ctcagatgtc ccaggagaga 540

ggagtacagc cagcaccttt cctacagacc cagtttcccc attgacaacc accctcagcc 600

ttgcacacca cagctctgct gccttacctg cacgcacctc caacaccacc atcacagcga 660

acacctcaga tgcctacctt aatgcctctg aaacaaccac tctgagccct tctggaagcg 720

ctgtcatttc aaccacaaca atagctacta ctccatctaa gccaacatgt gatgaaaaat 780

atgcaaacat cactgtggat tacttatata acaaggaaac taaattattt acagcaaagc 840

taaatgttaa tgagaatgtg gaatgtggaa acaatacttg cacaaacaat gaggtgcata 900

accttacaga atgtaaaaat gcgtctgttt ccatatctca taattcatgt actgctcctg 960

ataagacatt aatattagat gtgccaccag gggttgaaaa gtttcagtta catgattgta 1020

cacaagttga aaaagcagat actactattt gtttaaaatg gaaaaatatt gaaaccttta 1080

cttgtgatac acagaatatt acctacagat ttcagtgtgg taatatgata tttgataata 1140

aagaaattaa attagaaaac cttgaacccg aacatgagta taagtgtgac tcagaaatac 1200

tctataataa ccacaagttt actaacgcaa gtaaaattat taaaacagat tttgggagtc 1260

caggagagcc tcagattatt ttttgtagaa gtgaagctgc acatcaagga gtaattacct 1320

```
ggaatccccc tcaaagatca tttcataatt ttaccctctg ttatataaaa gagacagaaa    1380
aagattgcct caatctggat aaaaacctga tcaaatatga tttgcaaaat ttaaaacctt    1440
atacgaaata tgttttatca ttacatgcct acatcattgc aaaagtgcaa cgtaatggaa    1500
gtgctgcaat gtgtcatttc acaactaaaa gtgctcctcc aagccaggtc tggaacatga    1560
ctgtctccat gacatcagat aatagtatgc atgtcaagtg taggcctccc agggaccgta    1620
atggccccca tgaacgttac catttggaag ttgaagctgg aaatactctg gttagaaatg    1680
agtcgcataa gaattgcgat ttccgtgtaa aagatcttca atattcaaca gactacactt    1740
ttaaggccta ttttcacaat ggagactatc ctggagaacc ctttatttta catcattcaa    1800
catcttataa ttctaaggca ctgatagcat ttctggcatt tctgattatt gtgacatcaa    1860
tagccctgct tgttgttctc tacaaaatct atgatctaca taagaaaaga tcctgcaatt    1920
tagatgaaca gcaggagctt gttgaaaggg atgatgaaaa acaactgatg aatgtggagc    1980
caatccatgc agatattttg ttggaaactt ataagaggaa gattgctgat gaaggaagac    2040
tttttctggc tgaatttcag agcatcccgc gggtgttcag caagtttcct ataaaggaag    2100
ctcgaaagcc ctttaaccag aataaaaacc gttatgttga cattcttcct tatgattata    2160
accgtgttga actctctgag ataaacggag atgcagggtc aaactacata aatgccagct    2220
atattgatgg tttcaaagaa cccaggaaat acattgctgc acaaggtccc agggatgaaa    2280
ctgttgatga tttctggagg atgatttggg aacagaaagc cacagttatt gtcatggtca    2340
ctcgatgtga agaaggaaac aggaacaagt gtgcagaata ctggccgtca atggaagagg    2400
gcactcgggc ttttggagat gttgttgtaa agatcaacca gcacaaaaga tgtccagatt    2460
acatcattca gaaattgaac attgtaaata aaaaagaaaa agcaactgga agagaggtga    2520
ctcacattca gttcaccagc tggccagacc acggggtgcc tgaggatcct cacttgctcc    2580
tcaaactgag aaggagagtg aatgccttca gcaatttctt cagtggtccc attgtggtgc    2640
actgcagtgc tggtgttggg cgcacaggaa cctatatcgg aattgatgcc atgctagaag    2700
gcctggaagc cgagaacaaa gtggatgttt atggttatgt tgtcaagcta aggcgacaga    2760
gatgcctgat ggttcaagta gaggcccagt acatcttgat ccatcaggct ttggtggaat    2820
acaatcagtt tggagaaaca gaagtgaatt tgtctgaatt acatccatat ctacataaca    2880
tgaagaaaag ggatccaccc agtgagccgt ctccactaga ggctgaattc cagagacttc    2940
cttcatatag gagctggagg acacagcaca ttggaaatca agaagaaaat aaaagtaaaa    3000
acaggaattc taatgtcatc ccatatgact ataacagagt gccacttaaa catgagctgg    3060
aaatgagtaa agagagtgag catgattcag atgaatcctc tgatgatgac agtgattcag    3120
aggaaccaag caaatacatc aatgcatctt ttataatgag ctactggaaa cctgaagtga    3180
tgattgctgc tcagggacca ctgaaggaga ccattggtga cttttggcag atgatcttcc    3240
aaagaaaagt caaagttatt gttatgctga cagaactgaa acatggagac caggaaatct    3300
gtgctcagta ctggggagaa ggaaagcaaa catatggaga tattgaagtt gacctgaaag    3360
```

acacagacaa atcttcaact tatacccttc gtgtctttga actgagacat tccaagagga 3420
aagactctcg aactgtgtac cagtaccaat atacaaactg gagtgtggag cagcttcctg 3480
cagaacccaa ggaattaatc tctatgattc aggtcgtcaa acaaaaactt ccccagaaga 3540
attcctctga agggaacaag catcacaaga gtacacctct actcattcac tgcagggatg 3600
gatctcagca aacgggaata ttttgtgctt tgttaaatct cttagaaagt gcggaaacag 3660
aagaggtagt ggatattttt caagtggtaa aagctctacg caaagctagg ccaggcatgg 3720
tttccacatt cgagcaatat caattcctat atgacgtcat tgccagcacc taccctgctc 3780
agaatggaca agtaaagaaa aacaaccatc aagaagataa aattgaattt gataatgaag 3840
tggacaaagt aaagcaggat gctaattgtg ttaatccact tggtgcccca gaaaagctcc 3900
ctgaagcaaa ggaacaggct gaaggttctg aacccacgag tggcactgag ggccagaac 3960
attctgtcaa tggtcctgca agtccagctt taaatcaagg ttcataggaa aagacataaa 4020
tgaggaaact ccaaacctcc tgttagctgt tatttctatt tttgtagaag taggaagtga 4080
aaataggtat acagtggatt aattaaatgc agcgaaccaa tatttgtaga agggttatat 4140
tttactactg tggaaaaata tttaagatag ttttgccaga acagtttgta cagacgtatg 4200
cttattttaa aattttatct cttattcagt aaaaaacaac ttctttgtaa tcgttatgtg 4260
tgtatatgta tgtgtgtatg ggtgtgtgtt tgtgtgagag acagagaaag agagagaatt 4320
ctttcaagtg aatctaaaag cttttgcttt tcctttgttt ttatgaagaa aaaatacatt 4380
ttatattaga agtgttaact tagcttgaag gatctgtttt taaaaatcat aaactgtgtg 4440
cagactcaat aaaatcatgt acatttctga aatgacctca agatgtcctc cttgttctac 4500
tcatatatat ctatcttata tacttactat tttacttcta gagatagtac ataaaggtgg 4560
tatgtgtgtg tatgctacta caaaaaagtt gttaactaaa ttaacattgg gaaatcttat 4620
attccatata ttagcattta gtccaatgtc tttttaagct tatttaatta aaaaatttcc 4680
agtgagctta tcatgctgtc tttacatggg gttttcaatt ttgcatgctc gattattccc 4740
tgtacaatat ttaaaattta ttgcttgata cttttgacaa caaattaggt tttgtacaat 4800
tgaacttaaa taaatgtcat taaaataaat aaatgcaata tgtattaata ttcattgtat 4860
aaaaatagaa gaatacaaac atatttgtta aatatttaca tatgaaattt aatatagcta 4920
tttttatgga atttttcatt gatatgaaaa atatgatatt gcatatgcat agttcccatg 4980
ttaaatccca ttcataactt tcattaaagc atttactttg aatttc 5026

<210> 59
<211> 624
<212> DNA
<213> human

<400> 59
ggcgggccgc tcccacttcg gcacgagggg cacgaggtaa atcttttctg cttactgaaa 60
aggaagagtc tgatgattag ttactgatcc tctttgcatt tgtaaagctt tggagatatt 120
gaatcatgtt accatttctg tttttttcca ccctgttttc ttccatattt actgaagctc 180

agaagcagta ttgggtctgc aactcatccg atgcaagtat ttcatacacc tactgtgata 240
aaatgcaata cccaatttca attaatgtta accctgtat agaattgaaa ggatccaaag 300
gattattgca cattttctac attccaagga gagatttaaa gcaattatat ttcaatctct 360
atataactgt caacaccatg aatcttccaa agcgcaaaga agttatttgc cgaggatctg 420
atgacgatta ctctttttgc agagctctga agggagagac tgtgaataca acaatatcat 480
tctccttcaa gggaataaaa ttttctaagg gaaaatacaa atgtgttgtt gaagctattt 540
ctgggagccc agaagaaatg ctcttttgct tggagtttgt catcctacac caacctaatt 600
caaattagaa taaattgagt attt 624

<210> 60
<211> 2088
<212> DNA
<213> human

<400> 60
gaattcggca cgagcgcgcg gcgaatctca acgctgcgcc gtctgcgggc gcttccgggc 60
caccagtttc tctgctttcc accctggcgc cccccagccc tggctcccca gctgcgctgc 120
cccgggcgtc cacgccctgc gggcttagcg ggttcagtgg gctcaatctg cgcagcgcca 180
cctccatgtt gaccaagcct ctacaggggc ctcccgcgcc ccccgggacc cccacgccgc 240
cgccaggagg caaggatcgg gaagcgttcg aggccgagta tcgactcggc cccctcctgg 300
gtaagggggg ctttggcacc gtcttcgcag gacaccgcct cacagatcga ctccaggtgg 360
ccatcaaagt gattccccgg aatcgtgtgc tgggctggtc cccctttgtca gactcagtca 420
catgcccact cgaagtcgca ctgctatgga aagtgggtgc aggtggtggg caccctggcg 480
tgatccgcct gcttgactgg tttgagacac aggaaggctt catgctggtc ctcgagcggc 540
ctttgcccgc ccaggatctc tttgactata tcacagagaa gggcccactg ggtgaaggcc 600
caagccgctg cttctttggc caagtagtgg cagccatcca gcactgccat tcccgtggag 660
ttgtccatcg tgacatcaag gatgagaaca tcctgataga cctacgccgt ggctgtgcca 720
aactcattga ttttggttct ggtgccctgc ttcatgatga accctacact gactttgatg 780
ggacaagggt gtacagcccc ccagagtgga tctctcgaca ccagtaccat gcactcccgg 840
ccactgtctg gtcactgggc atcctcctct atgacatggt gtgtggggac attccctttg 900
agagggacca ggagattctg gaagctgagc tccacttccc agcccatgtc tccccagact 960
gctgtgccct aatccgccgg tgcctggccc ccaaaccttc ttcccgaccc tcactggaag 1020
agatcctgct ggacccctgg atgcaaacac cagccgagga tgttacccct caacccctcc 1080
aaaggaggcc ctgccccttt ggcctggtcc ttgctaccct aagcctggcc tggcctggcc 1140
tggcccccaa tggtcagaag agccatccca tggccatgtc acagggatag atggacattt 1200
gttgacttgg ttttacaggt cattaccagt cattaaagtc cagtattact aaggtaaggg 1260
attgaggatc aggggttaga agacataaac caagtttgcc cagttccctt cccaatccta 1320

caaaggagcc ttcctcccag aacctgtggt ccctgatttt ggagggggaa cttcttgctt 1380
ctcattttgc taaggaagtt tattttggtg aagttgttcc cattttgagc cccgggactc 1440
ttattttgat gatgtgtcac cccacattgg cacctcctac taccaccaca caaacttagt 1500
tcatatgctt ttacttgggc aagggtgctt tccttccaat accccagtag cttttatttt 1560
agtaaaggga ccctttcccc tagcctaggg tcccatattg ggtcaagctg cttacctgcc 1620
tcagcccagg atttttattt ttgggggagg taatgccctg ttgttacccc aaggcttctt 1680
tttttttttt tttttttttg ggtgagggga ccctactttg ttatcccaag tgctcttatt 1740
ctggtgagaa gaaccttaat tccataattt gggaaggaat ggaagatgga caccaccgga 1800
caccaccaga caataggatg ggatggatgg tttttttgggg gatgggctag gggaaataag 1860
gcttgctgtt tgttttcctg gggcgctccc tccaattttg cagatttttg caacctcctc 1920
ctgagccggg attgtccaat tactaaaatg taaataatca cgtattgtgg ggaggggagt 1980
tccaagtgtg ccctcctttt ttttcctgcc tggattattt aaaaagccat gtgtggaaac 2040
ccactattta ataaaagtaa tagaatcaga aaaaaaaaaa aaaaaaaa 2088

<210> 61
<211> 2270
<212> DNA
<213> human

<400> 61
ctcctccagc ctctcacact ctcctcagct ctctcatctc ctggaaccat ggccagcaca 60
tccaccacca tcaggagcca cagcagcagc cgccggggtt tcagtgccaa ctcagccagg 120
ctccctgggg tcagccgctc tggcttcagc agcgtctccg tgtcccgctc caggggcagt 180
ggtggcctgg gtggtgcatg tggaggagct ggctttggca gccgcagtct gtatggcctg 240
gggggctcca agaggatctc cattggaggg ggcagctgtg ccatcagtgg cggctatggc 300
agcagagccg gaggcagcta tggctttggt ggcgccggga gtggatttgg tttcggtggt 360
ggagccggca ttggctttgg tctgggtggt ggagccggcc ttgctggtgg ctttgggggc 420
cctggcttcc ctgtgtgccc ccctggaggc atccaagagg tcaccgtcaa ccagagtctc 480
ctgactcccc tcaacctgca aatcgatccc accatccagc gggtgcgggc tgaggagcgt 540
gaacagatca agaccctcaa caacaagttt gcctccttca tcgacaaggt gcggttcctg 600
gagcagcaga acaaggttct ggaaacaaag tggaccctgc tgcaggagca gggcaccaag 660
actgtgaggc agaacctgga gccgttgttc gagcagtaca tcaacaacct caggaggcag 720
ctggacagca ttgtcgggga acggggccgc ctggactcag agctcagagg catgcaggac 780
ctggtggagg acttcaagaa caaatatgag gatgaaatca acaagcgcac agcagcagag 840
aatgaatttg tgactctgaa gaaggatgtg gatgctgcct acatgaacaa ggttgaactg 900
caagccaagg cagacactct cacagacgag atcaacttcc tgagagcctt gtatgatgca 960
gagctgtccc agatgcagac ccacatctca gacacatctg tggtgctgtc catggacaac 1020
aaccgcaacc tggacctgga cagcatcatc gctgaggtca aggcccaata tgaggagatt 1080

gctcagagaa gccgggctga ggctgagtcc tggtaccaga ccaagtacga ggagctgcag 1140

gtcacagcag gcagacatgg ggacgacctg cgcaacacca agcaggagat tgctgagatc 1200

aaccgcatga tccagaggct gagatctgag atcgaccacg tcaagaagca gtgcgccaac 1260

ctgcaggccg ccattgctga tgctgagcag cgtggggaga tggccctcaa ggatgccaag 1320

aacaagctgg aagggctgga ggatgccctg cagaaggcca agcaggacct ggcccggctg 1380

ctgaaggagt accaggagct gatgaatgtc aagctggccc tggacgtgga gatcgccacc 1440

taccgcaagc tgctggaggg tgaggagtgc aggctgaatg gcgaaggcgt tggacaagtc 1500

aacatctctg tggtgcagtc caccgtctcc agtggctatg gcggtgccag tggtgtcggc 1560

agtggcttag gcctgggtgg aggaagcagc tactcctatg gcagtggtct tggcgttgga 1620

ggtggcttca gttccagcag tggcagagcc attgggggtg gcctcagctc tgttggaggc 1680

ggcagttcca ccatcaagta caccaccacc tcctcctcca gcaggaagag ctataagcac 1740

taaagtgcgt ctgctagctc tcggtcccac agtcctcagg cccctctctg gctgcagagc 1800

cctctcctca ggttgcctgt cctctcctgg cctccagtct cccctgctgt cccaggtaga 1860

gctggggatg aatgcttagt gccctcactt cttctctctc tctctatacc atctgagcac 1920

ccattgctca ccatcagatc aacctctgat tttacatcat gatgtaatca ccactggagc 1980

ttcactgtta ctaaattatt aatttcttgc ctccagtgtt ctatctctga ggctgagcat 2040

tataagaaaa tgacctctgc tccttttcat tgcagaaaat tgccaggggc ttatttcaga 2100

acaacttcca cttactttcc actggctctc aaactctcta acttataagt gttgtgaacc 2160

cccacccagg cagtatccat gaaagcacaa gtgactagtc ctatgatgta caaagcctgt 2220

atctctgtga tgatttctgt gctcttcact gtttgcaatt gctaaataaa 2270

<210> 62
<211> 2048
<212> DNA
<213> human

<400> 62
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660
caaggtcaaa cctggttcca cttgcgtcgt ctttggcctg ggaggagttg gcctgtcagt 720
catcatgggc tgtaagtcag ctggtgcatc taggatcatt gggattgacc tcaacaaaga 780
caaatttgag aaggccatgg ctgtaggtgc cactgagtgt atcagtccca aggactctac 840
caaacccatc agtgaggtgc tgtcagaaat gacaggcaac aacgtgggat acacctttga 900
agttattggg catcttgaaa ccatgattga tgccctggca tcctgccaca tgaactatgg 960
gaccagcgtg gttgtaggag ttcctccatc agccaagatg ctcacctatg acccgatgtt 1020
gctcttcact ggacgcacat ggaagggatg tgtctttgga ggtttgaaaa gcagagatga 1080
tgtcccaaaa ctagtgactg agttcctggc aaagaaattt gacctggacc agttgataac 1140
tcatgtttta ccatttaaaa aaatcagtga aggatttgag ctgctcaatt caggacaaag 1200
cattcgaacg gtcctgacgt tttgagatcc aaagtggcag gaggtctgtg ttgtcatggt 1260
gaactggagt ttctcttgtg agagttccct catctgaaat catgtatctg tctcacaaat 1320
acaagcataa gtagaagatt tgttgaagac atagaaccct tataaagaat tattaacctt 1380
tataaacatt taaagtcttg tgagcacctg ggaattagta taataacaat gttaatattt 1440
ttgatttaca ttttgtaagg ctataattgt atcttttaag aaaacataca cttggatttc 1500
tatgttgaaa tggagatttt taagagtttt aaccagctgc tgcagatata taactcaaaa 1560
cagatatagc gtataaagat atagtaaatg catctcccag agtaatattc acttaacaca 1620
ttgaaactat tattttttag atttgaatat aaatgtattt tttaaacact tgttatgagt 1680
taacttggat tacattttga aatcagttca ttccatgatg catattactg gattagatta 1740
agaaagacag aaaagattaa gggacgggca catttttcaa cgattaagaa tcatcattac 1800
ataacttggt gaaactgaaa aagtatatca tatgggtaca caaggctatt tgccagcata 1860
tattaatatt ttagaaaata ttccttttgt aatactgaat ataaacatag agctagagtc 1920
atattatcat acttatcata atgttcaatt tgatacagta gaattgcaag tccctaagtc 1980
cctattcact gtgcttagta gtgactccat ttaataaaaa gtgtttttag tttttaacaa 2040
ctaaaccg 2048

<210> 63
<211> 2048
<212> DNA
<213> human

<400> 63
atgtgaaggc acaagctgct gttatataca acagagtgaa ctgagcatca gtcagaaaaa 60
gtctatgttt gcagaaatac agatccaaga caaagacagg atgggcactg ctggaaaagt 120
tattaaatgc aaagcagctg tgctttggga gcagaagcaa cccttctcca ttgaggaaat 180
agaagttgcc ccaccaaaga ctaaagaagt tcgcattaag attttggcca caggaatctg 240
tcgcacagat gaccatgtga taaaaggaac aatggtgtcc aagtttccag tgattgtggg 300
acatgaggca actgggattg tagagagcat tggagaagga gtgactacag tgaaaccagg 360

tgacaaagtc atccctctct ttctgccaca atgtagagaa tgcaatgctt gtcgcaaccc 420
agatggcaac ctttgcatta ggagcgatat tactggtcgt ggagtactgg ctgatggcac 480
caccagattt acatgcaagg gcaaaccagt acaccacttc atgaacacca gtacatttac 540
cgagtacaca gtggtggatg aatcttctgt tgctaagatt gatgatgcag ctcctcctga 600
gaaagtctgt ttaattggct gtgggttttc cactggatat ggcgctgctg ttaaaactgg 660
caaggtcaaa cctggttcca cttgcgtcgt ctttggcctg ggaggagttg gcctgtcagt 720
catcatgggc tgtaagtcag ctggtgcatc taggatcatt gggattgacc tcaacaaaga 780
caaatttgag aaggccatgg ctgtaggtgc cactgagtgt atcagtccca aggactctac 840
caaacccatc agtgaggtgc tgtcagaaat gacaggcaac aacgtgggat acacctttga 900
agttattggg catcttgaaa ccatgattga tgccctggca tcctgccaca tgaactatgg 960
gaccagcgtg gttgtaggag ttcctccatc agccaagatg ctcacctatg acccgatgtt 1020
gctcttcact ggacgcacat ggaagggatg tgtctttgga ggtttgaaaa gcagagatga 1080
tgtcccaaaa ctagtgactg agttcctggc aaagaaattt gacctggacc agttgataac 1140
tcatgtttta ccatttaaaa aaatcagtga aggatttgag ctgctcaatt caggacaaag 1200
cattcgaacg gtcctgacgt tttgagatcc aaagtggcag gaggtctgtg ttgtcatggt 1260
gaactggagt ttctcttgtg agagttccct catctgaaat catgtatctg tctcacaaat 1320
acaagcataa gtagaagatt tgttgaagac atagaaccct tataaagaat tattaacctt 1380
tataaacatt taaagtcttg tgagcacctg ggaattagta taataacaat gttaatattt 1440
ttgatttaca ttttgtaagg ctataattgt atcttttaag aaaacataca cttggatttc 1500
tatgttgaaa tggagatttt taagagtttt aaccagctgc tgcagatata taactcaaaa 1560
cagatatagc gtataaagat atagtaaatg catctcccag agtaatattc acttaacaca 1620
ttgaaactat tattttttag atttgaatat aaatgtattt tttaaacact tgttatgagt 1680
taacttggat tacattttga aatcagttca ttccatgatg catattactg gattagatta 1740
agaaagacag aaaagattaa gggacgggca catttttcaa cgattaagaa tcatcattac 1800
ataacttggt gaaactgaaa aagtatatca tatgggtaca caaggctatt tgccagcata 1860
tattaatatt ttagaaaata ttccttttgt aatactgaat ataaacatag agctagagtc 1920
atattatcat acttatcata atgttcaatt tgatacagta gaattgcaag tccctaagtc 1980
cctattcact gtgcttagta gtgactccat ttaataaaaa gtgtttttag tttttaacaa 2040
ctaaaccg 2048

<210> 64
<211> 2816
<212> DNA
<213> human

<400> 64
tcgttgatat caaagacagt tgaaggaaat gaattttgaa acttcacggt gtgccaccct 60

acagtactgc cctgaccctt acatccagcg tttcgtagaa acccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100

tttgtgagaa cttgcattat ttgtgtcctc ccctcatgtg taggtagaac atttcttaat 2160

gctgtgtacc tgcctctgcc actgtatgtt ggcatctgtt atgctaaagt ttttcttgta 2220

catgaaaccc tggaagacct actacaaaaa aactgttgtt tggcccccat agcaggtgaa 2280

ctcattttgt gcttttaata gaaagacaaa tccaccccag taatattgcc cttacgtagt 2340

tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 65
<211> 2816
<212> DNA
<213> human

<400> 65
tcgttgatat caaagacagt tgaaggaaat gaattttgaa acttcacggt gtgccaccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420

agtccctata acacagacca cgcgcagaac agcgtcacgg cgccctcgcc ctacgcacag 480

cccagctcca ccttcgatgc tctctctcca tcacccgcca tcccctccaa caccgactac 540

ccaggcccgc acagtttcga cgtgtccttc cagcagtcga gcaccgccaa gtcggccacc 600

tggacgtatt ccactgaact gaagaaactc tactgccaaa ttgcaaagac atgccccatc 660

cagatcaagg tgatgacccc acctcctcag ggagctgtta tccgcgccat gcctgtctac 720

aaaaaagctg agcacgtcac ggaggtggtg aagcggtgcc ccaaccatga gctgagccgt 780

gaattcaacg agggacagat tgcccctcct agtcatttga ttcgagtaga ggggaacagc 840

catgcccagt atgtagaaga tcccatcaca ggaagacaga gtgtgctggt accttatgag 900

ccaccccagg ttggcactga attcacgaca gtcttgtaca atttcatgtg taacagcagt 960

tgtgttggag ggatgaaccg ccgtccaatt ttaatcattg ttactctgga aaccagagat 1020

gggcaagtcc tgggccgacg ctgctttgag gcccggatct gtgcttgccc aggaagagac 1080

aggaaggcgg atgaagatag catcagaaag cagcaagttt cggacagtac aaagaacggt 1140

gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt ttttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460
ctgatactgt tcagtgcatt tagccaggag acttacgttt tgagtaagtg agatccaagc 2520
agacgtgtta aaatcagcac tcctggactg gaaattaaag attgaaaggg tagactactt 2580
ttcttttttt tactcaaaag tttagagaat ctctgtttct ttccatttta aaaacatatt 2640
ttaagataat agcataaaga ctttaaaaat gttcctcccc tccatcttcc cacacccagt 2700
caccagcact gtattttctg tcaccaagac aatgatttct tgttattgag gctgttgctt 2760
ttgtggatgt gtgattttaa ttttcaataa acttttgcat cttggtttaa aagaaa 2816

<210> 66
<211> 5838
<212> DNA
<213> human

<400> 66
ccgggcaggt ggctcatgct cgggagcgtg gttgagcggc tggcgcggtt gtcctggagc 60
aggggcgcag gaattctgat gtgaaactaa cagtctgtga gccctggaac ctccgctcag 120

agaagatgaa ggatatcgac ataggaaaag agtatatcat ccccagtcct gggtatagaa 180
gtgtgaggga gagaaccagc acttctggga cgcacagaga ccgtgaagat tccaagttca 240
ggagaactcg accgttggaa tgccaagatg ccttggaaac agcagcccga gccgagggcc 300
tctctcttga tgcctccatg cattctcagc tcagaatcct ggatgaggag catcccaagg 360
gaaagtacca tcatggcttg agtgctctga agcccatccg gactacttcc aaacaccagc 420
acccagtgga caatgctggg ctttttttcct gtatgacttt ttcgtggctt tcttctctgg 480
cccgtgtggc ccacaagaag gggagctct caatggaaga cgtgtggtct ctgtccaagc 540
acgagtcttc tgacgtgaac tgcagaagac tagagagact gtggcaagaa gagctgaatg 600
aagttgggcc agacgctgct tccctgcgaa gggttgtgtg gatcttctgc cgcaccaggc 660
tcatcctgtc catcgtgtgc ctgatgatca cgcagctggc tggcttcagt ggaccagcct 720
tcatggtgaa acacctcttg gagtataccc aggcaacaga gtctaacctg cagtacagct 780
tgttgttagt gctgggcctc ctcctgacgg aaatcgtgcg gtcttggtcg cttgcactga 840
cttgggcatt gaattaccga accggtgtcc gcttgcgggg ggccatccta accatggcat 900
ttaagaagat ccttaagtta aagaacatta aagagaaatc cctgggtgag ctcatcaaca 960
tttgctccaa cgatgggcag agaatgtttg aggcagcagc cgttggcagc ctgctggctg 1020
gaggacccgt tgttgccatc ttaggcatga tttataatgt aattattctg ggaccaacag 1080
gcttcctggg atcagctgtt tttatcctct tttacccagc aatgatgttt gcatcacggc 1140
tcacagcata tttcaggaga aaatgcgtgg ccgccacgga tgaacgtgtc cagaagatga 1200
atgaagttct tacttacatt aaatttatca aaatgtatgc ctgggtcaaa gcattttctc 1260
agagtgttca aaaaatccgc gaggaggagc gtcggatatt ggaaaaagcc gggtacttcc 1320
agggtatcac tgtgggtgtg gctcccattg tggtggtgat tgccagcgtg gtgaccttct 1380
ctgttcatat gaccctgggc ttcgatctga cagcagcaca ggctttcaca gtggtgacag 1440
tcttcaattc catgactttt gctttgaaag taacaccgtt ttcagtaaag tccctctcag 1500
aagcctcagt ggctgttgac agatttaaga gtttgtttct aatggaagag gttcacatga 1560
taaagaacaa accagccagt cctcacatca agatagagat gaaaaatgcc accttggcat 1620
gggactcctc ccactccagt atccagaact cgcccaagct gacccccaaa atgaaaaaag 1680
acaagagggc ttccaggggc aagaaagaga aggtgaggca gctgcagcgc actgagcatc 1740
aggcggtgct ggcagagcag aaaggccacc tcctcctgga cagtgacgag cggcccagtc 1800
ccgaagagga agaaggcaag cacatccacc tgggccacct gcgcttacag aggacactgc 1860
acagcatcga tctggagatc caagagggta aactggttgg aatctgcggc agtgtgggaa 1920
gtggaaaaac ctctctcatt tcagccattt taggccagat gacgcttcta gagggcagca 1980
ttgcaatcag tggaaccttc gcttatgtgg cccagcaggc ctggatcctc aatgctactc 2040
tgagagacaa catcctgttt gggaaggaat atgatgaaga aagatacaac tctgtgctga 2100
acagctgctg cctgaggcct gacctggcca ttcttcccag cagcgacctg acggagattg 2160

gagagcgagg agccaacctg agcggtgggc agcgccagag gatcagcctt gcccgggcct 2220
tgtatagtga caggagcatc tacatcctgg acgacccccct cagtgcctta gatgcccatg 2280
tgggcaacca catcttcaat agtgctatcc ggaaacatct caagtccaag acagttctgt 2340
ttgttaccca ccagttacag tacctggttg actgtgatga agtgatcttc atgaaagagg 2400
gctgtattac ggaaagaggc acccatgagg aactgatgaa tttaaatggt gactatgcta 2460
ccatttttaa taacctgttg ctgggagaga caccgccagt tgagatcaat tcaaaaaagg 2520
aaaccagtgg ttcacagaag aagtcacaag acaagggtcc taaaacagga tcagtaaaga 2580
aggaaaaagc agtaaagcca gaggaagggc agcttgtgca gctggaagag aaagggcagg 2640
gttcagtgcc ctggtcagta tatggtgtct acatccaggc tgctgggggc cccttggcat 2700
tcctggttat tatggccctt ttcatgctga atgtaggcag caccgccttc agcacctggt 2760
ggttgagtta ctggatcaag caaggaagcg ggaacaccac tgtgactcga gggaacgaga 2820
cctcggtgag tgacagcatg aaggacaatc ctcatatgca gtactatgcc agcatctacg 2880
ccctctccat ggcagtcatg ctgatcctga aagccattcg aggagttgtc tttgtcaagg 2940
gcacgctgcg agcttcctcc cggctgcatg acgagctttt ccgaaggatc cttcgaagcc 3000
ctatgaagtt ttttgacacg acccccacag ggaggattct caacaggttt tccaaagaca 3060
tggatgaagt tgacgtgcgg ctgccgttcc aggccgagat gttcatccag aacgttatcc 3120
tggtgttctt ctgtgtggga atgatcgcag gagtcttccc gtggttcctt gtggcagtgg 3180
ggccccttgt catcctcttt tcagtcctgc acattgtctc cagggtcctg attcgggagc 3240
tgaagcgtct ggacaatatc acgcagtcac ctttcctctc ccacatcacg tccagcatac 3300
agggccttgc caccatccac gcctacaata aagggcagga gtttctgcac agataccagg 3360
agctgctgga tgacaaccaa gctccttttt ttttgtttac gtgtgcgatg cggtggctgg 3420
ctgtgcggct ggacctcatc agcatcgccc tcatcaccac cacggggctg atgatcgttc 3480
ttatgcacgg gcagattccc ccagcctatg cgggtctcgc catctcttat gctgtccagt 3540
taacggggct gttccagttt acggtcagac tggcatctga gacagaagct cgattcacct 3600
cggtggagag gatcaatcac tacattaaga ctctgtcctt ggaagcacct gccagaatta 3660
agaacaaggc tccctcccct gactggcccc aggagggaga ggtgaccttt gagaacgcag 3720
agatgaggta ccgagaaaac ctccctcttg tcctaaagaa agtatccttc acgatcaaac 3780
ctaaagagaa gattggcatt gtggggcgga caggatcagg gaagtcctcg ctggggatgg 3840
ccctcttccg tctggtggag ttatctggag gctgcatcaa gattgatgga gtgagaatca 3900
gtgatattgg ccttgccgac ctccgaagca aactctctat cattcctcaa gagccggtgc 3960
tgttcagtgg cactgtcaga tcaaatttgg acccctttcaa ccagtacact gaagaccaga 4020
tttgggatgc cctggagagg acacacatga aagaatgtat tgctcagcta cctctgaaac 4080
ttgaatctga agtgatggag aatggggata acttctcagt gggggaacgg cagctcttgt 4140
gcatagctag agccctgctc cgccactgta agattctgat tttagatgaa gccacagctg 4200

ccatggacac agagacagac ttattgattc aagagaccat ccgagaagca tttgcagact 4260

gtaccatgct gaccattgcc catcgcctgc acacggttct aggctccgat aggattatgg 4320

tgctggccca gggacaggtg gtggagtttg acaccccatc ggtccttctg tccaacgaca 4380

gttcccgatt ctatgccatg tttgctgctg cagagaacaa ggtcgctgtc aagggctgac 4440

tcctccctgt tgacgaagtc tcttttcttt agagcattgc cattccctgc ctggggcggg 4500

cccctcatcg cgtcctccta ccgaaacctt gcctttctcg attttatctt tcgcacagca 4560

gttccggatt ggcttgtgtg tttcactttt agggagagtc atattttgat tattgtattt 4620

attccatatt catgtaaaca aaatttagtt tttgttctta attgcactct aaaaggttca 4680

gggaaccgtt attataattg tatcagaggc ctataatgaa gctttatacg tgtagctata 4740

tctatatata attctgtaca tagcctatat ttacagtgaa aatgtaagct gtttatttta 4800

tattaaaata agcactgtgc taataacagt gcatattcct ttctatcatt tttgtacagt 4860

ttgctgtact agagatctgg ttttgctatt agactgtagg aagagtagca tttcattctt 4920

ctctagctgg tggtttcacg gtgccaggtt ttctgggtgt ccaaaggaag acgtgtggca 4980

atagtgggcc ctccgacagc cccctctgcc gcctccccac agccgctcca ggggtggctg 5040

gagacgggtg ggcggctgga gaccatgcag agcgccgtga gttctcaggg ctcctgcctt 5100

ctgtcctggt gtcacttact gtttctgtca ggagagcagc ggggcgaagc ccaggcccct 5160

tttcactccc tccatcaaga atggggatca cagagacatt cctccgagcc ggggagtttc 5220

tttcctgcct tcttcttttt gctgttgttt ctaaacaaga atcagtctat ccacagagag 5280

tcccactgcc tcaggttcct atggctggcc actgcacaga gctctccagc tccaagacct 5340

gttggttcca agccctggag ccaactgctg ctttttgagg tggcactttt tcatttgcct 5400

attcccacac ctccacagtt cagtggcagg gctcaggatt tcgtgggtct gttttccttt 5460

ctcaccgcag tcgtcgcaca gtctctctct ctctctcccc tcaaagtctg caactttaag 5520

cagctcttgc taatcagtgt ctcacactgg cgtagaagtt tttgtactgt aaagagacct 5580

acctcaggtt gctggttgct gtgtggtttg gtgtgttccc gcaaaccccc tttgtgctgt 5640

ggggctggta gctcaggtgg gcgtggtcac tgctgtcatc agttgaatgg tcagcgttgc 5700

atgtcgtgac caactagaca ttctgtcgcc ttagcatgtt tgctgaacac cttgtggaag 5760

caaaaatctg aaaatgtgaa taaaattatt ttggattttg taaaaaaaaa aaaaaaaaaa 5820

aaaaaaaaaa aaaaaaaa 5838

<210> 67
<211> 6841
<212> DNA
<213> human

<400> 67
gccggagggc gcccgagggg ccccgggccg cggcgctcag ggcccgggcg gccggcggcg 60

gccccggggc tggggggagt ccagcccgga tattgagtgc agccattgag aaaagccaaa 120

ctcttgtgtg tgcgcgtctc gatagccccc aagatggccg ccaatgtggg atcgatgttt 180

caatattgga agcgatttga tctacggcga ctccagaagg agcttaattc cgtcgcttct 240
gagctgtctg cacggcagga ggagagtgaa cattctcata aacatttaat tgaactccgc 300
cgggaattta agaaaaatgt acctgaggaa atcagagaga tggtggctcc tgtattaaaa 360
agcttccaag ccgaggtggt ggcccttagt aagagaagtc aggaggcgga ggctgctttt 420
ctgagtgttt acaagcaatt aattgaagca ccagaccccg tgcctgtgtt tgaggcggca 480
cgcagcctag acgacagact gcagcccccc agctttgacc ccagtgggca gccccggcga 540
gacctccaca cttcgtggaa gaggaacccc gagctcctca gccccaaaga gcagagagag 600
gggacgtcgc ctgccgggcc cacgctgacc gagggaagcc gcctcccagg cattcccggg 660
aaagccctcc tgacagaaac cttgctgcag agaaatgagg cggaaaaaca aaagggcctt 720
caagaagtac agatcacttt ggcggccaga ctgggggagg cagaggagaa aatcaaagtc 780
ctacattcag cgctaaaggc tacgcaggca gagctgctag agctgcggcg gaagtacgac 840
gaggaggcag catccaaggc agatgaagtc ggcctgatca tgaccaacct ggagaaagct 900
aatcagcgag ctgaggctgc ccagcgggag gtggaaagtc tccgggaaca gctggcctct 960
gtcaacagct ccatccgcct ggcttgctgc tctccccagg ggcccagtgg ggataaggtg 1020
aacttcactc tgtgctcggg ccctcggctg gaggccgcgc tggcctccaa ggacagggag 1080
atcctgcggc tgctgaagga cgtgcagcac ctccagagct cactgcagga gctggaggag 1140
gcatccgcca accagatcgc cgacctggag cggcagctca cggccaagtc cgaggccata 1200
gaaaagctgg aagagaagct ccaggcccag tctgactatg aggaaattaa aacggagctg 1260
agcatcctga aagccatgaa gctggcctcc agcacctgca gcctccccca gggcatggcc 1320
aagcctgaag actcactgct tattgcaaag gaggccttct tccccacgca gaaattcctt 1380
ctggagaagc ccagcctcct ggccagccct gaggaagacc catcagagga cgattccatc 1440
aaggattcac tgggcacgga gcagtcctac ccctcccctc agcagctccc acctccacca 1500
gggccagaag accccctgtc tcccagcccc gggcagcccc tgctgggccc cagcttgggg 1560
cctgacggca ctcggacttt ctcgctgtcc cccttcccca gcctggcatc aggggagaga 1620
ctgatgatgc ccccagccgc cttcaaggga gaggcgggcg gcctgctggt gttcccccca 1680
gccttctatg gcgccaagcc ccccacagcc cctgccaccc cggcccctgg ccctgagcca 1740
ctgggcggtc ctgagcccgc ggatggtggt gggggcggag cggcggggcc cggggcagag 1800
gaggagcagc tggacacggc agagatcgcc ttccaggtga aggagcagct gctgaaacac 1860
aacatcgggc agcgggtgtt tgggcattac gtgctggggc tgtcgcaggg ctcggtcagc 1920
gagatcctag cccggcccaa gccctggcgc aagctcacgg tgaagggcaa ggagcccttc 1980
atcaagatga agcagttcct gtcggatgag cagaatgtac tggcgctcag gaccatccaa 2040
gtgcggcagc gaggcagcat caccccgaga atccgcacgc ctgagacagg ctcagacgac 2100
gccatcaaga gcattctaga gcaggccaag aaggagatcg agtcgcagaa gggcggcgag 2160
cccaagacct cggtggcccc gctgagcatc gccaacggca cgacccccgc cagcacctcg 2220

gaggacgcca tcaagagcat cctggagcag gcacgccgtg agatgcaggc gcaacagcag 2280
gcgctgctgg agatggaggt ggcgcccagg ggccgctcgg tgccccccctc gcccccggag 2340
cggccatcac tggccaccgc gagccagaac ggggccccgg ccttggtgaa gcaggaggag 2400
ggcagcgggg gccccgcgca ggcgccgctc ccggtcctgt cccccgccgc cttcgtgcag 2460
agcatcatcc gcaaggtcaa gtccgagatc ggcgacgccg gctacttcga ccaccactgg 2520
gcctccgacc gcggcctgct cagccgcccc tacgcctccg tgtcgccctc gctgtcctcc 2580
tcctcctcct ctggctactc tggccagccc aacggccgcg cctggccccg cggggacgag 2640
gcccctgtgc cccccgagga cgaggcggcg gcaggggcgg aggacgaacc ccccaggacg 2700
ggcgagctca aggctgaggg cgcgacggcc gaggcgggcg cgcggctgcc ctactacccg 2760
gcctacgtgc cgcgcaccct gaagcccacc gtgccgccgc tgacccccga gcagtacgag 2820
ctgtacatgt accgtgaggt agacacgctg gagctcaccc gccaggtcaa ggagaagctg 2880
gccaagaacg gcatctgcca gaggatcttc ggggagaagg tgctgggcct gtcacagggc 2940
agcgtgagcg acatgctgtc ccggccgaag ccatggagca agctgacgca gaaggggcgg 3000
gagcccttca tccgcatgca gctgtggctc tctgaccagc tcggccaggc agtgggccag 3060
cagcctggtg cctcccaggc cagtcccaca gaaccaaggt cctcaccatc cccacccccc 3120
agccccacag agcctgagaa gagctcccag gagccgttga gcctgtccct ggagagcagc 3180
aaggagaacc agcagccaga gggccgctcc agctcctcgt tgagcgggaa gatgtactca 3240
ggcagccagg ccccaggggg catccaggag atcgtggcca tgtcccccga gctggacacg 3300
tactccatca ccaagagggt gaaggaggtc ctcacagaca acaatctagg gcagcggctg 3360
tttggggaaa gcatcctggg tctgacacag ggctccgtgt ctgacctgct gtcccggccc 3420
aaaccctggc acaagctgag cctgaagggg cgggagcctt ttgtccgcat gcagctgtgg 3480
ctcaatgacc cccataacgt ggagaagctg agggatatga agaagctgga gaagaaagcc 3540
tacctgaaac gtcgctatgg cctcatcagc accggctcag acagtgagtc ccggccacc 3600
cgctcagagt gccccagccc ctgcctgcag ccccaggacc tgagcctcct gcagatcaag 3660
aagccccggg tggtgctggc acccgaggag aaggaggcac tgcggaaggc ctatcagctg 3720
gaaccctacc cctcgcagca gaccatcgag ctcctctcct tccagctcaa cctcaagacc 3780
aacaccgtca tcaactggtt ccacaactac aggtcccgga tgcgccggga gatgttggtg 3840
gaggggaccc aggatgagcc agaccttgat ccaagcgggg gtcctggaat cctaccgcca 3900
ggccactccc acccagaccc caccccgcag agccctgact ctgagactga ggaccagaag 3960
ccaaccgtga aggaactgga gcttcaggag ggccctgagg agaacagcac acccctgacc 4020
acccaggaca aggcccaagt gaggatcaag caggaacaga tggaggagga tgctgaggaa 4080
gaggcaggca gccagcccca ggactcaggg gagctggaca aaggccaagg tccccccaaa 4140
gaggagcatc ccgaccctcc gggtaatgat ggactcccaa aagtggctcc cgggcccctc 4200
cttccaggtg gatccacccc agactgtccc tcacttcatc cccaacagga gagtgaggcc 4260

ggggagcgac ttcacccgga ccctttaagt tttaagtcag cctcagagtc ctcacgctgc 4320
agcctggagg tgtcactgaa ctcgccctcg gccgcctcct caccaggcct catgatgtct 4380
gtgtcacctg tcccctcctc ctcagctccc atctccccat ccccacctgg cgccccccct 4440
gccaaagtgc cgagtgccag ccccactgct gacatggctg gagccttgca ccccagtgcc 4500
aaggtgaacc ccaacttgca gcggcggcat gagaagatgg ccaatctgaa caacatcatt 4560
taccgactag agcgggctgc caatcgggag gaggccctgg agtgggagtt ctgaaggcag 4620
ggtgaggggg caagggacat accctggtaa ctaccttcct tctcgcactt actctcctca 4680
acaggatggg gtaagggagg gaggaactca accatcaaaa tgtggacagc aatgttatgc 4740
cgtttacgtt ttttgttgta atcctagttc tatgaagctg tgtgagcagg tgggtcaaat 4800
gccattgcct ccacttttct gcaccccct gctcctcttc accctgaccc ctctgcagga 4860
ggcagaagca aaatggcacc acatattcac ctgaaaactc caaactcttt tagaaaaata 4920
aataaatatt tatagacctc ttttagatat tttaataaag gatcctttgg aatttatccc 4980
agctgatgct gttttgatat tacagagagt tataaaatca ggatgctgtc acaactgttg 5040
cgaagtatac actgaagttg tgtcgttttt gccactagat gagattaaaa gaagacaatt 5100
attcaaagcc atcacaaaac actataagac tgaccaaaat ttagataacc tttgaaccac 5160
gatttttttc cacatctgtc tgtgagacac agcgcaatgc tactgccctt ccagaaactg 5220
tgctaaaaag agaaagtcca aaagactcta aacaaaaacc tcgacgccgt tgaggatgtg 5280
tttcattctg gtggtctgtt ttgcaagctt gataacagaa tgtccgtgcc attgtaaatg 5340
ttgtagagat gtgggccgtg gcccaaccgt cctatatgag atgtagcatg gtacagaaca 5400
aactgcttac acaggtctca ctagttagaa acctgtgggc catggaggtc agacatccat 5460
cttgtccatc tataggcaag aagtgtttcc agatcctttg gaaaggtggg catggggcag 5520
gtgcttggag agtggcgttt gagccagagc gaccccattt cccgtgtgaa ccataggcac 5580
aacccaggaa gtttccccac ttgtaggagt gtgggtattc cagagcaaga ctgtggccac 5640
catcttcccc tcttggtgtt ttccgaaagt gacagtgttg gtcatcccat gaccactgaa 5700
gcttagtaac cagcgccaaa aagtagattc atcaaactag agaccccagc tccccttctc 5760
gccatcttct ttctcaagtt gaccgtggtg ctgtttctgg aaggcatctg caactccaag 5820
tccatgcaga actctggaag gccaagttca tcgcagcatg ttcaccatat cccagcctcc 5880
aaatctatcc tcctaccttc caacgcatga cctgttgggg agcagagact taaccccaa 5940
ctcagaggaa cccttcctcc agcgtctttg gcatggtttc tagggtgaga gttcccaatt 6000
tggatagaac ggccaccata ttggttactg aatctctctc ccttgttttt attacgtttc 6060
ctttttcaaa ctgtccatgg gaaggctgaa ttgagtgact ccccagaatg aagatgagaa 6120
ggtgaatata atcaatgcca atgtaatgcc agcgggtgag atggccgatg gaggtttcaa 6180
agatgtagct agcattttga aaccatatgg gcaaaacccg gcaaccagaa ggggacagat 6240
aaggaccgtt ccagaaatcc caactctcac acccagccca ggctgcagtc tccacaccaa 6300

acagtcaaca aaacacaaac cctgaaggaa aacctttcc atacacccag gctatgcatt 6360
gaagagtttt ccactgtata catttttatc cagatgaagg tatttttata ttttgacaat 6420
aggaaacagt gaccattttc agagtaatca aatctggaac aaatgaaaca tcttttagcc 6480
accaccaccc tgttgcaatt aagacaaccg tgggggaaca caccactttt tactgttgaa 6540
accaacacaa cgttgaaatc caggcttata cgcagactcc gattcctaga gaactaaatt 6600
tggctttagt gtgacgggat ttgattaagc acttagtata gtcttttgaa cacggaaatc 6660
ctgttgtact taaagctagc ggacccgtga acaactttgt caggttcacg tcctataacg 6720
gttaaaaaac acacacacac atacacaaac cgtttctatg agagattgat gaactttgtt 6780
taaaatttta aaaaaaggaa cacgttctgt aaacgagtcg ctaaatacag aattgtataa 6840
t 6841

<210> 68
<211> 1181
<212> DNA
<213> human

<400> 68
ggcacgaggc ccgggccccc caaagtcccg gccgggccga gggtcggcgg ccgccggcgg 60
gccgggcccg cgcacagcgc ccgcatgtac aacatgatgg agacggagct gaagccgccg 120
ggcccgcagc aaacttcggg gggcggcggc ggcaactcca ccgcggcggc ggccggcggc 180
aaccagaaaa acagcccgga ccgcgtcaag cggcccatga atgccttcat ggtgtggtcc 240
cgcgggcagc ggcgcaagat ggcccaggag aaccccaaga tgcacaactc ggagatcagc 300
aagcgcctgg gcgccgagtg gaaacttttg tcggagacgg agaagcggcc gttcatcgac 360
gaggctaagc ggctgcgagc gctgcacatg aaggagcacc cggattataa ataccggccc 420
cggcggaaaa ccaagacgct catgaagaag gataagtaca cgctgcccgg cgggctgctg 480
gcccccggcg gcaatagcat ggcgagcggg gtcggggtgg gcgccggcct gggcgcgggc 540
gtgaaccagc gcatggacag ttacgcgcac atgaacggct ggagcaacgg cagctacagc 600
atgatgcagg accagctggg ctacccgcag cacccgggcc tcaatgcgca cggcgcagcg 660
cagatgcagc ccatgcaccg ctacgacgtg agcgccctgc agtacaactc catgaccagc 720
tcgcagacct acatgaacgg ctcgcccacc tacagcatgt cctactcgca gcagggcacc 780
cctggcatgg ctcttggctc catgggttcg gtggtcaagt ccgaggccag ctccagcccc 840
cctgtggtta cctcttcctc ccactccagg gcgccctgcc aggccgggga cctccgggac 900
atgatcagca tgtatctccc cggcgccgag gtgccggaac ccgccgcccc cagcagactt 960
cacatgtccc agcactacca gagcggcccg gtgcccggca cggccattaa cggcacactg 1020
cccctctcac acatgtgagg gccggacagc gaactggagg ggggagaaat tttcaaagaa 1080
aaacgaggga aatgggaggg gtgcaaaaga ggagagtaag aaacagcatg gagaaaaccc 1140
ggtacgctca aaaaaaaaaa aaaaaaaaaa aaaaaaaaaa a 1181

<210> 69
<211> 4729
<212> DNA
<213> human

<400> 69
gctgctctcg ttcgcttggc tcagctcagc tcagctcagc gcagctccgc ggccgccaag 60

ccgaggcggg cacggtctcc gagtcgcgga cgccagctcc gagctccctc tctccgccgc 120

gcctccgcca ggtcgcgcct tcgtcgggac cacttcgggc aggagtcgcg tggcgaaggc 180

ctgcggccgc ggcacaaagt tgggggccgc gaagatgagg ctgtccccgg cgcccctgaa 240

gctgagccgg actccggcac tgctggccct ggcgctgccc ctggccgcgg cgctggcctt 300

ctccgacgag accctggaca aagtgcccaa gtcagagggc tactgcagcc gtatcctgcg 360

cgcccagggc acgcggcgcg agggctacac cgagttcagc ctccgcgtgg agggcgaccc 420

cgacttctac aagccgggaa ccagctaccg cgtaacactt tcagctgctc ctccctccta 480

cttcagagga ttcacattaa ttgccctcag agagaacaga gagggtgata aggaagaaga 540

ccatgctggg accttccaga tcatagacga agaagaaact cagtttatga gcaattgccc 600

tgttgcagtc actgaaagca ctccacggag gaggacccgg atccaggtgt tttggatagc 660

accaccagcg ggaacaggct gcgtgattct gaaggccagc atcgtacaaa aacgcattat 720

ttattttcaa gatgagggct ctctgaccaa gaaactttgt gaacaagatt ccacatttga 780

tggggtgact gacaaaccca tcttagactg ctgtgcctgc ggaactgcca agtacagact 840

cacattttat gggaattggt ccgagaagac acacccaaag gattaccctc gtcgggccaa 900

ccactggtct gcgatcatcg gaggatccca ctccaagaat tatgtactgt gggaatatgg 960

aggatatgcc agcgaaggcg tcaaacaagt tgcagaattg ggctcacccg tgaaaatgga 1020

ggaagaaatt cgacaacaga gtgatgaggt cctcaccgtc atcaaagcca aagcccagtg 1080

gccagcctgg cagcctctca acgtgagagc agcaccttca gctgaatttt ccgtggacag 1140

aacgcgccat ttaatgtcct tcctgaccat gatgggccct agtcccgact ggaacgtagg 1200

cttatctgca gaagatctgt gcaccaagga atgtggctgg gtccagaagg tggtgcaaga 1260

cctgattccc tgggacgctg gcaccgacag cggggtgacc tatgagtcac ccaacaaacc 1320

caccattccc caggagaaaa tccggcccct gaccagcctg gaccatcctc agagtccttt 1380

ctatgaccca gagggtgggt ccatcactca agtagccaga gttgtcatcg agagaatcgc 1440

acggaagggt gaacaatgca atattgtacc tgacaatgtc gatgatattg tagctgacct 1500

ggctccagaa gagaaagatg aagatgacac ccctgaaacc tgcatctact ccaactggtc 1560

cccatggtcc gcctgcagct cctccacctg tgacaaaggc aagaggatgc gacagcgcat 1620

gctgaaagca cagctggacc tcagcgtccc ctgccctgac acccaggact tccagccctg 1680

catgggccct ggctgcagtg acgaagacgg ccccacctgc accatgtccg agtggatcac 1740

ctggtcgccc tgcagcatct cctgcggcat gggcatgagg tcccgggaga ggtatgtgaa 1800

gcagttcccg gaggacggct ccgtgtgcac gctgcccact gaggaaacgg agaagtgcac 1860

ggtcaacgag gagtgctctc ccagcagctg cctgatgacc gagtggggcg agtgggacga 1920
gtacagcgcc acctgcggca tgggcatgaa gaagcggcac cgcatgatca agatgaaccc 1980
cgcagatggc tccatgtgca aagccgagac atcacaggca gagaagcgca tgatgccaga 2040
gtgccacacc atcccatgct tgctgtcccc atggtccgag tggagtgact gcagcgtgac 2100
ctgcgggaag ggcatgcgaa cccgacagcg gatgctcaag tctctggcag aacttggaga 2160
ctgcaatgag gatctggagc aggtggagaa gtgcatgctc cctgaatgcc ccattgactg 2220
tgagctcacc gagtggtccc agtggtcgga atgtaacaag tcatgtggga aaggccacgt 2280
gattcgaacc cggatgatcc aaatggagcc tcagtttgga ggtgcaccct gcccagagac 2340
tgtgcagcga aaaaagtgcc gcatccgaaa atgccttcga aatccatcca tccaaaagct 2400
acgctggagg gaggcccgag agagccggcg gagtgagcag ctgaaggaag agtctgaagg 2460
ggagcagttc ccaggttgta ggatgcgccc atggacggcc tggtcagaat gcaccaaact 2520
gtgcggaggt ggaattcagg aacgttacat gactgtaaag aagagattca aaagctccca 2580
gtttaccagc tgcaaagaca agaaggagat cagagcatgc aatgttcatc cttgttagca 2640
agggtacgag ttccccaggg ctgcactcta gattccagag tcaccaatgg ctggattatt 2700
tgcttgttta agacaattta aattgtgtac gctagttttc atttttgcag tgtggttcgc 2760
ccagtagtct tgtggatgcc agagacatcc tttctgaata cttcttgatg ggtacaggct 2820
gagtggggcg ccctcacctc cagccagcct cttcctgcag aggagtagtg tcagccacct 2880
tgtactaagc tgaaacatgt ccctctggag cttccacctg gccagggagg acggagactt 2940
tgacctactc cacatggaga ggcaaccatg tctggaagtg actatgcctg agtcccaggg 3000
tgcggcaggt aggaaacatt cacagatgaa gacagcagat tccccacatt ctcatctttg 3060
gcctgttcaa tgaaaccatt gtttgcccat ctcttcttag tggaacttta ggtctctttt 3120
caagtctcct cagtcatcaa tagttcctgg ggaaaaacag agctggtaga cttgaagagg 3180
agcattgatg ttgggtggct tttgttcttt cactgagaaa ttcggaatac atttgtctca 3240
cccctgatat tggttcctga tgccccccca acaaaaataa ataaataaat tatggctgct 3300
ttatttaaat ataaggtagc tagtttttac acctgagata aataataagc ttagagtgta 3360
tttttccctt gcttttgggg gttcagagga gtatgtacaa ttcttctggg aagccagcct 3420
tctgaacttt ttggtactaa atccttattg gaaccaagac aaaggaagca aaattggtct 3480
ctttagagac caatttgcct aaattttaaa atcttcctac acacatctag acgttcaagt 3540
ttgcaaatca gtttttagca agaaaacatt tttgctatac aaacattttg ctaagtctgc 3600
ccaaagcccc cccaatgcat tccttcaaca aaatacaatc tctgtacttt aaagttattt 3660
tagtcatgaa attttatatg cagagagaaa aagttaccga gacagaaaac aaatctaagg 3720
gaaaggaata ttatgggatt aagctgagca agcaattctg gtggaaagtc aaacctgtca 3780
gtgctccaca ccagggctgt ggtcctccca gacatgcata ggaatggcca caggtttaca 3840
ctgccttccc agcaattata agcacaccag attcagggag actgaccacc aagggatagt 3900

gtaaaaggac attttctcag ttgggtccat cagcagtttt tcttcctgca tttattgttg 3960
aaaactattg tttcatttct tcttttatag gccttattac tgcttaatcc aaatgtgtac 4020
cattggtgag acacatacaa tgctctgaat acactacgaa tttgtattaa acacatcaga 4080
atatttccaa atacaacata gtatagtcct gaatatgtac ttttaacaca agagagacta 4140
ttcaataaaa actcactggg tctttcatgt ctttaagcta agtaagtgtt cagaaggttc 4200
ttttttatat tgtcctccac ctccatcatt ttcaataaaa gatagggctt ttgctcccctt 4260
gttcttggag ggaccattat tacatctctg aactaccttt gtatccaaca tgttttaaat 4320
ccttaaatga attgctttct cccaaaaaaa gcacagtata aagaaacaca agatttaatt 4380
atttttctac ttggggggaa aaaagtcctc atgtagaagc acccactttt gcaatgttgt 4440
tctaagctat ctatctaact ctcagcccat gataaagttc cttaagctgg tgattcctaa 4500
tcaaggacaa gccaccctag tgtctcatgt ttgtatttgg tcccagttgg gtacatttta 4560
aaatcctgat tttggagact taaaaccagg ttaatggcta agaatgggta acatgactct 4620
tgttggattg ttattttttg tttgcaatgg ggaatttata agaagcatca agtctctttc 4680
ttaccaaagt cttgttaggt ggtttatagt tcttttggct aacaaatca 4729

<210> 70
<211> 443
<212> DNA
<213> human

<400> 70
tttttttttt tttttccatt aacccaacac aagtttattt actaagtcaa gtcaaactga 60
ggagtatttg tttctttggt agttggtaga caaagaatac atatacatat tctatttccc 120
attaagcatt cgatcatgtt acaaaacaat ggcctagaga actatcattg aagatttacc 180
aaatctgcct gaagcaaaat gtgataaact gcagaaatgg tggagaatga cactggaagt 240
cataaagttg attctcaaac acgggtttga actgcatagg accgcttata tgcatatttt 300
gataaatata ttgaaaattt ttttggaaat ttgcaacaat ttaaaaaact tgcagatgca 360
ccatgtagct tagaaatact gaaaaactaa gaaaaaggtg tgtcatgaat tcatataata 420
tatgtagaca ctagtctatt tta 443

<210> 71
<211> 2432
<212> DNA
<213> human

<400> 71
ggcgagtggc gagtggcgag tgtcaggggg gcggccggcg ggggcggggc ggccggagga 60
ggcgttggca gcgggctcgg acccacgcgg cgccgcggcc cgcctggcct gcagcgctcc 120
cacccccggc ggcggcacga tgccctttga cttcaggagg tttgacatct acaggaaggt 180
gcccaaggac cttacgcagc caacgtacac cggggccatt atctccatct gctgctgcct 240
cttcatcctc ttcctcttcc tctcggagct caccggattt ataacgacag aagttgtgaa 300

```
cgagctctat gtcgatgacc cagacaagga cagcggtggc aagatcgacg tcagtctgaa    360
catcagttta cccaatctgc actgcgagtt ggttgggctt gacattcagg atgagatggg    420
caggcacgaa gtgggccaca tcgacaactc catgaagatc ccgctgaaca atggggcagg    480
ctgccgcttc gaggggcagt tcagcatcaa caaggtcccc ggcaacttcc acgtgtccac    540
acacagtgcc acagcccagc cacagaaccc agacatgacg catgtcatcc acaagctctc    600
ctttggggac acgctacagg tccagaacat ccacggagct ttcaatgctc tcgggggagc    660
agacagactc acctccaacc ccctggcctc ccacgactac atcctgaaga ttgtgcccac    720
ggtttatgag gacaagagtg gcaagcagcg gtactcctac cagtacacgg tggccaacaa    780
ggaatacgtc gcctacagcc acacgggccg catcatccct gcaatctggt tccgctacga    840
cctcagcccc atcacggtca agtacacaga gagacggcag ccgctgtaca gattcatcac    900
cacgatctgt gccatcattg gcgggacctt caccgtcgcc ggcatcctgg actcatgcat    960
cttcacagcc tctgaggcct ggaagaagat ccagctgggc aagatgcatt gacgccacac   1020
ccagcctaat ggccgaggac cctgggcatc gccagccttg cctccagtgc cctgtctcct   1080
ttggccctca atctggtccc aaatctggct gtgtcccaaa gggtgtgtgg gaagtggggg   1140
gaaagtagag gatggctcga tgttttgcag ctacctcttt tccccgtgtt tctttttaga   1200
caaattacac tgcctgaagt tgcagttccc ctttccctgg ggagccccaa gaacagagtc   1260
aggcaagggg tggggagtcc agggatcttg gggacccctc ctaggagagc tgcagtctct   1320
tccctcaggg gaacatccca gaatgcatat cgatcagctc tcagccaggc ttcgacaatc   1380
tcgcagcccc cactaggtgg acacattaat gatttggttt ctcccctggg cagccaacct   1440
gccccagagg caccagacct gggctttcag ctttgggacc aggctgccca aaggtactcc   1500
tttatacacc cggcaccttc cacgaaagat ggtacttccc aagcaagccc ctatgatttg   1560
tcactataga tggaaccctg acttctgccc catcccttcc tgcccaacct agaacccagg   1620
cctcaagtct ttaccccacc cctttcttgt tcttccaaga agcagatgcc cagttgctca   1680
gcagcagcgg tagagacttg aatctgccca ccagtcacaa ggcgggtcac agattcctct   1740
tcctctcttc tcctcgttcc tctgaaccct ccaccaatgt gcctcagcct gtgtgctgtg   1800
tggcaacagc attctggttc ccactgccaa gatctcccac cactctgctg ggatctgcag   1860
tggcagggag tgggggttgt gtaaagggga agtcatcttt tgagatccag atagacatgg   1920
tttgtgcact tacgtccaga tgggaagcat ccttcctgca accctaaaat aatcatgcag   1980
cctctcagac ggacgccatc ggtcccaagg ccttaggtgg aggaagcaaa gcaggccagg   2040
cctgtcctgt ccgtggacct ctaccttctg gactccctac gggtgcagag cacttgggtt   2100
tctctacagc catcgtggcc cacttgacac tgtgctcctc catcagctgg tcacatgcca   2160
acacgttccc agcccctgag gcagctccag ggtgccccac ctgctcctga ggtgggtccc   2220
taccctgctg ctcctcttca tcctttccct tttgtcctga aagggaggag caatggtcca   2280
ggcattaatt ccacccaggg aattttagct atgccctcat gtcccaggga gagagccaca   2340
```

cgcctgtttt ccatttatag caagattgtt tgcatacttt tgtaatgaag gggagtgtcc 2400

agtggaagga tttttaaaat tatcttatgg at 2432

<210> 72
<211> 2782
<212> DNA
<213> human

<400> 72
gcggccgcgg cggggcggcg cgggaaccgg gccccggggg gagtcggccg ggctgctgct 60

gctgctgctc caggtgctgc cgcccggggc tacggcaggg ccgggacgcc ggggcacgcg 120

gggcgctggc ggcggcggcg tctgctggcc cggcgcggcc ccagcctttc cccgggacgc 180

gcggctgctg ctgctcctgc cgccgctgcc gccactgtcg ccgccgccgc cgagctccgc 240

gcccgcagcc tccgcctccc ggatggacgc tctgccccgc agcgggctga acctgaagga 300

ggagccgctg ctgcccgccg gcctgggctc agtgcgctcc tggatgcagg gcgcgggcat 360

cctggacgcc agcaccgcgg cgcagagtgg cgtgggtctg gcacgagcac attttgagaa 420

gcagcctccc tccaacctca ggaaatccaa cttcttccac ttcgtgctgg ccatgtacga 480

ccggcagggg cagcccgtgg aggtggagcg cacagccttc atcgacttcg tggaaaagga 540

ccgagagccc ggggcggaaa agactaacaa tgggatccat taccgcctcc ggctggtgta 600

taacaatgga ctgcggacag agcaagacct ctacgtgcgt ctcatcgact ccatgtccaa 660

acaggccatc atctatgagg ggcaggacaa gaaccccgaa atgtgccgag tgctgctcac 720

ccatgagatc atgtgcagcc ggtgctgtga ccggaagagc tgtggcaacc ggaatgagac 780

gccctcagac cccgtcatca ttgacaggtt cttcctcaag ttcttcctca aatgcaacca 840

gaactgcctg aagaatgcgg ggaatcccag agacatgcgc cgcttccagg tggtggtgtc 900

cacgacggtg agcgtggacg gacacgtgct ggccgtgtcc gacaacatgt ttgtgcacaa 960

caactccaag catggccgca gggcgcgccg cctggacccc tccgaagctg ccacccccctg 1020

catcaaggcc atcagccccg gggagggctg gaccacgggc ggcgccaccg tcattgtcat 1080

cggcgacaac ttcttcgacg ggttgcaggt cgtgttcgga aacgtgctcg tgtggagcga 1140

gctcatcacg ccccacgcca tccgggtgca gacgccccccg cggcacatcc ccggggtggt 1200

ggaggtgacc ctctcctaca agtccaagca gttttgcaag ggatgccccg gccgctttgt 1260

ctacacagct ctgaacgagc ccaccattga ctacggattc cagaggctac agaaagtcat 1320

tcccagacac ccccggagacc ccgagaggct gcccaaggaa gtgctgctga agcgggcggc 1380

cgacttggca gaagccctgt acggagtgcc cggcagtaac caggagctgc tcctgaagcg 1440

cgcggcggac gtggccgagg ctctgtacag cacccccccgc gcacccgggc cgctcgcacc 1500

cctggccccg agccacccac actccgccgt cgtgggcatc aacgccttca gcagcccgct 1560

ggccatcgcc gtcggggacg ccaccccggg gcccgagccg ggctacgcgc gcagctgcag 1620

cagcgcgtcc ccccgcgggt tcgcgcccag ccccggctcg cagcagagcg gctacggcgg 1680

cggcctcgga gctggcctgg gcggctacgg cgcgccgggc gtggccggcc tcggcgtgcc 1740

tgggtccccc agcttcctca atggctccac cgccacctcg cccttcgcca aggagcgcct 1800

tcgcccccgt gctgcgcccc ccaagctccc caccccaggc ctgccccaga gcccacggag 1860

aggggcttcc agaccagtct tttgaggatt ctgacaagtt ccactctcca gcccgggggc 1920

ttcagggcct ggcatactcc taattacggt ctgcagctgt tcccatggag cccggactgg 1980

aggtccctct gggattcaca gccacacccc ggatggtggc acagacagat gcagggccag 2040

ggccatgggc ggacctcaac ccgtgagctg aacggggaga ggccttcacc ccatgctcaa 2100

gcctccccgc tagcagcccc acaggcttct ctcgcctccc tgtcttgggg tagtcagaag 2160

ccccagcact gtgcagatgc tcttggcagg acagcatcgc agggaggtgc tgggattctg 2220

ggcctcactg tctgggtctt ggttcctctg aaagagatgg atcttgtgca gaccagggtt 2280

gttgagtgag gggagcgtgg gatggggacc gtgggaaaga ggacagctca gggagaagtg 2340

acctggaaag gtcctgtttg catctgaccc atctcaactg gcccagcatc ccaacttctc 2400

tgcagcgaaa gggtggcgcc ccgcagcctc gggaggcctg cccaggctcc cgtggagctt 2460

ccaacagctg cttggccccg cagctgcccc cacttccttt gagacctgca ctctcatgct 2520

tgccgcatca tgcctccctg tgggggcttt gggcatggag gaggcagaag agggggtgcc 2580

aggcctcctg tatttggggt cttcccccag tggatgtctc atggactctg gccccacaca 2640

ctcacaatga ctctggctgg ccccacgcag cgggcccagc cgccccccag gtggcctcac 2700

attctgctct gctaagtttg gagaaaacag aacaataaac cagatgcagg tggtgcccgc 2760

ccggcctctc acctgcctcc tt 2782

<210> 73
<211> 1722
<212> DNA
<213> human

<400> 73
ggggaaaaga gctaggaaag agctgcaaag cagtgtgggc tttttccctt tttttgctcc 60

ttttcattac ccctcctccg ttttcaccct tctccggact tcgcgtagaa cctgcgaatt 120

tcgaagagga ggtggcaaag tgggagaaaa gaggtgttag ggtttggggt ttttttgttt 180

ttgtttttgt tttttaattt cttgatttca acattttctc ccaccctctc ggctgcagcc 240

aacgcctctt acctgttctg cggcgccgcg caccgctggc agctgagggt tagaaagcgg 300

ggtgtatttt agattttaag caaaaatttt aaagataaat ccatttttct ctcccacccc 360

caacgccatc tccactgcat ccgatctcat tatttcggtg gttgcttggg ggtgaacaat 420

tttgtggctt tttttcccct ataattctga cccgctcagg cttgagggtt tctccggcct 480

ccgctcactg cgtgcacctg gcgctgccct gcttcccccca acctgttgca aggctttaat 540

tcttgcaact gggacctgct cgcaggcacc ccagccctcc acctctctct acatttttgc 600

aagtgtctgg gggagggcac ctgctctacc tgccagaaat tttaaaacaa aaacaaaaac 660

aaaaaaatct ccgggggccc tcttggcccc tttatccctg cactctcgct ctcctgcccc 720

accccgaggt aaagggggcg actaagagaa gatggtgttg ctcaccgcgg tcctcctgct 780
gctggccgcc tatgcggggc cggcccagag cctgggctcc ttcgtgcact gcgagccctg 840
cgacgagaaa gccctctcca tgtgcccccc cagccccctg ggctgcgagc tggtcaagga 900
gccgggctgc ggctgctgca tgacctgcgc cctggccgag ggcagtcgt gcggcgtcta 960
caccgagcgc tgcgcccagg ggctgcgctg cctcccccgg caggacgagg agaagccgct 1020
gcacgccctg ctgcacggcc gcggggtttg cctcaacgaa aagagctacc gcgagcaagt 1080
caagatcgag agagactccc gtgagcacga ggagcccacc acctctgaga tggccgagga 1140
gacctactcc cccaagatct tccggcccaa acacacccgc atctccgagc tgaaggctga 1200
agcagtgaag aaggaccgca gaaagaagct gacccagtcc aagtttgtcg ggggagccga 1260
gaacactgcc cacccccgga tcatctctgc acctgagatg agacaggagt ctgagcaggg 1320
cccctgccgc agacacatgg aggcttccct gcaggagctc aaagccagcc cacgcatggt 1380
gccccgtgct gtgtacctgc ccaattgtga ccgcaaagga ttctacaaga gaaagcagtg 1440
caaaccttcc cgtggccgca agcgtggcat ctgctggtgc gtggacaagt acgggatgaa 1500
gctgccaggc atggagtacg ttgacgggga ctttcagtgc cacaccttcg acagcagcaa 1560
cgttgagtga tgcgtccccc cccaaccttt ccctcacccc ctcccacccc cagccccgac 1620
tccagccagc gcctccctcc accccaggac gccactcatt tcatctcatt taagggaaaa 1680
atatatatct atctatttga ggaaaaaaaa aaaaaaaaaa aa 1722

<210> 74
<211> 2626
<212> DNA
<213> human

<400> 74
gggtacggct gcgagaagac gacagaaggg gatgtcacct gctttatttc tggctttggc 60
ctgtggtctg tgatacccat cctgcttgat gttctgcaga atggcacttg actgctgggc 120
atgcatgaag ttaagggcaa gaaacagtat gccatgtgtt ctgtaccatc atgtgtctct 180
tcttgcttct gggcccttct actggtgaac tttcatcaag atctgcgcca tgccgtgtca 240
ctatcaagcc attaagtttt gtctgggttg ctgtcagccc cagttggctt cctggtcaac 300
aaggacctca agaactgcct gtggaccgag gccccctacc agtgcatgag acacacacct 360
accctcccca gctttccagg aaccctactg gctgccagac tgatgggcgg gctggtatgt 420
gtggacatgt gttcactgtc attatgctgt ggctccaggt gagggtgagg actgggccta 480
tatagaatcc agataccatt gtcaacttcc cttattcccg tctaagatgt gagcagagtg 540
ccatagtagg ggttctggga agaggtattt ctgatttgtg ggcctctgct tgcttgactt 600
caggtcactt atacttctta ttttgcttgc ctgccttcat ccctcatttc ctccctctca 660
ttcttctttc ctccctccct ttcctggtag cctcctttcc tccccttctg ccttcccctt 720
ccttctttcc ttattctttt ttattttgtt taaatagtac cacagagaaa acaactgaaa 780

aaccacattt ttctacatac agctggggag gtagctgaga acttggcact gcgcacacat 840
actaggttga aagagagttg aggaaaccag aaggccaagt ggatctgctg gcaaaccctg 900
aacctgtctc ctgcgcttgc tctacagttc tgaagttgaa aatcgttttc atgcctagca 960
tctgcttgag ttataaaccc caaggcagcc atgtcataga ctagtgttta ctcttgtttt 1020
gactttgttt taatgcttcc taagacccaa gtgcctcctg ctgtttcctc ctttgtggta 1080
gcctctggcc atctggacct caatccccag ctttcccact ttcagcagtc ctttgctctc 1140
tttgcttcta cctcaaatag ccccaggagt gggctttagt ctccaatatg gagcatctca 1200
agcttctcct gggggatggg gattgggatg ggcggaatct gttttggatc tccgggttat 1260
ttccagtggg tgtaaaagca gagctgggcc tttccctctc ttatccctga gggtgggtaa 1320
gaaggactgt atctacacct gttcttccct accttctctt ttgttaggga ggcctcattc 1380
taagttcctc aagagagtcc ttggcttaaa gctgtagcaa gggtgtgcta ggtgggggat 1440
ttggagcaaa accgtcgagt aggcatgata ctggtatgga gtgggcctgc aaaatcagac 1500
agaaatggct tgagaagccg caggggggagc atgcctgtct ctcagtgata gagtatggga 1560
gggacctccc tagcttggaa aatgagaatt gaaggggtta tgaacaaata ggatgcctag 1620
ttgaggatgt tcccaaagtt ttgtccaatc ttatcattag tagattttat aagccacaga 1680
gacaaaccag aaacggaata atgttacttt ggatgcttta tttttttgtt ctaggtgtgg 1740
ctttgtacat gcagaagaat gctatatgct gcacattttg cctttaaagt cttacgactt 1800
tccccatttt agtctaatgg gaagatacag atgtgcaagt ctgctttttt gtttttttgtt 1860
attatttttt tttttgctct gtgttatgga cattttcaga catgcacaga agtggagagg 1920
atggtccttg gaccccatgt gtccatcacc tagctgcatc acttatcagc tatggtcaac 1980
ctggtttcat ctgtatctct ctcttttcac ctgtattgtt tattgaaaat ccaagacact 2040
atgccaatgc aaccgtgact actttgggag attggtagtc tcttttgatg gtgatagtga 2100
tggggtgcac tatcataatc acatcaggtc tgctttttgc ttttaatgtt aactaatgaa 2160
gttccagaga tgggccttag aaatgtgttt taagaattaa caaggagtct caaaaagaaa 2220
tgagagggat gcttcctttc ccttgcatct acaaaacaag agagagactg ttctgttgta 2280
aaactctttc aaaaattctg atatggtaag gtacttgaga cccttcacca gaatgtcaat 2340
cttttttttct gtgtaacatg gaaacttgtg tgaccattag cattgttatc agcttgtact 2400
ggtctcataa ctctggtttt ggaagaataa tttggaaatt gttgctgtgt tctgtgaaaa 2460
taacctcccc aaaataatta gtaactggtt gttctacttg gtaatttgac accctgttaa 2520
taacgcaatt atttctgtgt tcttaaacag tataaatagt tgtaagtttg catgcatgat 2580
ggaaaaataa aaacctgtat ctctgtcaaa aaaaaaaaaa aaaaaa 2626

<210> 75
<211> 3337
<212> DNA
<213> human

<400> 75
gtcgagcctc tagcccgccc gggtttcctt cgcagtcgcg caccgacgct caaacgcgcg 60
ctccaacccg cagcctcctc ctgcctcacc gcccgaagat ggcggctctc aaactcctct 120
cctccgggct tcggctctgc gcctctgccc gcggatctgg ggcaacctgg tacaagggat 180
gtgtttgttc cttttccacc agtgctcatc gccataccaa gttttataca gatccagtag 240
aagctgtaaa agacatccct gatggtgcca cggttttggt tggtggtttt gggctatgtg 300
gaattccaga gaatcttata gatgctttac tgaaaactgg agtaaaagga ctaactgcag 360
tcagcaacaa tgcaggggtt gacaattttg gtttggggct tttgcttcgg tcgaagcaga 420
taaaacgcat ggtctcttca tatgtgggag aaaatgcaga atttgaacga cagtacttat 480
ctggtgaatt agaagtggag ctgacaccac agggcacact tgcagagagg atccgtgcag 540
gcggggctgg agttcctgca ttttacaccc caacagggta tgggaccctg gtacaagaag 600
gaggatcgcc catcaaatac aacaaagatg gcagtgttgc cattgccagt aagccaagag 660
aggtgaggga gttcaatggt cagcacttta ttttggagga agcaattaca ggggattttg 720
ctttggtgaa agcctggaag gcggaccgag caggaaacgt gattttcagg aaaagtgcaa 780
ggaatttcaa cttgccaatg tgcaaagctg cagaaaccac agtggtagag gttgaagaaa 840
ttgtggatat tggagcattt gctccagaag acatccatat tcctcagatt tatgtacatc 900
gccttataaa gggagaaaaa tatgagaaaa gaattgagcg tttatcaatc cggaaagagg 960
gagatgggga agccaaatct gctaaacctg gagatgacgt aagggaacga atcatcaaga 1020
gggccgctct tgagtttgag gatggcatgt atgctaattt gggcatagga atccctctcc 1080
tggccagcaa ttttatcagc ccaaatataa ctgttcatct tcaaagtgaa aatggagttc 1140
tgggtttggg tccatatcca cgacaacatg aagctgatgc agatctcatc aatgcaggca 1200
aggaaacagt tactattctt ccaggagcct ctttttctc cagcgatgaa tcatttgcaa 1260
tgattagagg tggacacgtc gatctgacaa tgctaggagc gatgcaggtt tccaaatatg 1320
gtgacctggc taactggatg atacctggga agatggtgaa aggaatggga ggtgctatgg 1380
atttagtgtc cagtgcgaaa accaaagtgg tggtcaccat ggagcattct gcaaagggaa 1440
atgcacataa aatcatggag aaatgtacat taccattgac tggaaagcaa tgtgtcaacc 1500
gcattattac tgaaaaggct gtgtttgatg tggacaagaa gaaagggttg actctgattg 1560
agctctggga aggcctgaca gtggatgacg tacaaaagag tactgggtgt gattttgcag 1620
tttcaccaaa actcatgcca atgcagcaga tcgcaaattg aaatatggat atttgtacca 1680
ggctgcgtgt ttttcatttt aaacacacaa gatttaattg aaaggacatc aataatcata 1740
attgtgtatt taacaggtgg tttttatta gttttcttgt gtttcagact ttatgcagcc 1800
atataaactg ttctctaggc atgctgtgac attttaataa aaagcaaaag gagcatttat 1860
aattatctca tttgttaagg ctgagaaggt tgtttttata ataggtaatt atattgaatg 1920
cattttcact gaatatggta tgtatgctaa attatatgaa cctttcccca agaagggccc 1980
tagaaattga tgtggctttc ctcttaaata ttaattatta gtcctgaaag aaagataaca 2040

tatgtgattt ttgtggttag gagagttgct gtcatgattg tttttttcttc agcctcctct 2100
gacttttctt ttggggcttc agattttatg attacatctt gtccccctag aacatccccc 2160
ttcctcccat actgctttta aacagatgcc caagaaggca agcaggaatg cctcttgtgg 2220
gggagggcag ggagaaataa ctagttcaaa ccaactatct atctatgctt tgcaaagact 2280
aaggcgtatt ataggaagag ggctagaaac ctaactgatt cttctcagtt ttctcatttt 2340
aaaacagccc agtattcctt tgtatcctca agggtccttg agaatacttc tgttattgaa 2400
accctgtggg ctacttgtac tgtacctcct ctcaagccaa gaagggctgt gggataattt 2460
accatgaatc cttagtagca atgacagcag agttaaaaaa taaaaggtgt tttactttca 2520
ggctcttgtt ttggttcaga ggagatttta aatattgaat gacacttcta cagaacaacg 2580
gtttttcttc tgccaaggct acttccttta acgaagtgcc tttaattcag ccttatccaa 2640
ctagggaaaa taatgttgga caagtctagg atttgaagag tcagtgaact tttagtgtca 2700
gggaataaac atggtgggta gattaggttt gaaaaaaact tccttagagg tatttattct 2760
caatacctga caggggccca tgggaatgac ttcagaagca tcccggataa tagatgggta 2820
aaaagtctag gcaccctgaa gaacaggtga gacagctggc ctctggacag aggtaggcat 2880
agtacagtac gatatatcat tcctctggtc ctaaatatac aaacttattc atgtttttag 2940
gtgatgatgg tcattgaaac tcacttcttt tcaggtgtag ctacaattgt gtaatgtaca 3000
atattagaga aaggacaggc tttttatgag taacacacac catatataaa acagcctttc 3060
tggctgacca catggttaaa tgcatacctt cccagtactg gggggaaaat gacccttctt 3120
agaatgtgca agttccatag agtaatatat tgatatgatt ttgaaaagaa ttgttgatag 3180
ttacatcttc aaacttatca ttccagtatg catctttaag ataatgtgat tctaagtaga 3240
tgactttata ttcttgatta aagagtgcta tacatgttaa gaaatgcatt aaggaataca 3300
ataaatattc taaactgatg aaaaaaaaaa aaaaaaa 3337

<210> 76
<211> 2460
<212> DNA
<213> human

<400> 76
aaagtcaaac cccgacaccg cggcgggccg gtgagctcac tagctgaccc ggcaggtcag 60
gatctggctt agcggcgccg cgagctccag tgcgcgcacc cgtggccgcc tcccagccct 120
ctttgccgga cgagctctgg gccgccacaa gactaaggaa tggccacccc gcccaagaga 180
agctgcccgt ctttctcagc cagctctgag gggacccgca tcaagaaaat ctccatcgaa 240
gggaacatcg ctgcagggaa gtcaacattt gtgaatatcc ttaaacaatt gtgtgaagat 300
tgggaagtgg ttcctgaacc tgttgccaga tggtgcaatg ttcaaagtac tcaagatgaa 360
tttgaggaac ttacaatgtc tcagaaaaat ggtgggaatg ttcttcagat gatgtatgag 420
aaacctgaac gatggtcttt taccttccaa acatatgcct gtctcagtcg aataagagct 480

cagcttgcct ctctgaatgg caagctcaaa gatgcagaga aacctgtatt attttttgaa 540
cgatctgtgt atagtgacag gtatattttt gcatctaatt tgtatgaatc tgaatgcatg 600
aatgagacag agtggacaat ttatcaagac tggcatgact ggatgaataa ccaatttggc 660
caaagccttg aattggatgg aatcatttat cttcaagcca ctccagagac atgcttacat 720
agaatatatt tacggggaag aaatgaagag caaggcattc ctcttgaata tttagagaag 780
cttcattata aacatgaaag ctggctcctg cataggacac tgaaaaccaa cttcgattat 840
cttcaagagg tgcctatctt aacactggat gttaatgaag actttaaaga caaatatgaa 900
agtctggttg aaaaggtcaa agagtttttg agtactttgt gatcttgctg aagactacag 960
gcagccaaat ggttccagat acttcagctt tgtgtatctt cgtaacttca tattaatata 1020
agtttcttta gaaaacccaa gtttttaatc gtttttgttt taaggaaaaa agatttttaa 1080
aatgaatctt atgcaaaact ttttgatcag tttcttttct tttgtttttt ttttaaaaaa 1140
gacatttaaa gacaaagaca ttatttctca tagcaggaaa tgtagaggta gatggttcca 1200
gtatcagcat agtgactaaa ctacattata aaagatccag cttccttctg tcattcccct 1260
cttttgtctt cctcagcagg ttggcttttt tccctggtgc ctctcacttc gttggtgacc 1320
agtttcttaa actgaaagct ttaatgttac atagtaaatg gtagtgtgtc ctgtgtaaat 1380
tagtgtacct attaaaagtt gcaaagtgga attaaaggaa tccctagaat aaggattctg 1440
aagttttatt ttaaattatt atcttcttaa cagtttagtc ccacctctta cttcctgcct 1500
cagtctgctt tctctactgt ctggattaat taggcagcct gctataaagt taaagtcaca 1560
catttctatt ttgcaaacac tgtgattact ctttgctttg tagtttgctt tgctttgtag 1620
ggttctgctt ttaagttttt ctctttttca gacaaattac tgataaaaat gatattgctc 1680
tatatgtaat atatcctgaa agcattattt tttgttgaat aggaaataaa attaatgaag 1740
acagaggcta gaaagcatcc attaattaat gagacacact taactactta tctctaaacc 1800
atctatgtga atatttgtaa aaataatgaa tggactcatc ttagttctgt atataaatat 1860
attttctttc tagtttgttt agttaaggtg tgcagtgttt ttcctgtgta ttaaaccttt 1920
ccattttacg ttttagaaaa ttttatgtat tttaaaataa ggggaagagt cattttcacc 1980
tttaaactac tatttttctt tccaagtcat ttttgttttt ggtttcttat tcaaagatga 2040
taatttagtg gattaaccag tccagacgca ctgatctttg caaaggagac ttaatttcaa 2100
atctgtaatt accatacata aactgtctca ttatacgtat gcattttttt agtttgtttt 2160
tgtttggtat aaattaattt gttaattaaa tatttcttaa gtataaacct tatgaactac 2220
agtggagcta cactcattga aatgtaattt cagttctaaa aagatgtaat aatcatttta 2280
gaattaaaat ttattctact tttaaataaa ttatgaatat taaaggtgaa aattgtataa 2340
attactttga ttccatttta agtggagaca tatttcagtg atttttagta acctttaaaa 2400
atgtataatg acttttaaaa tttgtagaat tgaaaagacg ctaataaaaa tttattattt 2460

<210> 77

<211> 7680
<212> DNA
<213> human

<400> 77
gcggacactc ctctcggctc ctccccggca gcggcggcgg ctcggagcgg gctccggggc 60
tcgggtgcag cggccagcgg gcctggcggc gaggattacc cggggaagtg gttgtctcct 120
ggctggagcc gcgagacggg cgctcagggc gcggggccgg cggcggcgaa cgagaggacg 180
gactctggcg gccgggtcgt tggccggggg agcgcgggca ccgggcgagc aggccgcgtc 240
gcgctcacca tggtcagcta ctgggacacc ggggtcctgc tgtgcgcgct gctcagctgt 300
ctgcttctca caggatctag ttcaggttca aaattaaaag atcctgaact gagtttaaaa 360
ggcacccagc acatcatgca agcaggccag acactgcatc tccaatgcag gggggaagca 420
gcccataaat ggtctttgcc tgaaatggtg agtaaggaaa gcgaaaggct gagcataact 480
aaatctgcct gtggaagaaa tggcaaacaa ttctgcagta ctttaacctt gaacacagct 540
caagcaaacc acactggctt ctacagctgc aaatatctag ctgtacctac ttcaaagaag 600
aaggaaacag aatctgcaat ctatatattt attagtgata caggtagacc tttcgtagag 660
atgtacagtg aaatccccga aattatacac atgactgaag gaagggagct cgtcattccc 720
tgccgggtta cgtcacctaa catcactgtt actttaaaaa agtttccact tgacactttg 780
atccctgatg gaaaacgcat aatctgggac agtagaaagg gcttcatcat atcaaatgca 840
acgtacaaag aaatagggct tctgacctgt gaagcaacag tcaatgggca tttgtataag 900
acaaactatc tcacacatcg acaaaccaat acaatcatag atgtccaaat aagcacacca 960
cgcccagtca aattacttag aggccatact cttgtcctca attgtactgc taccactccc 1020
ttgaacacga gagttcaaat gacctggagt taccctgatg aaaaaaataa gagagcttcc 1080
gtaaggcgac gaattgacca aagcaattcc catgccaaca tattctacag tgttcttact 1140
attgacaaaa tgcagaacaa agacaaagga ctttatactt gtcgtgtaag gagtggacca 1200
tcattcaaat ctgttaacac ctcagtgcat atatatgata aagcattcat cactgtgaaa 1260
catcgaaaac agcaggtgct tgaaaccgta gctggcaagc ggtcttaccg gctctctatg 1320
aaagtgaagg catttccctc gccggaagtt gtatggttaa aagatgggtt acctgcgact 1380
gagaaatctg ctcgctattt gactcgtggc tactcgttaa ttatcaagga cgtaactgaa 1440
gaggatgcag ggaattatac aatcttgctg agcataaaac agtcaaatgt gtttaaaaac 1500
ctcactgcca ctctaattgt caatgtgaaa ccccagattt acgaaaaggc cgtgtcatcg 1560
tttccagacc cggctctcta cccactgggc agcagacaaa tcctgacttg taccgcatat 1620
ggtatccctc aacctacaat caagtggttc tggcacccct gtaaccataa tcattccgaa 1680
gcaaggtgtg acttttgttc caataatgaa gagtccttta tcctggatgc tgacagcaac 1740
atgggaaaca gaattgagag catcactcag cgcatggcaa taatagaagg aaagaataag 1800
atggctagca ccttggttgt ggctgactct agaatttctg gaatctacat ttgcatagct 1860
tccaataaag ttgggactgt gggaagaaac ataagctttt atatcacaga tgtgccaaat 1920

gggtttcatg ttaacttgga aaaaatgccg acggaaggag aggacctgaa actgtcttgc 1980
acagttaaca agttcttata cagagacgtt acttggattt tactgcggac agttaataac 2040
agaacaatgc actacagtat tagcaagcaa aaaatggcca tcactaagga gcactccatc 2100
actcttaatc ttaccatcat gaatgtttcc ctgcaagatt caggcaccta tgcctgcaga 2160
gccaggaatg tatacacagg ggaagaaatc ctccagaaga aagaaattac aatcagagat 2220
caggaagcac catacctcct gcgaaacctc agtgatcaca cagtggccat cagcagttcc 2280
accactttag actgtcatgc taatggtgtc cccgagcctc agatcacttg gtttaaaaac 2340
aaccacaaaa tacaacaaga gcctggaatt attttaggac caggaagcag cacgctgttt 2400
attgaaagag tcacagaaga ggatgaaggt gtctatcact gcaaagccac caaccagaag 2460
ggctctgtgg aaagttcagc atacctcact gttcaaggaa cctcggacaa gtctaatctg 2520
gagctgatca ctctaacatg cacctgtgtg gctgcgactc tcttctggct cctattaacc 2580
ctccttatcc gaaaaatgaa aaggtcttct tctgaaataa agactgacta cctatcaatt 2640
ataatggacc cagatgaagt tcctttggat gagcagtgtg agcggctccc ttatgatgcc 2700
agcaagtggg agtttgcccg ggagagactt aaactgggca aatcacttgg aagaggggct 2760
tttggaaaag tggttcaagc atcagcattt ggcattaaga aatcacctac gtgccggact 2820
gtggctgtga aaatgctgaa agagggggcc acggccagcg agtacaaagc tctgatgact 2880
gagctaaaaa tcttgaccca cattggccac catctgaacg tggttaacct gctgggagcc 2940
tgcaccaagc aaggagggcc tctgatggtg attgttgaat actgcaaata tggaaatctc 3000
tccaactacc tcaagagcaa acgtgactta ttttttctca acaaggatgc agcactacac 3060
atggagccta agaaagaaaa aatggagcca ggcctggaac aaggcaagaa accaagacta 3120
gatagcgtca ccagcagcga aagctttgcg agctccggct ttcaggaaga taaaagtctg 3180
agtgatgttg aggaagagga ggattctgac ggtttctaca aggagcccat cactatggaa 3240
gatctgattt cttacagttt tcaagtggcc agaggcatgg agttcctgtc ttccagaaag 3300
tgcattcatc gggacctggc agcgagaaac attcttttat ctgagaacaa cgtggtgaag 3360
atttgtgatt ttggccttgc ccgggatatt tataagaacc ccgattatgt gagaaaagga 3420
gatactcgac ttcctctgaa atggatggct cccgaatcta tctttgacaa aatctacagc 3480
accaagagcg acgtgtggtc ttacggagta ttgctgtggg aaatcttctc cttaggtggg 3540
tctccatacc caggagtaca aatggatgag gacttttgca gtcgcctgag ggaaggcatg 3600
aggatgagag ctcctgagta ctctactcct gaaatctatc agatcatgct ggactgctgg 3660
cacagagacc caaaagaaag gccaagattt gcagaacttg tggaaaaact aggtgatttg 3720
cttcaagcaa atgtacaaca ggatggtaaa gactacatcc caatcaatgc catactgaca 3780
ggaaatagtg ggtttacata ctcaactcct gccttctctg aggacttctt caaggaaagt 3840
atttcagctc cgaagtttaa ttcaggaagc tctgatgatg tcagatatgt aaatgctttc 3900
aagttcatga gcctggaaag aatcaaaacc tttgaagaac ttttaccgaa tgccacctcc 3960

atgtttgatg actaccaggg cgacagcagc actctgttgg cctctcccat gctgaagcgc 4020
ttcacctgga ctgacagcaa acccaaggcc tcgctcaaga ttgacttgag agtaaccagt 4080
aaaagtaagg agtcggggct gtctgatgtc agcaggccca gtttctgcca ttccagctgt 4140
gggcacgtca gcgaaggcaa gcgcaggttc acctacgacc acgctgagct ggaaaggaaa 4200
atcgcgtgct gctccccgcc cccagactac aactcggtgg tcctgtactc caccccaccc 4260
atctagagtt tgacacgaag ccttatttct agaagcacat gtgtatttat acccccagga 4320
aactagcttt tgccagtatt atgcatatat aagtttacac ctttatcttt ccatgggagc 4380
cagctgcttt ttgtgatttt tttaatagtg cttttttttt ttgactaaca agaatgtaac 4440
tccagataga gaaatagtga caagtgaaga acactactgc taaatcctca tgttactcag 4500
tgttagagaa atccttccta aacccaatga cttccctgct ccaacccccg ccacctcagg 4560
gcacgcagga ccagtttgat tgaggagctg cactgatcac ccaatgcatc acgtacccca 4620
ctgggccagc cctgcagccc aaaacccagg gcaacaagcc cgttagcccc aggggatcac 4680
tggctggcct gagcaacatc tcgggagtcc tctagcaggc ctaagacatg tgaggaggaa 4740
aaggaaaaaa agcaaaaagc aagggagaaa agagaaaccg ggagaaggca tgagaaagaa 4800
tttgagacgc accatgtggg cacggagggg gacggggctc agcaatgcca tttcagtggc 4860
ttcccagctc tgacccttct acatttgagg gcccagccag gagcagatgg acagcgatga 4920
ggggacattt tctggattct gggaggcaag aaaaggacaa atatcttttt tggaactaaa 4980
gcaaatttta gacctttacc tatggaagtg gttctatgtc cattctcatt cgtggcatgt 5040
tttgatttgt agcactgagg gtggcactca actctgagcc catacttttg gctcctctag 5100
taagatgcac tgaaaactta gccagagtta ggttgtctcc aggccatgat ggccttacac 5160
tgaaaatgtc acattctatt ttgggtatta atatatagtc cagacactta actcaatttc 5220
ttggtattat tctgttttgc acagttagtt gtgaaagaaa gctgagaaga atgaaaatgc 5280
agtcctgagg agagttttct ccatatcaaa acgagggctg atggaggaaa aaggtcaata 5340
aggtcaaggg aagaccccgt ctctatacca accaaaccaa ttcaccaaca cagttgggac 5400
ccaaaacaca ggaagtcagt cacgtttcct tttcatttaa tggggattcc actatctcac 5460
actaatctga aaggatgtgg aagagcatta gctggcgcat attaagcact ttaagctcct 5520
tgagtaaaaa ggtggtatgt aatttatgca aggtatttct ccagttggga ctcaggatat 5580
tagttaatga gccatcacta gaagaaaagc ccattttcaa ctgctttgaa acttgcctgg 5640
ggtctgagca tgatgggaat agggagacag ggtaggaaag ggcgcctact cttcagggtc 5700
taaagatcaa gtgggccttg gatcgctaag ctggctctgt ttgatgctat ttatgcaagt 5760
tagggtctat gtatttagga tgcgcctact cttcagggtc taaagatcaa gtgggccttg 5820
gatcgctaag ctggctctgt ttgatgctat ttatgcaagt tagggtctat gtatttagga 5880
tgtctgcacc ttctgcagcc agtcagaagc tggagaggca acagtggatt gctgcttctt 5940
ggggagaaga gtatgcttcc ttttatccat gtaatttaac tgtagaacct gagctctaag 6000

taaccgaaga atgtatgcct ctgttcttat gtgccacatc cttgtttaaa ggctctctgt 6060
atgaagagat gggaccgtca tcagcacatt ccctagtgag cctactggct cctggcagcg 6120
gcttttgtgg aagactcact agccagaaga gaggagtggg acagtcctct ccaccaagat 6180
ctaaatccaa acaaaagcag gctagagcca gaagagagga caaatctttg ttgttcctct 6240
tctttacaca tacgcaaacc acctgtgaca gctggcaatt ttataaatca ggtaactgga 6300
aggaggttaa actcagaaaa aagaagacct cagtcaattc tctacttttt tttttttttt 6360
tccaaatcag ataatagccc agcaaatagt gataacaaat aaaaccttag ctgttcatgt 6420
cttgatttca ataattaatt cttaatcatt aagagaccat aataaatact ccttttcaag 6480
agaaaagcaa aaccattaga attgttactc agctccttca aactcaggtt tgtagcatac 6540
atgagtccat ccatcagtca aagaatggtt ccatctggag tcttaatgta gaaagaaaaa 6600
tggagacttg taataatgag ctagttacaa agtgcttgtt cattaaaata gcactgaaaa 6660
ttgaaacatg aattaactga taatattcca atcatttgcc atttatgaca aaaatggttg 6720
gcactaacaa agaacgagca cttcctttca gagtttctga gataatgtac gtggaacagt 6780
ctgggtggaa tggggctgaa accatgtgca agtctgtgtc ttgtcagtcc aagaagtgac 6840
accgagatgt taattttagg gacccgtgcc ttgtttccta gcccacaaga atgcaaacat 6900
caaacagata ctcgctagcc tcatttaaat tgattaaagg aggagtgcat ctttggccga 6960
cagtggtgta actgtgtgtg tgtgtgtgtg tgtgtgtgtg tgtgtgtgtg tgtgggtgtg 7020
ggtgtatgtg tgttttgtgc ataactattt aaggaaactg gaattttaaa gttactttta 7080
tacaaaccaa gaatatatgc tacagatata agacagacat ggtttggtcc tatatttcta 7140
gtcatgatga atgtattttg tataccatct tcatataata tacttaaaaa tatttcttaa 7200
ttgggatttg taatcgtacc aacttaattg ataaacttgg caactgcttt tatgttctgt 7260
ctccttccat aaatttttca aaatactaat tcaacaaaga aaaagctctt ttttttccta 7320
aaataaactc aaatttatcc ttgtttagag cagagaaaaa ttaagaaaaa ctttgaaatg 7380
gtctcaaaaa attgctaaat attttcaatg gaaaactaaa tgttagttta gctgattgta 7440
tggggttttc gaacctttca ctttttgttt gttttaccta tttcacaact gtgtaaattg 7500
ccaataattc ctgtccatga aaatgcaaat tatccagtgt agatatattt gaccatcacc 7560
ctatggatat tggctagttt tgcctttatt aagcaaattc atttcagcct gaatgtctgc 7620
ctatatattc tctgctcttt gtattctcct ttgaacccgt taaaacatcc tgtggcactc 7680

<210> 78
<211> 3160
<212> DNA
<213> human

<400> 78
attctcgccg cgcccgggcg gacgatccag cgaacagccc cgcttctaac ccgagatgct 60
gctgccggcg cccgcgctcc gccgcgccct gctgtcccgc ccctggaccg gggccggcct 120

gcggtggaag cacacctcct ccctgaaggt ggccaacgag cccgtcttag ccttcacgca 180
gggcagccct gagcgagatg ccctgcaaaa ggccttgaag gacctgaagg gccggatgga 240
agccatccca tgcgtggtgg gggatgagga ggtgtggacg tcggacgtgc agtaccaagt 300
gtcgcctttt aaccatggac ataaggtggc caagttctgt tatgcagaca agagcctgct 360
caacaaagcc attgaggctg ccctggctgc ccggaaagag tgggacctga agcctattgc 420
agaccgggcc cagatcttcc tgaaggcggc agacatgctg agtgggccgc gcagggctga 480
gatcctcgcc aagaccatgg tgggacaggg taagaccgtg atccaagcgg agattgacgc 540
tgcagcggaa ctcatcgact tcttccggtt caatgccaag tatgcggtgg agctggaggg 600
gcagcagccc atcagcgtgc cccgagcac caacagcacg gtgtaccggg gtctggaggg 660
cttcgtggcg gccatctcgc cctttaactt cactgcaatc ggcggcaacc tggcgggggc 720
accggccctg atgggcaacg tggtcctatg gaagcccagt gacactgcca tgctggccag 780
ctatgctgtc taccgcatcc ttcgggaggc tggcctgccc cccaacatca tccagtttgt 840
gccagctgat gggcccctat ttggggacac tgtcaccagc tcagagcacc tctgtggcat 900
caacttcaca ggcagtgtgc ccaccttcaa acacctgtgg aagcaggtgg cccagaacct 960
ggaccggttc cacaccttcc cacgcctggc tggagagtgc ggcggaaaga acttccactt 1020
cgtgcaccgc tcggccgacg tggagagcgt ggtgagcggg accctccgct cagccttcga 1080
gtacggtggc cagaagtgtt ccgcctgctc gcgtctctac gtgccgcact cgctgtggcc 1140
gcagatcaaa gggcggctgc tggaggagca cagtcggatc aaagtgggcg accctgcaga 1200
ggattttggg accttcttct ctgcagtgat tgatgccaag tcctttgccc gtatcaagaa 1260
gtggctggag cacgcgcgct cctcgcccag cctcaccatc ctggctgggg gcaagtgtga 1320
tgactccgtg ggctactttg tggagccctg catcgtggag agcaaggacc ctcaggagcc 1380
catcatgaag gaggagatct tcgggcctgt actgtctgtg tacgtctacc cggacgacaa 1440
gtacaaggag acgctgcagc tggttgacag caccaccagc tatggcctca cgggggcagt 1500
gttctcccag gataaggacg tcgtgcagga ggccacaaag gtgctgagga atgctgccgg 1560
caacttctac atcaacgaca agtccactgg ctcgatagtg ggccagcagc cctttggggg 1620
ggcccgagcc tctggaacca atgacaagcc agggggccca cactacatcc tgcgctggac 1680
gtcgccgcag gtcatcaagg agacacataa gcccctgggg gactggagct acgcgtacat 1740
gcagtgagcc cctctcgggc tccaccgtcc agctgtctgt ccgtccaggt ggccgacctc 1800
actgcacaga ccccactcca gcccctccac cccttcttca tgcacagctg cctttctata 1860
atccgggctt gactcccttc ttaccactgt attctggcct ctcccatgcc tcaggctctg 1920
gtttgagatc gtgctgggga ggaacatggc cactacccct tatcccatcg gccatgtggg 1980
aggtatgacc ctggtgcctg gcaggttctc cctctgccct ccactgggcc cagtggctca 2040
gggacctggg gaaaggagat ggagcagctc ttgggatcct ttggggaaaa ggaggccatt 2100
ctgggcccct tggcaaacct caccactcac agaggctcct ggccttgatc cctgcccctc 2160

caggtgtcca gggtaaagtg taactcagac tgacctgtgg ggcacagggg gcaccagctg 2220
gccttgccct ctctggtctg ggctgtctac cttcctcact gtatctttgc ccagacccac 2280
ctgggccagt aggcccctgt ccccagccac acaccttaga tgctggcatg ccttactcca 2340
ggtgcctgtg tttggccgag gcctgtgtga ttcccggtct gcaccacatg gcggggttgg 2400
ggggccgctg gaggccacct gccaaggcgt gggatgggat ggtcctgccg gtttaggccg 2460
tgattctgga aaaccttgga tgggccttcg tcctatgtca gccttccctt tgatcctcag 2520
gccctacctg tagagacctc cactcctaga gccagtctca gggtctggga tttccctgca 2580
ggagctcagc caccactgtg ccatggtgac acaggccaag gcagacattg gccctccctt 2640
ctcccagccc ccagaggcct ggccttgggt tcgtcagcat gggccgagga cgttgcctgt 2700
agaatcctcc tctgcctggg agtggctctg tgtggaccag tccctcactg gcccattctt 2760
tttttgacgc agccaatctg tgaccacgat tcctcccaca gatgcctcct gcttggattc 2820
tgagtggtca gagatctgta aagcatgact ttcaaggatg gttcttaggg gactgtgaaa 2880
gtgttgggtc ttcctccagg atgcctgcat gggaccccac ccggagctgg tgtggccatt 2940
ccccaagtgc cactggccca tggatggggg tgggtgctgg tgccagctgg gctgggtgtg 3000
ggttctgtgt ccttccagga tatgtgtcat ttcccatgag gggccggggc aggtggctgg 3060
gtgggggcac aggctggagt attcttagtt ctactggttc tacactgtga ggtggcaatg 3120
ggatttgctc agatgccacc caataaaatg cctgttactt 3160

<210> 79
<211> 1546
<212> DNA
<213> human

<400> 79
cacggccgga gagacgcgga ggaggagaca tgagccggcg ggcgcccaga cggagcggcc 60
gtgacgcttt cgcgctgcag ccgcgcgccc cgaccccgga gcgctgaccc ctggccccac 120
gcagctccgc gcccgggccg gagagcgcaa ctcggcttcc agacccgccg cgcatgctgt 180
ccccggactg agccgggcag ccagcctccc acggacgccc ggacggccgg ccggccagca 240
gtgagcgagc ttccccgcac cggccaggcg cctcctgcac agcggctgcc gccccgcagc 300
ccctgcgcca gcccggaggg cgcagcgctc gggaggagcc gcgcggggcg ctgatgccgc 360
agggcgcgcc gcggagcgcc ccggagcagc agagtctgca gcagcagcag ccggcgagga 420
gggagcagca gcagcggcgg cggcggcggc ggcggcggcg gaggcgcccg gtcccggccg 480
cgcggagcgg acatgtgcag gctgggctag gagccgccgc ctccctcccg cccagcgatg 540
tattcagcgc cctccgcctg cacttgcctg tgtttacact tcctgctgct gtgcttccag 600
gtacaggtgc tggttgccga ggagaacgtg gacttccgca tccacgtgga gaaccagacg 660
cgggctcggg acgatgtgag ccgtaagcag ctgcggctgt accagctcta cagccggacc 720
agtgggaaac acatccaggt cctgggccgc aggatcagtg cccgcggcga ggatggggac 780
aagtatgccc agctcctagt ggagacagac accttcggta gtcaagtccg gatcaagggc 840

aaggagacgg aattctacct gtgcatgaac cgcaaaggca agctcgtggg gaagcccgat 900
ggcaccagca aggagtgtgt gttcatcgag aaggttctgg agaacaacta cacggccctg 960
atgtcggcta agtactccgg ctggtacgtg ggcttcacca agaaggggcg gccgcggaag 1020
ggccccaaga cccgggagaa ccagcaggac gtgcatttca tgaagcgcta ccccaagggg 1080
cagccggagc ttcagaagcc cttcaagtac acgacggtga ccaagaggtc ccgtcggatc 1140
cggcccacac accctgccta ggccaccccg ccgcggcccc tcaggtcgcc ctggccacac 1200
tcacactccc agaaaactgc atcagaggaa tatttttaca tgaaaaataa ggaagaagct 1260
ctatttttgt acattgtgtt taaaagaaga caaaaactga accaaaactc ttgggggggag 1320
gggtgataag gattttattg ttgacttgaa acccccgatg acaaaagact cacgcaaagg 1380
gactgtagtc aacccacagg tgcttgtctc tctctaggaa cagacaactc taaactcgtc 1440
cccagaggag gacttgaatg aggaaaccaa cactttgaga aaccaaagtc ctttttccca 1500
aaggttctga aaggaaaaaa aaaaaaaaac aaaaaaaaaa aaaaaa 1546

<210> 80
<211> 2819
<212> DNA
<213> human

<400> 80
ctgggcccag ctcccccgag aggtggtcgg atcctctggg ctgctcggtc gatgcctgtg 60
ccactgacgt ccaggcatga ggtggttcct gccctggacg ctggcagcag tgacagcagc 120
agccgccagc accgtcctgg ccacggccct ctctccagcc cctacgacca tggactttac 180
cccagctcca ctggaggaca cctcctcacg cccccaattc tgcaagtggc catgtgagtg 240
cccgccatcc ccaccccgct gcccgctggg ggtcagcctc atcacagatg gctgtgagtg 300
ctgtaagatg tgcgctcagc agcttgggga caactgcacg gaggctgcca tctgtgaccc 360
ccaccggggc ctctactgtg actacagcgg ggaccgcccg aggtacgcaa taggagtgtg 420
tgcacaggtg gtcggtgtgg gctgcgtcct ggatggggtg cgctacaaca acggccagtc 480
cttccagcct aactgcaagt acaactgcac gtgcatcgac ggcgcggtgg gctgcacacc 540
actgtgcctc cgagtgcgcc ccccgcgtct ctggtgcccc cacccgcggc gcgtgagcat 600
acctggccac tgctgtgagc agtgggtatg tgaggacgac gccaagaggc cacgcaagac 660
cgcaccccgt gacacaggag ccttcgatgc tgtgggtgag gtggaggcat ggcacaggaa 720
ctgcatagcc tacacaagcc cctggagccc ttgctccacc agctgcggcc tgggggtctc 780
cactcggatc tccaatgtta acgcccagtg ctggcctgag caagagagcc gcctctgcaa 840
cttgcggcca tgcgatgtgg acatccatac actcattaag gcagggaaga agtgtctggc 900
tgtgtaccag ccagaggcat ccatgaactt cacacttgcg ggctgcatca gcacacgctc 960
ctatcaaccc aagtactgtg gagtttgcat ggacaatagg tgctgcatcc cctacaagtc 1020
taagactatc gacgtgtcct tccagtgtcc tgatgggctt ggcttctccc gccaggtcct 1080

atggattaat gcctgcttct gtaacctgag ctgtaggaat cccaatgaca tctttgctga 1140
cttggaatcc taccctgact tctcagaaat tgccaactag gcaggcacaa atcttgggtc 1200
ttggggacta acccaatgcc tgtgaagcag tcagccctta tggccaataa cttttcacca 1260
atgagcctta gttaccctga tctggaccct tggcctccat ttctgtctct aaccattcaa 1320
atgacgcctg atggtgctgc tcaggcccat gctatgagtt ttctccttga tatcattcag 1380
catctactct aaagaaaaat gcctgtctct agctgttctg gactacaccc aagcctgatc 1440
cagcctttcc aagtcactag aagtcctgct ggatcttgcc taaatcccaa gaaatggaat 1500
caggtagact tttaatatca ctaatttctt ctttagatgc caaaccacaa gactctttgg 1560
gtccattcag atgaatagat ggaatttgga acaatagaat aatctattat ttggagcctg 1620
ccaagaggta ctgtaatggg taattctgac gtcagcgcac caaaactatc ctgattccaa 1680
atatgtatgc acctcaaggt catcaaacat ttgccaagtg agttgaatag ttgcttaatt 1740
ttgattttta atggaaagtt gtatccatta acctgggcat tgttgaggtt aagtttctct 1800
tcacccctac actgtgaagg gtacagatta ggtttgtccc agtcagaaat aaaatttgat 1860
aaacattcct gttgatggga aaagccccca gttaatactc cagagacagg gaaaggtcag 1920
cccgtttcag aaggaccaat tgactctcac actgaatcag ctgctgactg gcagggcttt 1980
gggcagttgg ccaggctctt ccttgaatct tctcccttgt cctgcttggg gttcatagga 2040
attggtaagg cctctggact ggcctgtctg gcccctgaga gtggtgccct ggaacactcc 2100
tctactctta cagagccttg agagacccag ctgcagacca tgccagaccc actgaaatga 2160
ccaagacagg ttcaggtagg ggtgtgggtc aaaccaagaa gtgggtgccc ttggtagcag 2220
cctggggtga cctctagagc tggaggctgt gggactccag gggcccccgt gttcaggaca 2280
catctattgc agagactcat ttcacagcct ttcgttctgc tgaccaaatg gccagttttc 2340
tggtaggaag atggaggttt accggttgtt tagaaacaga aatagactta ataaaggttt 2400
aaagctgaag aggttgaagc taaaaggaaa aggttgttgt taatgaatat caggctatta 2460
tttattgtat taggaaaata taatatttac tgttagaatt cttttattta gggccttttc 2520
tgtgccagac attgctctca gtgctttgca tgtattagct cactgaatct tcacgacaat 2580
gttgagaagt tcccattatt atttctgttc ttacaaatgt gaaacggaag ctcatagagg 2640
tgagaaaact caaccagagt cacccagttg gtgactggga aagttaggat tcagatcgaa 2700
attggactgt ctttataacc catattttcc ccctgttttt agagcttcca aatgtgtcag 2760
aataggaaaa cattgcaata aatggcttga tttttaaaa aaaaaaaaaa aaaaaaaaa 2819

<210> 81
<211> 2584
<212> DNA
<213> human

<400> 81
tggcggcggc ggcggcggtt gtcccggctg tgccggttgg tgtggcccgt cagcccgcgt 60
accacagcgc ccgggccgcg tcgagcccag tacagccaag ccgctgcggc cgggtccggc 120

gcgggcggcg cgcgcagacg gagggcggcg gccgcggcca gggcggcccg tgggaccgcg 180
ggcccccggc gcagcgctgc ccggctcccg gccctgccgg cctcctccct tggcgccgcg 240
gccatggcgg ccagcgcgaa gcggaagcag gaggagaagc acctgaagat gctgcgggac 300
atgaccggcc tcccgcgcaa ccgaaagtgc ttcgactgcg accagcgcgg ccccacctac 360
gttaacatga cggtcggctc cttcgtgtgt acctcctgct ccggcagcct gcgaggatta 420
aatccaccac acagggtgaa atctatctcc atgacaacat tcacacaaca ggaaattgaa 480
ttcttacaaa aacatggaaa tgaagtctgt aaacagattt ggctaggatt atttgatgat 540
agatcttcag caattccaga cttcagggat ccacaaaaag tgaaagagtt tctacaagaa 600
aagtatgaaa agaaaagatg gtatgtcccg ccagaacaag ccaaagtcgt ggcatcagtt 660
catgcatcta tttcagggtc ctctgccagt agcacaagca gcacacctga ggtcaaacca 720
ctgaaatctc ttttagggga ttctgcacca acactgcact taaataaggg cacacctagt 780
cagtccccag ttgtaggtcg ttctcaaggg cagcagcagg agaagaagca atttgacctt 840
ttaagtgatc tcggctcaga catctttgct gctccagctc ctcagtcaac agctacagcc 900
aattttgcta actttgcaca tttcaacagt catgcagctc agaattctgc aaatgcagat 960
tttgcaaact ttgatgcatt tggacagtct agtggttcga gtaattttgg aggtttcccc 1020
acagcaagtc actctccttt tcagccccaa actacaggtg gaagtgctgc atcagtaaat 1080
gctaattttg ctcattttga taacttcccc aaatcctcca gtgctgattt tggaaccttc 1140
aatacttccc agagtcatca aacagcatca gctgttagta aagtttcaac gaacaaagct 1200
ggtttacaga ctgcagacaa atatgcagca cttgctaatt tagacaatat cttcagtgcc 1260
gggcaaggtg gtgatcaggg aagtggcttt gggaccacag gtaaagctcc tgttggttct 1320
gtggtttcag ttcccagtca gtcaagtgca tcttcagaca agtatgcagc tctggcagaa 1380
ctagacagcg ttttcagttc tgcagccacc tccagtaatg cgtatacttc cacaagtaat 1440
gctagcagca atgtttttgg aacagtgcca gtggttgctt ctgcacagac acagcctgct 1500
tcatcaagtg tgcctgctcc atttggacgt acgccttcca caaatccatt tgttgctgct 1560
gctggtcctt ctgtggcatc ttctacaaac ccatttcaga ccaatgccag aggagcaaca 1620
gcggcaacct ttggcactgc atccatgagc atgcccacgg gattcggcac tcctgctccc 1680
tacagtcttc ccaccagctt tagtggcagc tttcagcagc ctgccttttcc agcccaagca 1740
gctttccctc aacagacagc tttttctcaa cagcccaatg gtgcaggttt tgcagcattt 1800
ggacaaacaa agccagtagt aacccctttt ggtcaagttg cagctgctgg agtatctagt 1860
aatcctttta tgactggtgc accaacagga caatttccaa caggaagctc atcaaccaat 1920
cctttcttat agccttatat agacaattta ctggaacgaa cttttatgtg gtcacattac 1980
atctctccac ctcttgcact gttgtcttgt ttcactgatc ttagctttaa acacaagaga 2040
agtctttaaa aagcctgcat tgtgtattaa acaccaggta atatgtgcaa aaccgagggc 2100
tccagtaaca ccttctaacc tgtgaattgg cagaaaaggg tagcggtatc atgtatatta 2160

aaattggcta atattaagtt attgcagata ccacattcat tatgctgcag tactgtacat 2220
attttttctta gaaattagct atttgtgcat atcagtattt gtaactttaa cacattgtta 2280
tgtgagaaat gttactgggg aaatagatca gccactttta aggtgctgtc atatatcttg 2340
gaatgaatga cctaaaatca ttttaaccat tgctactgga aagtaacaga gtcaaaattg 2400
gaaggtttta ttcattcttg aatttttcct ttctaaagag ctcttctatt tatacatgcc 2460
taaattcttt taaaatgtag agggatacct gtctgcataa taaagctgat catgttttgc 2520
tacagtttgc aggtgaaaaa aaataaatat tataaaataa aaaaaaaaaa aaagaaaaaa 2580
aaaa 2584

<210> 82
<211> 2115
<212> DNA
<213> human

<400> 82
gaaatgaacc tctcttattg atttttattg gcctagagcc aggagtactg cattcagttg 60
actttcaggg taaaaagaaa acagtcctgg ttgttgtcat cataaacata tggaccagtg 120
tgatggtgaa atgagatgag gctccgcaat ggaactgtag ccactgcttt agcatttatc 180
acttccttcc ttactttgtc ttggtatact acatggcaaa atgggaaaga aaaactgatt 240
gcttatcaac gagaattcct tgctttgaaa gaacgtcttc gaatagctga acacagaatc 300
tcacagcgct cttctgaatt aaatacgatt gtgcaacagt tcaagcgtgt aggagcagaa 360
acaaatggaa gtaaggatgc gttgaataag ttttcagata ataccctaaa gctgttaaag 420
gagttaacaa gcaaaaaatc tcttcaagtg ccaagtattt attatcattt gcctcattta 480
ttgaaaaatg aaggaagtct tcaacctgct gtacagattg gcaacggaag aacaggagtt 540
tcaatagtca tgggcattcc cacagtgaag agagaagtta aatcttacct catagaaact 600
cttcattccc ttattgataa cctgtatcct gaagagaagt tggactgtgt tatagtagtc 660
ttcataggag agacagatat tgattatgta catggtgttg tagccaacct ggagaaagaa 720
ttttctaaag aaatcagttc tggcttggtg gaagtcatat cacccccctga aagctattat 780
cctgacttga caaacctaaa ggagacattt ggagactcca aagaaagagt aagatggaga 840
acaaagcaaa acctagatta ctgttttcta atgatgtatg ctcaagaaaa gggcatatat 900
tacattcagc ttgaagatga tattattgtc aaacaaaatt attttaatac cataaaaaat 960
tttgcacttc aactttcttc tgaggaatgg atgattctag agttttccca gctgggcttc 1020
attggtaaaa tgtttcaagc gccggatctt actctgattg tagaattcat attcatgttt 1080
tacaaggaga aacccattga ttggctcctg gaccatattc tctgggtgaa agtctgcaac 1140
cctgaaaaag atgcaaaaca ttgtgataga cagaaagcaa atctgcgaat tcgcttcaga 1200
ccttcccttt tccaacatgt tggtctgcac tcatcactat caggaaaaat ccaaaaactc 1260
acggataaag attatatgaa accattactt cttaaaatcc atgtaaaccc acctgcggag 1320

gtatctactt ccttgaaggt ctaccaaggg catacgctgg agaaaactta catgggagag 1380
gatttcttct gggctatcac accgatagct ggagactaca tcttgtttaa atttgataaa 1440
ccagtcaatg tagaaagtta tttgttccat agcggcaacc aagaacatcc tggagatatt 1500
ctgctaaaca caactgtgga agttttgcct tttaagagtg aaggtttgga aataagcaaa 1560
gaaaccaaag acaaacgatt agaagatggc tatttcagaa taggaaaatt tgagaatggt 1620
gttgcagaag gaatggtgga tccaagtctc aatcccattt cagcctttcg actttcagtt 1680
attcagaatt ctgctgtttg ggccattctt aatgagattc atattaaaaa agccaccaac 1740
tgatcatctg agaaaccaac acattttttc ctgtgaattt gttaattaaa gatagttaag 1800
catgtatctt ttttttattt ctacttgaac actacctctt gtgaagtcta ctgtagataa 1860
gacgattgtc atttccactt ggaaagtgaa tctcccataa taattgtatt tgtttgaaac 1920
taagctgtcc tcagatttta acttgactca aacatttttc aattatgaca gcctgttaat 1980
atgacttgta ctattttggt attatactaa tacataagag ttgtacatat tgttacattc 2040
tttaaatttg agaaaaacta atgttacata cattttatga agggggtact tttgaggttc 2100
acttatttta ctatt 2115

<210> 83
<211> 1635
<212> DNA
<213> human

<400> 83
gggggtggcg gggacgcgag tggcggccgc ggggccccgg acaagggtcc gcagagctgc 60
agccttcgag ggccagccct ctccgagtcc ggggctgggt cccaccagtg acaaggcggc 120
agccccgcgc acaccaaaga gaaagcggct gtggcggcag cggcagcccc agccatgctg 180
tgttatgtga cgaggccgga cgcggtgctg atggaggtgg aggtggaggc gaaagccaac 240
ggcgaggact gcctcaacca ggtgtgcagg cgactgggaa tcatagaagt tgactatttt 300
ggactgcaat ttacgggtag caaaggtgaa agtttatggc taaacctgag aaaccggatc 360
tcccagcaga tggatgggct agccccttac aggcttaaac ttagagtcaa gttcttcgtg 420
gagcctcatc tcatcttaca ggagcagact aggcatatct tttcttgca catcaaggag 480
gccctcttgg caggccacct cttgtgttcc ccagagcagg cagtggaact cagtgccctc 540
ctggcccaga ccaagtttgg agactacaac cagaacactg ccaagtataa ctatgaggag 600
ctctgtgcca aggagctctc ctctgccacc ttgaacagca ttgttgcaaa acataaggag 660
ttggagggga ccagccaggc ttcagctgaa taccaagttt tgcagattgt gtcggcaatg 720
gaaaactatg gcatagaatg gcattctgtg cgggatagcg aagggcagag actgctcatt 780
ggggttggac ctgaaggaat ctcaatttgt aaagatgact ttagcccaat taataggata 840
gcttatcctg tggtgcagat ggccacccag tcaggaaaga atgtatattt gacggtcacc 900
aaggaatctg ggaacagcat cgtgctcttg tttaaaatga tcagcaccag ggcggccagc 960
gggctctacc gagcgataac agagacgcac gcattctaca ggtgtgacac agtgaccagc 1020

gccgtgatga tgcagtatag ccgtgacttg aagggccact tggcatctct gtttctgaat 1080
gaaaacatta accttggcaa gaaatatgtc tttgatatta aaagaacatc aaaggaggtg 1140
tatgaccatg ccaggagggc tctgtacaat gctggcgttg tggacctcgt ttcaagaagc 1200
aaccagagcc cttcacactc gcctctgaag tcctcagaaa gcagcatgaa ctgcagcagc 1260
tgcgagggcc tcagctgcca gcagacccgg gtgctgcagg agaagctacg caagctgaag 1320
gaagccatgc tgtgcatggt gtgctgcgag gaggagatca actccacctt ctgtccctgt 1380
ggccacactg tgtgctgtga gagctgcgcc gcccagctac agtcatgtcc cgtctgcagg 1440
tcgcgtgtgg agcatgtcca gcacgtctat ctgccaacgc acaccagtct tctcaatctg 1500
actgtaatct aatctgttgt gcttttgttg gacttggcat gtttccatga actgcactat 1560
tataaactat taaaatgata gatgttggag aaagtaatta ttccaacacc catctgccca 1620
tgcgatgtta aaaaa 1635

<210> 84
<211> 2118
<212> DNA
<213> human

<400> 84
ctgtgaagat ggcgctctcc agggtgtgct gggctcggtc ggctgtgtgg ggctcggcag 60
tcacccctgg acattttgtc acccggaggc tgcaacttgg tcgctctggc ctggcttggg 120
gggcccctcg gtcttcaaag cttcaccttt ctccaaaggc agatgtgaag aacttgatgt 180
cttatgtggt aaccaagaca aaagcgatta atgggaaata ccatcgtttc ttgggtcgtc 240
atttcccccg cttctatatc ctgtacacaa tcttcatgaa aggattgcag atgttatggg 300
ctgatgccaa aaaggctaga agaataaaga caaatatgtg gaagcacaat ataaagtttc 360
atcaacttcc ataccgggag atggagcatt tgagacagtt ccgccaagac gtcaccaagt 420
gtcttttcct aggtattatt tccattccac cttttgccaa ctacctggtc ttcttgctaa 480
tgtacctgtt tcccaggcaa ctactgatca ggcatttctg gaccccaaaa caacaaactg 540
atttcttaga tatctatcat gctttccgga agcagtccca cccagaaatt attagttatt 600
tagaaaaggt catccctctc atttctgatg caggactccg gtggcgtctg acagatctgt 660
gcaccaagat acagcgtggt acccacccag caatacatga tatcttggct ctgagagagt 720
gtttctctaa ccatcctctg ggcatgaacc aactccaggc tttgcacgtg aaagccttga 780
gccgggccat gcttctcaca tcttacctgc ctcctccctt gttgagacat cgtttgaaga 840
ctcatacaac tgtgattcac caactggaca aggctttggc aaagctgggg attggccagc 900
tgactgctca ggaagtaaaa tcggcttgtt atctccgtgg cctgaattct acgcatattg 960
gtgaagatag gtgtcgaact tggctgggag aatggctgca gatttcctgc agcctgaaag 1020
aagctgagct gtctcttctg ctgcacaacg tggtcctgct ctccaccaac taccttggga 1080
caaggcgctg aatgaaccat ggagcggatg gcattgtcct gcagtcgtat agtatagcag 1140

tgcaggaaca aacagcactt gccagcaaag tctgtgtgta ctgttaagtg tgtgggaggc 1200
agagagagga gcaggggcca tgggcttcac agcatggcac acctgtggga actgcagaca 1260
ttcctctcac agctagaact gaaacaaacc ctcttgctag ggtggtccg tgtgaggtgt 1320
catcctgtcc ccctcataat tactaatagc tggaactggc agcagcctct actgggcttt 1380
tactgtgatg tgttcagttc atgtcctagg aagtcagctt ttgccccagg tgggaatcct 1440
tatttggctt aggactgatc cacttccatg ttacttacat ctgtgggttt ttgttgttgc 1500
tgttagaaaa tttttggctg gtgaaaacag cactcctttg gctggagcac ttgtgtccat 1560
gcatgtactt gggtgtttcc ctccatcctt tctgatatga ccaaaaatca agttgttttg 1620
ttttttgtca ccttcactgg catgggctaa ccacttcttt ttcaaaccct ctgaacacct 1680
ttttctgatg ggtaacttgc aggaatattc tattggaaaa gataacagga agtacaagtg 1740
cttcttgacc ccttcctcaa tgtttctagc cttcactctc cattgtcttt tctgggctgt 1800
attacagccc tctgtggatc ttcaactctg ctgcctccac tgtgatgcag cagtccaact 1860
gtaactgaca gtggctgcct tctctgggcc atggatcaca cctgtaaggt actaattact 1920
gcccagcctg gggagatcag gagaggtctg catagttagt aagttgggtt tagcttttgt 1980
gtgtgcatca gtgacttaga gttctgtaat aacttattgt aaatgcatga agcactgttt 2040
ttaaacccaa gtaaagactg cttgaaacct gttgatggaa aaaaaaaaaa aaaaaaaaaa 2100
aaaaaaaaaa aaaaaaaa 2118

<210> 85
<211> 4221
<212> DNA
<213> human

<400> 85
cgataacgat ttgtgttgtg agaggcgcaa gctgcgattt ctgctgaact tggaggcatt 60
tctacgactt ttctctcagc tgaggctttt cctccgaccc tgatgctctt caattcggtg 120
ctccgccagc cccagcttgg cgtcctgaga aatggatggt cttcacaata ccctcttcaa 180
tcccttctga ctggttatca gtgcagtggt aatgatgaac acacttctta tggagaaaca 240
ggagtcccag ttcctccttt tggatgtacc ttctcttctg ctcccaatat ggaacatgta 300
ctagcagttg ccaatgaaga aggctttgtt cgattgtata acacagaatc acaaagtttc 360
agaaagaagt gcttcaaaga atggatggct cactggaatg ccgtctttga cctggcctgg 420
gttcctggtg aacttaaact tgttacagca gcaggtgatc aaacagccaa attttgggac 480
gtaaaagctg gtgagctgat tggaacatgc aaaggtcatc aatgcagcct caagtcagtt 540
gccttttcta agtttgagaa agctgtattc tgtacgggtg gaagagatgg caacattatg 600
gtctgggata ccaggtgcaa caaaaaagat gggttttata ggcaagtgaa tcaaatcagt 660
ggagctcaca atacctcaga caagcaaacc ccttcaaaac ccaagaagaa acagaattca 720
aaaggacttg ctccttctgt ggatttccag caaagtgtta ctgtggtcct ctttcaagac 780
gagaatacct tagtctcagc aggagctgtg gatgggataa tcaaagtatg ggatttacgt 840

aagaattata ctgcttatcg acaagaaccc atagcatcca agtctttcct gtacccaggt 900
agcagcactc gaaaacttgg atattcaagt ctgattttgg attccactgg ctctacttta 960
tttgctaatt gcacagacga taacatctac atgtttaata tgactgggtt gaagacttct 1020
ccagtggcta ttttcaatgg acaccagaac tctacctttt atgtaaaatc cagccttagt 1080
ccagatgacc agtttttagt cagtggctca agtgatgaag ctgcctacat atggaaggtc 1140
tccacaccct ggcaacctcc tactgtgctc ctgggtcatt ctcaagaggt cacgtctgtg 1200
tgctggtgtc catctgactt cacaaagatt gctacctgtt ctgatgacaa tacactaaaa 1260
atctggcgct tgaatagagg cttagaggag aaaccaggag gtgataaact ttccacggtg 1320
ggttgggcct ctcagaagaa aaaagagtca agacctggcc tagtaacagt aacgagtagc 1380
cagagtactc ctgccaaagc cccagggta aagtgcaatc catccaattc ttccccgtca 1440
tccgcagctt gtgccccaag ctgtgctgga gacctccctc ttccttcaaa tactcctacg 1500
ttctctatta aaacctctcc tgccaaggcc cggtctccca tcaacagaag aggctctgtc 1560
tcctccgtct ctcccaagcc accttcatct ttcaagatgt cgattagaaa ctgggtgacc 1620
cgaacacctt cctcatcacc acccatcact ccacctgctt cggagaccaa gatcatgtct 1680
ccgagaaaag cccttattcc tgtgagccag aagtcatccc aagcagaggc ttgctctgag 1740
tctagaaata gagtaaagag gaggctagac tcaagctgtc tggagagtgt gaaacaaaag 1800
tgtgtgaaga gttgtaactg tgtgactgag cttgatggcc aagttgaaaa tcttcatttg 1860
gatctgtgct gccttgctgg taaccaggaa gaccttagta aggactctct aggtcctacc 1920
aaatcaagca aaattgaagg agctggtacc agtatctcag agcctccgtc tcctatcagt 1980
ccgtatgctt cagaaagctg tggaacgcta cctcttcctt tgagaccttg tggagaaggg 2040
tctgaaatgg taggcaaaga gaatagttcc ccagagaata aaaactggtt gttggccatg 2100
gcagccaaac ggaaggctga gaatccatct ccacgaagtc cgtcatccca gacacccaat 2160
tccaggagac agagcggaaa gacattgcca agcccggtca ccatcacgcc cagctccatg 2220
aggaaaatct gcacatactt ccatagaaag tcccaggagg acttctgtgg tcctgaacac 2280
tcaacagaat tatagattct aatctgagtg agttactgag ctttggtcca ctaaaacaag 2340
ctgagctttg gtccactaaa acaagatgaa aaatacaaga gtgactctat aactctggtc 2400
tttaagaaag ctgccttttc atttttagac aaaatctttt caacgctgaa atgtacctaa 2460
tctggttcta ctaccataat gtatatgcag cttcccgagg atgaatgctg tgtttaaatt 2520
tcataaagta aatttgtcac tctagcattt tgaatgaata gtcttcactt tttaaattat 2580
tcatcttctc tataataatg acatcccagt tcatggaggc aaaaaacaag tttcttgtta 2640
tcctgaaact ttctatgctc agtggaaagt atctgccagc cacagcatga ggcctgtgaa 2700
ggctgactga gaaatcctct gctgaagacc cctggttctg ttctgcctcc aacatgtata 2760
attttatttg aaatacataa tcttttcact atgcttttgt ggggtttttt ttaagtatgt 2820
gtaaaaatgt gatgctcaga taagtacatt tatatcagtt cagtgttaaa atgcagtctc 2880

ttgagttaaa gtcatcttta ttttaaatgc agtgataaat gtcaactctt cggagaaact 2940
aggagaacaa caacagaaag ctgtgtttgt ctttttctc tcaaatatat ctcccgtatg 3000
agatttcagg tccccatgtt ttcaccaagc aatctgctat gtcagccaac ccaacatcac 3060
tttctacagg aggttatgat ttttgccatt tactagagga agatgtttta tgaaatcaat 3120
ttggggtttg aattcaggtg cagtcatcag ttctttaggg gctgcaatgt tttaaaaaaa 3180
ataagtcatc agattttaag aaaaaagtga tgatttctta ttgatatttt tgtaacagaa 3240
tatagctctt aactgaaaat ccagaaccag aaacataaat cttgagtttc ttttcatgta 3300
cataaaaagc aatagccttt tagtatagat agccctgagc caaaaagtaa tagaattttc 3360
tctagatatt taatacagag agtgtataga ctgactctaa gttaataatg tgcaaaatat 3420
cttaaacatc cctcccctta ttcaacaatt atgtatcagt gatcttgaac cattgtttta 3480
tatttttcac ctttgtaacc tcatggaaag aggctttaca tactttctat gtactattta 3540
cttagaaggg agccccctc cagtcatgaa acttcatttg ttttatccat atccctgagg 3600
actgtgtaga ctttatgtca gttctgtgta gactttatgt cagtttttgt cattatttga 3660
aaatctattc tgacaacttt ttaattcctt tgatcttata agttaaagct gtaacaactg 3720
aaattgcatg gatcaagtaa gcatagtttt atccagggag aaaaataaaa ggaagccata 3780
gaattgctct ggtcaaaacc aagcacacca tagccttaac tgaatattta ggaaatctgc 3840
ctaatctgct tatatttggt gtttgttttt tgactgttgg gctttgggaa gatgttattt 3900
atgaccaata tctgccagta acgctgttta tctcacttgc tttgaaagcc aatgggggaa 3960
aaaaatccat gaaaaaaaaa agattgataa agtagatgat tttgtttgta tccctaccca 4020
tctcctggca gccctactga gtgaaattgg gatacatttg gctgtcagaa attataccga 4080
gtctactggg tataacatgt ctcacttgga aagctagtac ttttaaatgg gtgccaaagg 4140
tcaactgtaa tgagataatt atccctgcct gtgtccatgt cagactttga gctgatcctg 4200
aataataaag ccttttacct t 4221

<210> 86
<211> 867
<212> DNA
<213> human

<400> 86
cgtttcagcg tggcggcgct ggtgctggcg ttggccctgg aggacggccc cgagtgatgg 60
ctggcgcctg cctcccgggt gtctcccggg tacagatgga gtcgtcccgc ggccgccggc 120
ggcaaggtcg gcagctgcga ggccaagaga gaccccagga cacacacagc tgcctcccgg 180
tgcgagaaga agaccccggc ttgagagtga gatggcgttt aatgattgct tcagtttgaa 240
ctaccctggc aacccctgcc caggggactt gatcgaagtg ttccgtcctg gctatcagca 300
ctgggccctg tacttgggtg atggttacgt tatcaacata gcacctgtag atggcattcc 360
tgcgtccttt acaagcgcca agtctgtatt cagcagtaag gccctggtga aaatgcagct 420

cttgaaggat gttgtgggaa atgacacata cagaataaac aataaatacg atgaaacgta 480

cccccctctc cctgtggaag aaatcataaa gcggtcagag tttgtaattg gacaggaggt 540

ggcctataac ttacttgtca acaactgtga acattttgtg acattgcttc gctatggaga 600

aggagtttca gagcaggcca accgagcgat aagtaccgtt gagtttgtga cagctgctgt 660

tggtgtcttc tcattcctgg gcttgtttcc aaaaggacaa agagcaaaat actattaaca 720

atttaccaaa gagatattga tattgaagga atttgggagg aggaaaagaa acctggggtg 780

aatacttatt ttcagtgcat cattactgtt ccagattcct atgatggatg gcagactctt 840

taataaattg cttactgata ttatctt 867

<210> 87
<211> 561
<212> DNA
<213> human

<220>
<221> misc_feature
<222> (534)..(534)
<223> any kind of base

<400> 87
tttttttttc aaattttatt ttttgtactt ttattgaaaa ggtacattta aaaaaataca 60

cagacatttt accatttaca ggttgcagat atagatgctc taaaagagtc cactctattt 120

tgttgttcta tgataactct tgcccctgat atcacaaaca ttccagtctt gttgatatcg 180

gcttaaaaag gggggcatgg gagcatgacc tgcaatatat tcagcgaaca gaaagacaaa 240

ttgttcaatt ataaattttt ttatcttctg tacattattg cattagggag ccacaaaatt 300

atgtagcatc attacaaatg aaaacaggtt aaaaatgaag aagatactta tatagaaata 360

catggattca ttgtcttctt gcagaatgca caagaggtgc aaaaatgtgc aatttaggaa 420

gctctttttc tgtttgtata cgtttgctta gcaacacaaa ccagtgagga agctacaaaa 480

taagttaaac aaaaatagca aacaggtagt aattatagct atgttatatg gctntctatt 540

tcatttaaat atctccaaat a 561

<210> 88
<211> 471
<212> DNA
<213> human

<400> 88
tttcatgaat aattttattt ttattttggt catgactttt taaaattaat ggaaagtctc 60

agtctgctca aatgataaac caaaaaatgg ggtcatgaag caaattcagt ctttgcattt 120

cttcaacaac caactcacat ttctctgctc cttgactcag aggctggaca tgtgctaaca 180

gctttttttgc ttctgtatat ccttaatagg atggcagaat cccggtgtta aaagaatctt 240

aaagtcagcc gtgtcaccat gggacctcga ttagcaaaca gatgtagaca ttccttccaa 300

attcagtcaa agaaaaaact ctaataccag cagccatgtt cagggtgtgc acgacatgct 360

gagcgcttgg gatacatccc cttgtttaat tctcacagaa actttgtgag acaagtacta 420
taatccctgt ttaacagatg ggcaaactga ggtttggaaa aacactcatt t 471

<210> 89
<211> 727
<212> DNA
<213> human

<400> 89
taaaatatta ccttttattt tctcaggcac acaagtgatt tggataccaa ttatcaagac 60
attttactga tttcccttta gaatgatcta ttttaaatct tcagccacct tagaaaaagt 120
ttgcagcaat cactttgcaa ttcataagta tcaggttaga atttagttgt ggaataagtt 180
aacagtttat gttcaatatg tgaggttatt tgaactcctg agttttaaat atcctgtgga 240
acagtgattc ctctcccttc taatggcttt tcagattaaa gcaatgactt taaaaagatt 300
acatcctaaa tacttgatta caacagaaat cgaccaatct aaaaatcaga tagtgttata 360
ctgaacatca ttctgatata atgagtagcc tctggctgaa acaaaattcc accaccaagg 420
ccatcaacca ggttagtact gtttttcctg gggtctatgt aaactctcct tttctctgca 480
aatgctgctt ggctgtgaac agcatggatt tacctgcacc aatgtggcac acacctagca 540
actttctcaa gcattctaaa gatatcccca gagctacaat attgacatat gcacagcact 600
ttctctagac agtccaatca gcgtgcacat cacacacaca gaatgctggg atatgctata 660
ctgcacactt agtacacagg tggaatagag tacaatgact aaagctcaca gaaaatgttt 720
tagtctt 727

<210> 90
<211> 460
<212> DNA
<213> human

<400> 90
tttttgatat aaagggcttt tattttcttt acagttttct gtattttcca aatttctaca 60
taaacataca cataaaagtc ataaaaatgt ccctcaaaat gacactccct ccaatgcagg 120
gagtgaggag gtgtgtgctg ggacgccaca gggtggctgc tgcagctaat ccctgtgcag 180
gtgcagccac tgctacactc cagcgtctga ggcccctctg cactggcagt gttagaatgg 240
ctgtctgaga gccaaggctg ttcacccgag cagagagtca tggagctggt acaggcagga 300
gccaaggtaa gcactaggct ggtgtgtgcc ctccaggggg caccctcctt gaagcaggcc 360
ctccaaggta ctcgcccccct gaggcagccg ataacagccg gaagccttcc cctgggggga 420
cctggccatt tgtggagcag ggaaaggctc cactgccagt 460

<210> 91
<211> 392
<212> DNA
<213> human

<400> 91
tttttttttt ttttttgag ttgctgaaaa gtttactttt gagttttaaa ctgtactttg 60

aaaactatat tgtgaactgt gttgacatga ggaaaggctg ggctccctga aaacatccag 120

ttccacagca gggcgggccc aagcccagcc aatccccagg caatgcaggg cagggatccc 180

acctggtata agtacgggca gagggcagag ccaggctgta tcgaggggcg gcgctgggac 240

cctcctcgcc cagaattcct actcatcccc agcacagaag tgctctgtag ggccagctga 300

ggacaccccg gttcactgag ggtggcccac agtaagtcgc cgtctggcag taagttagct 360

ctgcaggtgt ggaccccaag accacacacg gc 392

<210> 92
<211> 496
<212> DNA
<213> human

<400> 92
tttttgtat ttttctcaat ataatgttgc aagatgtcat tatattctca ttacccataa 60

cttcccatcc aaatcttaat ggcacatttg atatttttc aaatggcttt gagatataat 120

tcacatacca cacaattcat gtatctaaag tatacagttc tgtagttctg tggttgttgg 180

ttacatccac cagactgagg gctccctgag ggttggtgcc tcagctttcc cttcacttac 240

tcattcacca aacattcagc gcctactgag tactggggtt atgatggcca acaggagaga 300

cagtctctgc ctactgtttg gtgggggtgg agtacttagt aaccgtcgtc attattgagt 360

gcttacctgt gctgggcaca gtgctgctac tgggtgactg tgtccccagg gctgtcccag 420

gctgggggtc tggaggagta gttatcagtt gaactgagtt aatccacagt ggaaggtaac 480

ccactccctg ccctag 496

<210> 93
<211> 472
<212> DNA
<213> human

<400> 93
ttaacacaaa agcttttact tggaaactgg caaatactgg actagaatac tgacatgctc 60

acgctctggc gggagctcgg atgcagaagc tattgcacaa agcccctctg attgccttgc 120

tcctctttgg catgtatcag cagagcccca agggccaatt gcccacaggt ggggactgtt 180

ctccatcaag gtatggggac ccctacttcc ttgttttgtt aaaaagtgca ggtaggcgaa 240

gaaagcccag gcagttgacc cagtctttga aacagctgac tccccagagc tggggccagg 300

ggagcctggt cttgaggggt aagggctgca gggccaggct gatggcctgt gtcatggcat 360

tggccatctc ctctccagct tctcctcagc catcgcccgt ccgtcatggt ggtgtcggct 420

gcacctggac cttcctgcct ctccgctcag ggcagcagca gtgagtgcag ca 472

<210> 94
<211> 585
<212> DNA
<213> human

<400> 94

tttttatatt atagtaaatt tatttggtat aattacatat taacattatt tacaatgcta 60

atttttttat ttataaagta tctttatatg gacaaaaaga tacaaactgt tatcatttta 120

agtacaaagt atttctagaa atacattata agccattaaa aaaaaaaacg atatttcaag 180

attggttctt acatgctatg accaactcat atgaaagagc aagttgctcc cccttcattc 240

cttcccccaa ctccaaaggg aaaggaattt gatatttagg ctttaaaaaa tttcctacta 300

cctttatctt ttaaaaaacc ctactcaaaa caactatctc ttataaggga aaatatcata 360

gataagattt tcctttagaa aatgacatta aaagtggcat gagccctaga atgatatgtg 420

tattagaggc acttaaaaaa aatcagaagg gatccatagg aaggaattta attcagcaaa 480

tactgagtgt ccactgcatg caaggtaccc tgccagaaat ctcaaatgag taagttgtcc 540

ttagggatag aaggtgctaa gcatcatgca aaagatatga actga 585

<210> 95
<211> 400
<212> DNA
<213> human

<400> 95
ttattgtttt ttaaaaatac aattttgaaa tttattgttg aaaatggaca catggaacaa 60

accaaacctt gttttatcat gtaattttca gaaaatatgt gatccataaa gattaaaaga 120

aagttgtatt aagtctggca gctttagtat taacttgaaa taaaatatgg caagctttcc 180

acgtcctcct ttatttccac aatccatatg tacgagctag attccagtca gaacttccac 240

aaatacttca ctctttggta gcagcggtta taaattacgc ctttgctaat ttgcgttgtt 300

cccaaccagg agaaacatta ccacaaaaaa agtcagtttc atcctgcagt gttcccgcag 360

caaccatatt aaagctgaag aataaagctc ctttgtagta 400

<210> 96
<211> 461
<212> DNA
<213> human

<400> 96
ttatgccatg aattcctttt tactgaaata ccgtaggact cactaccaca ataagtactt 60

aagctgaaag agtttctaat gggagccaag taaattcagc tctccactgt gcaaagcatc 120

ttgtcatttc tataataaaa gtctttccat gttcagtcca ctttggctct ggaacctgga 180

tgagtcatgc ttggggccca gggtgcctgt gaggatgctg catgagaatt tcagctgtgg 240

tggcagtggc tgggagtccc actgactcag ggggaggcca ggcgagatga gctggaactt 300

ttaggggaga gctggcactt agggacatca ttgattgtgc ttttcttagc ctagttctgt 360

cctgcaattt atttttctta tcatgtgact gtcggctgaa gtctggggtt atttggtttc 420

tttttcttct tccttgctgg acagtctcca tggggtcacg g 461

<210> 97
<211> 542
<212> DNA

<213> human

<400> 97
taaatccagt aaagcatagt actagattca tcatacttgt acaatacaac gggcgacatg 60
aaaatggcaa aggctctcct ttttgtgaac aatttaatac aactggtggt ccaataactc 120
tacaatcagc cttgtagagg tcattaaaga cagaatcctg aaagtccgtg actacaaata 180
cattttcaaa ttccggagaa tccaaacctt caaattcttc cactgactcc atctttacaa 240
agcccacttt aatgtccttt aaggctttta taagttcttc ttgttttcca gcttcttgaa 300
ccaatatcac tcttgtttca atctgaggca tctcttcttc tacatatgaa gtagatccaa 360
taagtaagtt ttccttggaa atctcagtaa ctttagaatc aaaaatggaa gagtctgcca 420
agctagtcct cccagtagtg gatgttaata cactattttc agccatgatt tgtattcttc 480
taaatcagca ctctcaaaaa agccctagga gttccacctc ttcaaacgcc gactcctctc 540
ac 542

<210> 98
<211> 1017
<212> DNA
<213> human

<400> 98
atgggcacct ggcttctggc ctgcacctgc gtctgcacct gtgtctgctc gggagtctct 60
gtctcagggg atggacgagg gccaagggct ggaacctcca cctgcctcac caacaacatt 120
ctcaggattg attgccactg gtctgcccca gagctgggtc agggctccag ccccgggctc 180
cccttcatca gccccgtggt gctgacacat gccctttca gcaaccaggc tgctggtggc 240
acacagaagt gcatctggca gggcagtgag tgcactgtag tgttgccgcc caaggcagca 300
ctcctgccat ctgacaattt catcatcact ttctaccact gcatgtccgg gagggatcag 360
agcacgtcag ctcgccactg catcctgacc tggagcctca gtcctgcctt ggagtcaatg 420
accacacttc tcagctatga gctggacttc aagaggcagg aagaggcctg ggagcgggcc 480
cagcacaggg atcacattgt cggggtgacc tggctcatac ttgaagcctt tgagctggac 540
cctggcttta tccttgaggc caggctgcgt gtccagacgg ccatgctggg ggatgacggg 600
gcacaggagg agcgagggga gccagcccat gggaagagtg aggcccagga gtgtggttca 660
cacaaggtcc ttcagcaggt gacacaaacc tccaaggccc atcacaaggt ccttcagcag 720
atgacacaaa cctccaaggc tcatcacaaa ccttccactt tggcccaggg cactaaaggg 780
cgcacttttg ccagccctgg gcccttcctg cccacggacc ctctgatccc accctggggg 840
tggccaggca acacctttgt tgctgtgtcc atctttctcc tgctgactgg cccgacctac 900
ctcctgttca agctgtcgcc cagactcctc actttgggca aaggacaaga agcaactcgg 960
atgggggccc acagggctgg tgtgctgctg agccaggact gtgctggcac ccgatga 1017

<210> 99
<211> 1099
<212> DNA

<213> human

<400> 99
agccccaagc ttaccacctg cacccggaga gctgtgtcac catgtgggtc ccggttgtct 60

tcctcaccct gtccgtgacg tggattggtg agaggggcca tggttggggg gatgcaggag 120

agggagccag ccctgactgt caagctgagg ctctttcccc cccaacccag caccccagcc 180

cagacaggga gctgggctct tttctgtctc tcccagcccc actccaagcc cataccccca 240

gcccctccat attgcaacag tcctcactcc cacaccaggt ccccgctccc tcccacttac 300

cccagaactt tctccccatt gcccagccag ctccctgctc ccagctgctt tactaaaggg 360

gaagttcctg ggcatctccg tgtttctctt tgtggggctc aaaacctcca aggacctctc 420

tcaatgccat tggttccttg gaccgtatca ctggtccacc tcctgagccc ctcaatccta 480

tcacagtcta ctgacttttc ccattcagct gtgagtgccc aaccctatcc cagagacctt 540

gatgcttggc ctcccaatct tgccctagga tacccagatg ccaaccagac acctccttct 600

tcctagccag gctatctggc ctgagacaac aaatgggtcc ctcagtctgg caatgggact 660

ctgagaactc ctcattccct gactcttagc cccagactct tcattcagtg gcccacattt 720

tccttaggaa aaacatgagc atccccagcc acaactgcca gctctctgat tccccaaatc 780

tgcatccttt tcaaaaccta aaaacaaaaa gaaaaacaaa taaaacaaaa ccaactcaga 840

ccagaactgt tttctcaacc tgggacttcc taaactttcc aaaaccttcc tcttccagca 900

actgaacctc gccataaggc acttatccct ggttcctagc accccttatc ccctcagaat 960

ccacaacttg taccaagttt cccttctccc agtccaagac cccaaatcac cacaaaggac 1020

ccaatcccca gactcaagat atggtctggg cgctgtcttg tgtctcctac cctgatccct 1080

gggttcaact ctgctccca 1099

<210> 100
<211> 1095
<212> DNA
<213> human

<400> 100
ccaagcttac cacctgcacc cggagagctg tgtcaccatg tgggtcccgg ttgtcttcct 60

caccctgtcc gtgacgtgga ttggtgagag gggccatggt tgggggggatg caggagaggg 120

agccagccct gactgtcaag ctgaggctct ttccccccca acccagcacc ccagcccaga 180

cagggagctg ggctcttttc tgtctctccc agccccactt caagcccata cccccagccc 240

ctccatattg caacagtcct cactcccaca ccaggtcccc gctccctccc acttacccca 300

gaactttctc cccattgccc agccagctcc ctgctcccag ctgctttact aaaggggaag 360

ttcctgggca tctccgtgtt tctctttgtg gggctcaaaa cctccaagga cctctctcaa 420

tgccattggt tccttggacc gtatcactgg tccatctcct gagcccctca atcctatcac 480

agtctactga cttttcccat tcagctgtga gtgtccaacc ctatcccaga gaccttgatg 540

cttggcctcc caatcttgcc ctaggatacc cagatgccaa ccagacacct ccttcttcct 600

```
agccaggcta tctggcctga gacaacaaat gggtccctca gtctggcaat gggactctga    660
gaactcctca ttccctgact cttagcccca gactcttcat tcagtggccc acattttcct    720
taggaaaaac atgagcatcc ccagccacaa ctgccagctc tctgagtccc caaatctgca    780
tccttttcaa aacctaaaaa caaaaagaaa aacaaataaa acaaaaccaa ctcagaccag    840
aactgttttc tcaacctggg acttcctaaa ctttccaaaa ccttcctctt ccagcaactg    900
aacctcgcca taaggcactt atccctggtt cctagcaccc cttatcccct cagaatccac    960
aacttgtacc aagtttccct tctcccagtc caagacccca aatcaccaca aaggacccaa   1020
tccccagact caagatatgg tctgggcgct gtcttgtgtc tcctaccctg atccctgggt   1080
tcaactctgc tccca                                                    1095
```